# EUROPEAN PATENT SPECIFICATION

(11) **EP 2 859 868 B1**
(45) Date of publication and mention of the grant of the patent: **03.05.2017**
(21) Application number: 13803729.6
(22) Date of filing: 05.06.2013
(51) Int. Cl.: A61F 13/534

(54) **FIBER-STACKING DEVICE**
FASERSCHICHTUNGSVORRICHTUNG
DISPOSITIF D'EMPILAGE DE FIBRES

(30) Priority: 11.06.2012 JP 2012131845; 11.06.2012 JP 2012131846
(43) Date of publication of application: 15.04.2015
(73) Proprietor: Kao Corporation, Chuo-Ku Tokyo 103-8210 (JP)
(72) Inventor: MOTEGI, Tomoyuki, Haga-gun Tochigi 321-3497 (JP); MARUYAMA, Hiroshi, Haga-gun Tochigi 321-3497 (JP); MATSUNAGA, Ryuji, Haga-gun Tochigi 321-3497 (JP)
(74) Representative: Vossius & Partner Patentanwälte Rechtsanwälte mbB
(86) International application number: PCT/JP2013/065555
(87) International publication number: WO 2013/187291

(56) References cited:
- EP-A1- 1 943 991
- EP-A1- 2 554 145
- EP-A2- 0 226 939
- JP-A- H07 119 013
- JP-A- 2006 122 109
- JP-A- 2012 016 584

## Description

### Technical Field

The present invention relates to a fiber stacking device that includes a rotating drum including a collecting/stacking recess on the outer peripheral surface thereof and is used to obtain a shaped product (absorbent member) having a predetermined shape by sucking and stacking a shaped-product material, such as a fiber material or a water-absorbent polymer, in the collecting/stacking recess.

### Background Art

A fiber stacking device is known as a device for manufacturing absorbent members used for absorbent articles, such as disposable diapers, sanitary napkins, and incontinence pads. The fiber stacking device includes: a rotating drum that includes an collecting/stacking recess on an outer peripheral surface thereof; supplies a shaped-product material, such as pulp, to the outer peripheral surface in a dispersed airborne state while rotating the rotating drum; stacks the shaped-product material in the collecting/stacking recess by suction from a bottom surface of the collecting/stacking recess that is formed of a porous member including a plurality of suction holes; releases a fiber stack, which is present in the collecting/stacking recess, from the collecting/stacking recess by suction from suction means that is disposed so as to face the collecting/stacking recess; and transfers the fiber stack onto the suction means. Further, a technique, which stacks a shaped-product material in a collecting/stacking recess corresponding to each absorbent member so that a portion in which the shaped-product material is stacked with a high basis weight and a portion in which the shaped-product material is stacked with a basis weight lower than the high basis weight are formed, is also known from the viewpoint of the improvement of absorbing ability and a wearing feeling.

In relation to the fiber stacking device having the above-mentioned structure, for example, JP 2000-234255 A discloses a technique in which the aperture ratio (the opening area ratio) of a bottom section of the collecting/stacking recess formed of a mesh made of metal is set to be partially different in order to adjust the density and the amount of the fiber stack present in the collecting/stacking recess. Further, JP 2002-272782 A discloses a technique in which a suction chamber formed in a rotating drum is partitioned into a plurality of suction chambers in a longitudinal section taken along a flow direction, suction means is provided for each suction chamber, and the respective suction forces of the suction means are set to be different from each other, in a fiber stacking device having the above-mentioned structure (a fiber stacking device which forms an absorbent member by stacking pulverized pulp, which is transported on an air stream, on the surface of a suction-stacking section through suction from an inner surface and in which a suction chamber provided in a stacking body is partitioned into a plurality of suction chambers in a longitudinal section taken along a flow direction and suction means is provided for each suction chamber). Further, JP 62-206071 A discloses a rotating drum in which a space member including a plurality of openings (large openings), which have a diameter larger than the diameter of the opening of a metal mesh, and a gas flow rate control layer including a plurality of opening (small openings), which have a diameter smaller than the diameter of the opening of the space member, are laminated in this order on an inner surface (the side on which a shaped-product material is not stacked) of a web layer that forms a bottom surface of a collecting/stacking recess and is formed of the metal mesh (a porous member). According to JP 62-206071 A, air flows through the web layer in a predetermined pattern by the operation of the gas flow rate control layer. Accordingly, it is possible to stack fiber on the web layer with a desired basis weight.

Furthermore, US 2009/281511 A1 discloses a fiber stacking device in which a honeycomb structure rectifier for rectifying an air stream is integrally provided on the inside of an air-permeable porous plate that forms a bottom section of a suction-stacking section and includes a plurality of suction hole. Since the suction holes of the porous plate are formed in the shape of a bowl from the surface toward the inside in the fiber stacking device disclosed in US 2009/281511 A1, an absorbent-core material is not fitted completely into the suction hole and does not fall out into the inside. Accordingly, the loss of a raw material can be reduced. According to US 2009/281511 A1, since the fiber stacking device of this structure is used, the profile of an absorbent member is stabilized and variation in the weight of the absorbent member is suppressed.

Further, JP 2000-178866 A discloses a device including a stationary drum of which inside is maintained at negative pressure, and a rotating drum that rotates along the outer peripheral surface of the stationary drum and includes a plurality of collecting/stacking recesses on the outer peripheral surface thereof, as a device for manufacturing a shaped product. Each of the collecting/stacking recesses is divided into a plurality of unit stackers that can suck a material independently of each other, and a material can be sucked and stacked on each unit stacker. In the device disclosed in JP 2000-178866 A, suction openings corresponding to the respective unit stackers are formed on the inner peripheral surface of the rotating drum, and suction ports are provided on the outer peripheral surface of the stationary drum. Furthermore, the respective unit stackers communicate with corresponding suction openings through communication pipes. Accordingly, when the rotating drum rotates and the suction openings overlap the suction ports of the stationary drum, the unit stackers communicating with the suction ports of the respective collecting/stacking recesses are selectively sucked.

EP 0 226 939 describes an apparatus for forming a fibrous web, and includes a fibrous supply mechanism for providing fibers of web material. The fibers are deposited onto a foraminous web forming layer which may optionally have a pocket recess formed therein. A foraminous spacing mechanism supports the web forming layer while allowing a substantially unrestricted gas flow from a region immediately adjacent to and downstream from the web forming layer. A gas flow regulating layer has a selected pattern of apertures therethrough, and is fixedly positioned in adjacent facing relation with the foraminous spacing means. The regulating layer provides a selected pattern of gas flow through the web forming layer. The regulating layer, the foraminous spacing mechanism and the gas flow regulating layer are constructed to form an installable and replaceable assembly, and a flow forcing mechanism provides a flow of gas through the web forming layer.

### Summary of Invention

### Technical Problem

In the fiber stacking device having the above-mentioned structure, as disclosed in JP 2000-234255 A, it is possible to control the amount of a shaped-product material to be stacked on the bottom section (the basis weight of the shaped product) by appropriately setting an opening area ratio of the bottom section of the collecting/stacking recess (a ratio of the total area of suction holes per unit area). In general, when the opening area ratio is reduced, a suction force for sucking a shaped-product material is reduced and the amount of a shaped-product material to be stacked is reduced. Accordingly, when the opening area ratio of the bottom section of the collecting/stacking recess is set to be partially different, a strong suction section in which a suction force is relatively large and a weak suction section in which a suction force is relatively small are formed on the bottom section even though suction is performed with a constant suction force from the inside of the device by using single suction means. Accordingly, a shaped product, which includes a high basis-weight section in which a shaped-product material is stacked on the suction section with a relatively high basis weight and a low basis-weight section in which a shaped-product material is stacked on the suction section with a relatively low basis weight, can be manufactured, and it is possible to adjust the basis weight of a shaped product by a device having a relatively simple structure.

However, when the opening area ratio of the bottom section of the collecting/stacking recess is intentionally reduced as disclosed in JP 2000-234255 A, a difference in a suction force for sucking a shaped-product material becomes significant at suction holes, the vicinity thereof, and other portions in a weak suction section of the bottom section that is formed by the intentional reduction of the opening area ratio. For this reason, a large difference in the amount of a shaped-product material to be stacked may occur between both portions. As a result, there is a concern that fiber-stacking irregularity caused by a difference in the amount of a shaped-product material may occur in a low basis-weight section which is stacked on the weak suction section and in which the amount of a shaped-product material should be originally uniform. For example, when an absorbent member is an absorbent member used for an absorbent article, there is a concern that the occurrence of fiber-stacking irregularity may cause the reduction of absorption performance, the deterioration of a wearing feeling of an absorbent article, or the like. For this reason, the occurrence of fiber-stacking irregularity is not preferable. JP 2000-234255 A does not disclose fiber-stacking irregularity that is caused by the intentional reduction of the opening area ratio of the bottom section of the collecting/stacking recess, and a fiber stacking device that can adjust the amount of a shaped-product material to be stacked without the occurrence of fiber-stacking irregularity has not been provided yet.

Further, since the technique disclosed in JP 62-206071 A has the following problems 1 to 3, there is a room for further improvement. Problem 1: since one small opening of the gas flow rate control layer is disposed over a plurality of large openings of the space member, there is a possibility that fiber-stacking irregularity may occur. Problem 2: since the number of the small openings of the gas flow rate control layer corresponding to each of the plurality of large openings of the space member is different, there is a possibility that fiber-stacking irregularity may occur. Problem 3: pockets (recesses) in which a shaped-product material is partially stacked with a high basis weight are formed on the metal mesh (the porous member), end portions having a shape surrounding the regions of the pockets are formed on the gas flow rate control layer, and the end portions are disposed over the plurality of openings of the space member. For this reason, there is a possibility that a high basis-weight section (so-called middle-high section) cannot be formed according to design.

Since a main object of the technique disclosed in US 2009/281511 A1 is to stabilize the profile of the absorbent member, the technique disclosed in US 2009/281511 A1 is not to adjust the basis weight of each portion of the absorbent member unlike the technique disclosed in JP 2002-272782 A. Since the technique disclosed in JP 2002-272782 A can adjust the basis weight of each portion of the absorbent member but requires a plurality of suction means for suction in the plurality of chambers, the costs of manufacturing facilities are increased. For this reason, there is a concern that the manufacturing cost of an absorbent member may be increased. Further, the technique disclosed in JP 2002-272782 A cannot change a suction force, which is generated by the suction chamber, in the flow direction (the circumferential direction or the rotating direction of the rotating drum), a shaped-product material cannot but be stacked with a uniform basis weight in the flow direction, variation in the distribution of a basis weight is small. For this reason, there is a concern that it is not possible to sufficiently cope with various demands on the absorbent member. Furthermore, in the technique disclosed in JP 2002-272782 A, a gap is likely to be formed between the collecting/stacking recess, which is rotatably arranged on the outer peripheral surface of the rotating drum, and a partition wall, which is arranged in the rotating drum and partitions the suction chamber into a plurality of regions. Therefore, there is concern that the air stream leaks from the gap. For this reason, there is a concern that a difference in basis weight according to design cannot be obtained in the absorbent member. Moreover, in the technique disclosed in JP 2000-178866 A, a part of the collecting/stacking recess communicating with the suction ports selectively sucks and holds a raw material only when the suction openings of the rotating drum and the suction ports of the stationary drum overlap each other. Therefore, since a time when a raw material is not sucked and not held is present in the fiber stacking step even though short, and an air stream flowing in the duct for supplying a raw material to the collecting/stacking recess is disturbed during the time, there is a concern that a disadvantage of turning up a shaped product stacked in the collecting/stacking recess may occur. Further, since the structure of the device disclosed in JP 2000-178866 A is complicated, there is a room for improvement in terms of operation, maintenance, and the like.

### Solution to Problem

The invention is specified in the claims.

The invention (a first aspect of the invention) provides a fiber stacking device including a collecting/stacking recess in which a shaped-product material is stacked and which is formed on an outer surface thereof, and stacking a shaped-product material, which is transported on an air stream generated by suction from inside, on a bottom surface of the collecting/stacking recess, which is formed on a porous member including a plurality of suction holes, while transporting the collecting/stacking recess in one direction,
wherein an adjuster, which adjusts the air stream, is arranged on an inner surface of the porous member so as to overlap at least a part of the inner surface of the porous member, and
the adjuster includes a plurality of openings that penetrates the adjuster in a thickness direction, and the area of an opening end portion of each opening relatively distant from the porous member is smaller than the area of an opening end portion thereof relatively close to the porous member.

The invention (the first aspect of the invention) provides a method of manufacturing an absorbent member by using the fiber stacking device, and the method includes a fiber stacking step of sucking and stacking a shaped-product material, which is an absorbent-core material supplied on an air stream, in the collecting/stacking recess of the fiber stacking device.

The invention (a second aspect of the invention) provides a fiber stacking device that includes a rotating drum including a collecting/stacking recess formed on an outer peripheral surface thereof and forms a shaped product by stacking a shaped-product material, which is transported on an air stream generated by suction from inside of the rotating drum, on a bottom section of the collecting/stacking recess,
wherein the bottom section of the collecting/stacking recess is formed of an air-permeable porous member,
the shaped-product material is stacked on an outer surface of the porous member,
a flow rate adjusting member that adjusts the flow rate of the air stream is arranged on an inner surface of the porous member,
the porous member and the flow rate adjusting member are integrally rotated by the rotation of the rotating drum,
a bottom-section corresponding section of the flow rate adjusting member, which overlaps the bottom section of the collecting/stacking recess in a planar view of the collecting/stacking recess, includes a plurality of openings that penetrate the bottom-section corresponding section in a thickness direction and an opening defining section that partitions and forms the respective openings, and
a part of the plurality of openings are porous member-side-small-open-type openings each of which the area of an opening end portion relatively close to the porous member is smaller than the area of an opening end portion relatively distant from the porous member.

Further, the invention (the second aspect of the invention) provides a method of manufacturing an absorbent member by using the fiber stacking device, and the method is used to manufacture an absorbent member that includes a high basis-weight section which is stacked on a portion of the bottom section of the collecting/stacking recess corresponding to the porous member-side-small-open-type opening and in which the stacked amount of a shaped-product material is relatively large and a low basis-weight section which is stacked on other portions of the bottom section and in which the stacked amount of the shaped-product material is relatively small.

### Advantageous Effects of Invention

According to the invention (a first aspect of the invention), it is possible to adjust the amount of a shaped-product material to be stacked without the occurrence of fiber-stacking irregularity, and to efficiently manufacture a high-quality shaped product in which the stacked amount of a shaped-product material is partially different. Further, according to a fiber stacking device, the method of manufacturing an absorbent member by using the fiber stacking device of the invention (a second aspect of the invention), it is possible to accurately adjust the basis weight of each portion of a shaped product.

### Brief Description of Drawings

[Fig. 1] Fig. 1 is a schematic perspective view illustrating an embodiment (first embodiment) of a fiber stacking device of the invention (a first aspect of the invention).
[Fig. 2] Fig. 2 is a diagram illustrating an outer peripheral portion (collecting/stacking recess), which is spread in a planar shape, of a rotating drum of the fiber stacking device illustrated in Fig. 1.
[Fig. 3] Fig. 3 is an exploded perspective view of the outer peripheral portion of the rotating drum illustrated in Fig. 2.
[Fig. 4] Fig. 4 is a perspective view schematically illustrating the cross-section taken along line I-I of Fig. 2.
[Fig. 5] Fig. 5 is a cross-sectional view schematically illustrating a part of the cross-section taken along line I-I of Fig. 2.
[Fig. 6] Fig. 6 is a perspective view schematically illustrating the cross-section taken along line II-II of Fig. 2.
[Fig. 7] Fig. 7 is a perspective view illustrating a fiber stack that is released from the collectirlg/stacking recess of the rotating drum of the fiber stacking device illustrated in Fig. 1.
[Fig. 8] Fig. 8 is a perspective view of an outer peripheral portion (collecting/stacking recess) of a rotating drum of another embodiment (second embodiment) of the fiber stacking device of the invention (the first aspect of the invention).
[Fig. 9] Fig. 9 is an exploded perspective view of the outer peripheral portion of the rotating drum illustrated in Fig. 8.
[Fig. 10] Figs. 10(a) and 10(b) are diagrams schematically illustrating the cross-section of a part of a bottom section of the collecting/stacking recess of the outer peripheral portion of the rotating drum illustrated in Fig. 8 taken along a drum's width direction (a direction parallel to the rotation axis of the rotating drum), Fig. 10(a) is a perspective view of a region (a high basis-weight fiber-stack region) in which an adjuster is not arranged, and Fig. 10(b) is a perspective view of a region (a low basis-weight fiber-stack region) in which the adjuster is arranged.
[Fig. 11] Fig. 11 is a perspective view illustrating a fiber stack that is released from the collecting/stacking recess of the rotating drum illustrated in Fig. 8.
[Fig. 12] Figs. 12(a) to 12(e) are plan views schematically illustrating the correspondence between a first layer and a second layer of the adjuster according to the invention (the first aspect of the invention).
[Fig. 13] Fig. 13 is a diagram, which corresponds to Fig. 4, of still another embodiment of the fiber stacking device of the invention (the first aspect of the invention).
[Fig. 14] Figs. 14(a) to 14(d) are cross-sectional views schematically illustrating the cross-section of an opening of the adjuster according to the invention (the first aspect of the invention) taken along the drum's width direction.
[Fig. 15] Fig. 15 is a schematic perspective view illustrating yet another embodiment of the fiber stacking device of the invention (the first aspect of the invention) while a part thereof is transparently illustrated.
[Fig. 16] Fig. 16 is a schematic perspective view of an embodiment of a fiber stacking device of the invention (a second aspect of the invention).
[Fig. 17] Fig. 17 is a perspective view illustrating a rotating drum of the fiber stacking device illustrated in Fig. 16.
[Fig. 18] Fig. 18 is a diagram illustrating the structure of the rotating drum illustrated in Fig. 17.

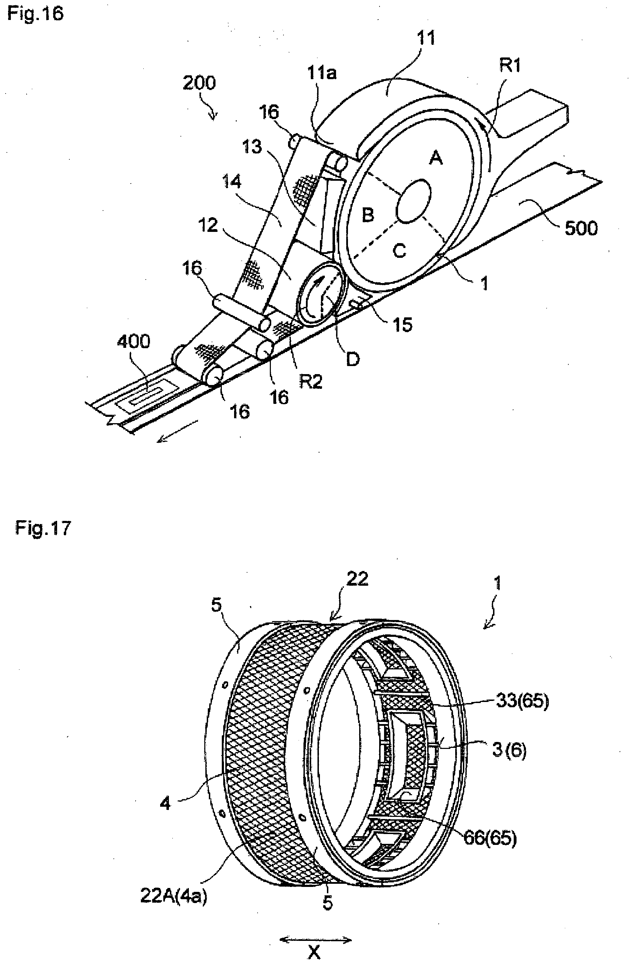

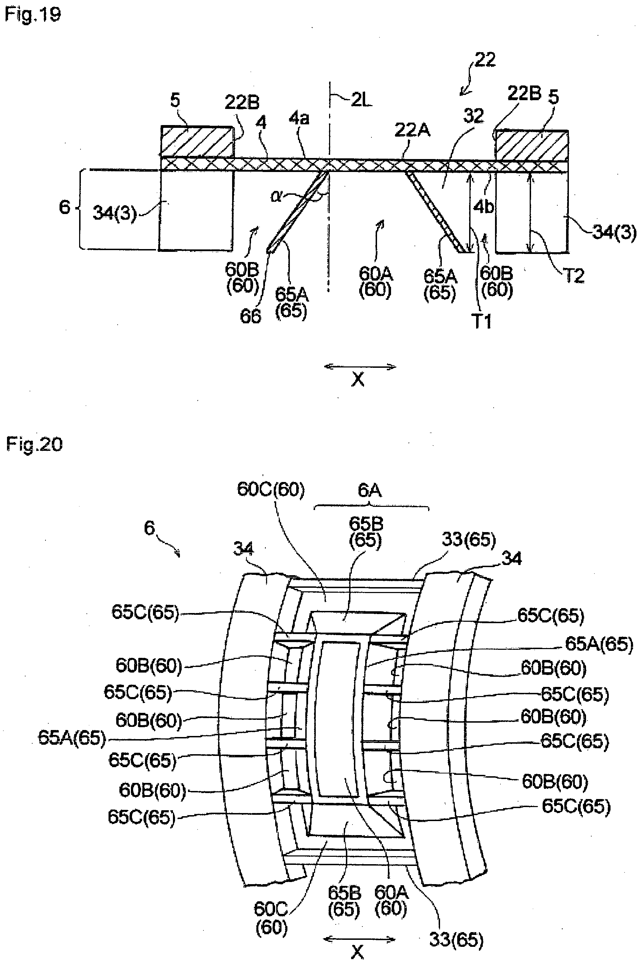

[Fig. 19] Fig. 19 is a cross-sectional view illustrating the cross-sections (end faces) of specific portions of a collecting/stacking recess and a flow rate adjusting member of the rotating drum illustrated in Fig. 17 taken along a drum's width direction (a direction parallel to the rotation axis of the rotating drum). The specific portions are portions on which defining members denoted in Fig. 20 by reference numeral 65A and extending in a drum's circumferential direction are disposed and defining members denoted in Fig. 20 by reference numeral 65C and extending in a drum's width direction are not disposed.
[Fig. 20] Fig. 20 is an enlarged perspective view of a part of the flow rate adjusting member of the rotating drum illustrated in Fig. 17.
[Fig. 21] Fig. 21 is a cross-sectional view (corresponding to Fig. 19) illustrating a state where a shaped-product material is stacked in the collecting/stacking recess of the rotating drum illustrated in Fig. 17.
[Fig. 22] Fig. 22(a) is a perspective view illustrating a fiber stack that is released from the collecting/stacking recess illustrated in Fig. 21, and Fig. 22(b) is a cross-sectional view taken along line I-I of Fig. 22(a).
[Fig. 23] Fig. 23 is a diagram (corresponding to a lower diagram of Fig. 18) illustrating main parts of another embodiment of the rotating drum according to the invention (the second aspect of the invention).
[Fig. 24] Fig. 24 is a diagram (corresponding to Fig. 19) illustrating main parts of still another embodiment of the rotating drum according to the invention (the second aspect of the invention).
[Fig. 25] Fig. 25 is a diagram (corresponding to Fig. 18) illustrating main parts of yet another embodiment of the rotating drum according to the invention (the second aspect of the invention).
[Fig. 26] Fig. 26 is a cross-sectional view (corresponding to Fig. 19) illustrating the cross-sections of a collecting/stacking recess and a flow rate adjusting member of the rotating drum illustrated in Fig. 25 taken along a drum's width direction (a direction parallel to the rotation axis of the rotating drum).
[Fig. 27] Fig. 27 is a perspective view (corresponding to Fig. 22(a)) illustrating a fiber stack that is released from the collecting/stacking recess illustrated in Fig. 26.
[Fig. 28] Fig. 28 is a diagram (corresponding to Fig. 19) illustrating main parts of another embodiment of the rotating drum according to the invention (the second aspect of the invention).
[Fig. 29] Fig. 29 is a schematic perspective view illustrating another embodiment of the fiber stacking device of the invention (the second aspect of the invention) while a part thereof is transparently illustrated.

### Description of Embodiments

The invention (a first aspect of the invention) relates to a fiber stacking device that can adjust the amount of a shaped-product material to be stacked without generating fiber-stacking irregularity. Further, the invention (a second aspect of the invention) relates to a fiber stacking device that can accurately adjust the basis weight of each portion of a shaped product.

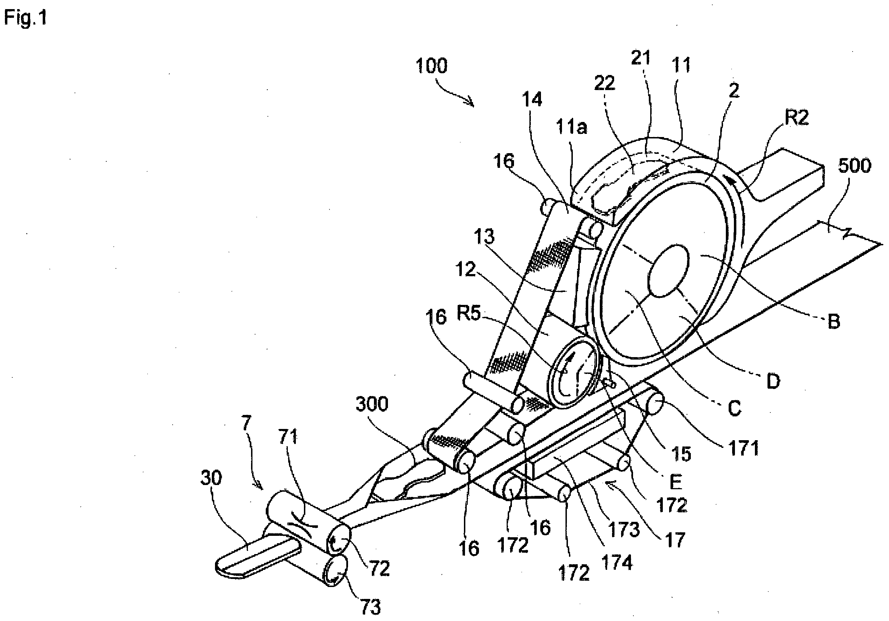

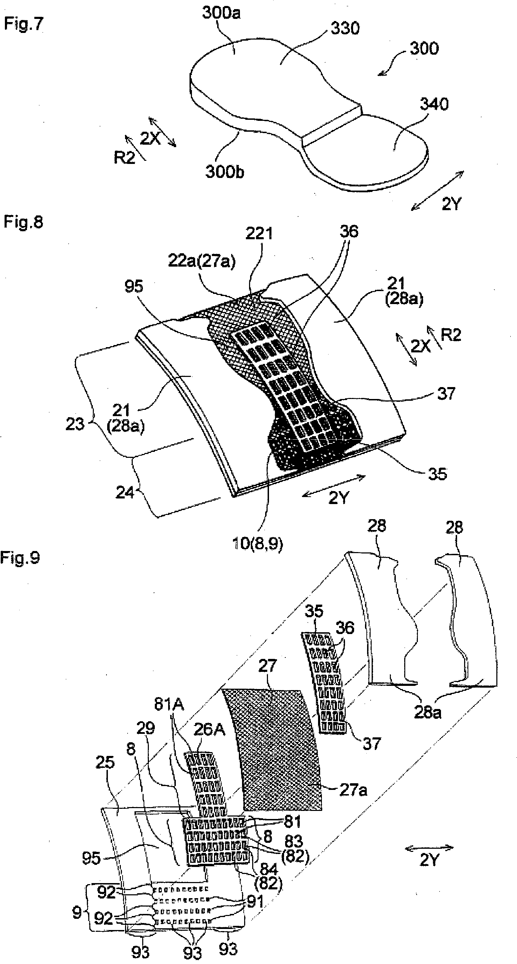

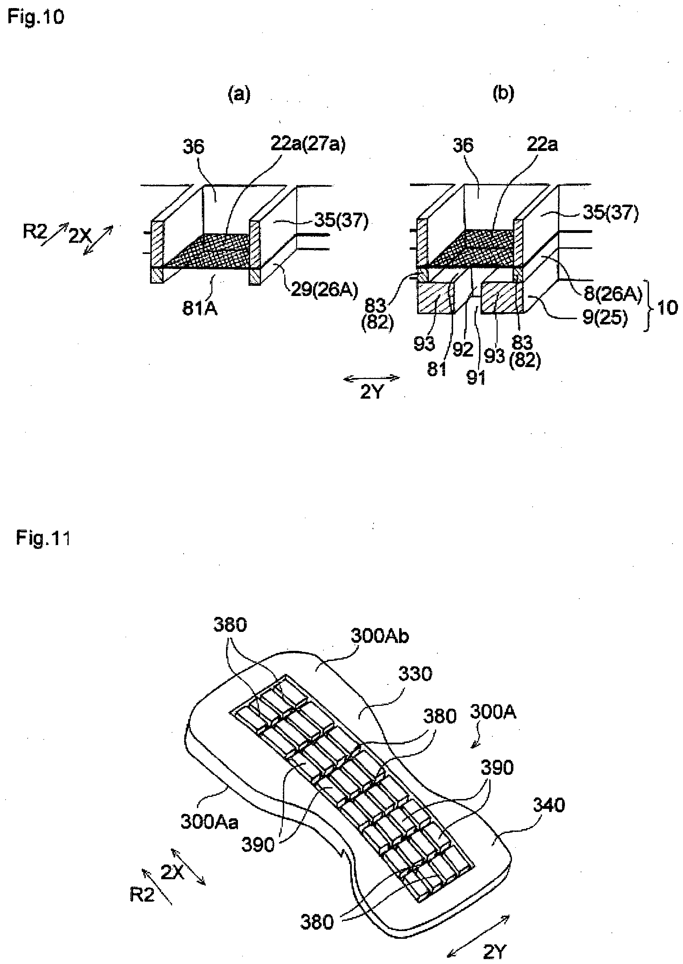

The invention (the first aspect of the invention) will be described below on the basis of preferred embodiments thereof with reference to the drawings. Fig. 1 illustrates the outline of a first embodiment of the fiber stacking device of the invention. A fiber stacking device 100 of the first embodiment includes a rotating drum 2 that is driven to rotate in the direction of arrow R2, a duct 11 that supplies a shaped-product material to an outer peripheral surface 21 of the rotating drum 2, a transfer roller 12 that is disposed obliquely below the rotating drum 2 and is driven to rotate in the direction of arrow R5, a vacuum conveyor 17 that is arranged below the transfer roller 12, and a cutter 7. The fiber stacking device 100 is further provided with a vacuum box 13 that is provided between the duct 11 and the transfer roller 12 in a circumferential direction of the rotating drum 2, a mesh belt 14 that is arranged so as to pass between the vacuum box 13 and the rotating drum 2 and between the transfer roller 12 and the rotating drum 2, and windshield plates 15 that are provided close to the outer peripheral surface of the transfer roller 12. Meanwhile, the vacuum box 13 and the windshield plates 15 are means for stably transferring a fiber stack, which is present in a collecting/stacking recess 22, without causing the fiber stack to lose its shape. When a fiber stack, which is relatively less likely to lose its shape like a fiber stack 300 illustrated in Fig. 7, is obtained, the vacuum box 13 and the windshield plates 15 may not be particularly provided or do not need to be used even if being provided. Meanwhile, for example, a fiber stack 300A illustrated in Fig. 11 includes recesses (grooves) 38 that have the shape of a lattice in planar view and protrusions 390 that are formed at the respective cells of the lattice, and is a fiber stack that is relatively likely to lose its shape. When the fiber stack that is likely to lose its shape is obtained, the vacuum box 13 and the windshield plates 15 are effective.

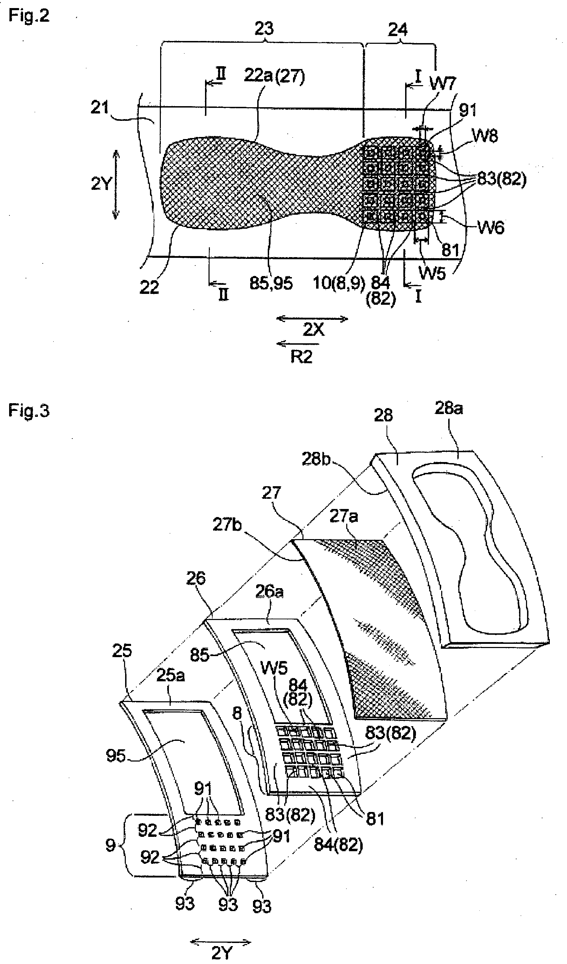

The rotating drum 2 is formed in a cylindrical shape as illustrated in Fig. 1, and rotates about a horizontal axis by receiving power from a prime mover, such as a motor. As illustrated in Fig. 2, the rotating drum 2 includes collecting/stacking recesses 22 which are formed on the outer peripheral surface 21 thereof and in which a shaped-product material are stacked. The plurality of recesses 22 are formed at a predetermined interval in the circumferential direction of the rotating drum 2 (a 2X direction). In Fig. 2, the 2X direction is the circumferential direction of the rotating drum 2 and a 2Y direction is a width direction of the rotating drum 2 (a direction parallel to the rotation axis of the rotating drum 2).

As illustrated in Fig. 3, the rotating drum 2 includes a cylindrical drum body (not illustrated) that is formed of a metallic rigid body, a suction adjusting plate 25 that is fixed so as to overlap an outer peripheral portion of the drum body, a space plate 26 that is fixed so as to overlap an outer surface 25a side of the suction adjusting plate 25, a porous plate 27 (a porous member) that is fixed so as to overlap an outer surface 26a side of the space plate 26, and a pattern forming plate 28 that is fixed so as to overlap an outer surface 27a side of the porous plate 27. These plates 25 to 28 are fixed to the drum body by known fixing means, such as bolts or an adhesive. A bottom surface 22a of the collecting/stacking recess 22, which is a surface on which a shaped-product material is stacked, is formed of the porous plate 27.

Meanwhile, in this specification, the outer surfaces of the respective components (the suction adjusting plate 25, the space plate 26, the porous plate 27, the pattern forming plate 28, and the like) of the rotating drum 2 are the surfaces of the components that face the supply side of a shaped-product material when the shaped-product material is stacked. Further, the inner surfaces of the respective components are the surfaces of the components that face the side opposite to the supply side of a shaped-product material (the inside of the rotating drum) when the shaped-product material is stacked. When a shaped product manufactured by the fiber stacking device 100 is an absorbent member used for an absorbent article, such as a disposable diaper or a sanitary napkin, the shaped-product material is an absorbent-core material.

The shaped-product material contains a fiber material. Various materials, which have been conventionally used for absorbent members of absorbent articles, such as a sanitary napkin or a panty liner and a disposable diaper, can be used as the shaped-product material, which serves as the absorbent-core material, without particular limitation. For example, pulp fiber such as fibrillated pulp, short fiber of cellulosic fiber, such as rayon fiber and cotton fiber, short fiber of synthetic fiber such as polyethylene, and the like are used. One of these fiber materials can be used alone or the combination of two or more thereof can be used. Further, a water-absorbent polymer and a fiber material may be used together as the absorbent-core material. Furthermore, as a fibrous material, a fibrous water-absorbent polymer can be used alone or can be used together with a fiber material. Moreover, a deodorizer, an antimicrobial agent, or the like can also be used together with a fiber material or the like, as necessary.

The pattern forming plate 28 includes an outer surface 28a that forms the outer peripheral surface 21 of the rotating drum 2 and an inner surface 28b that faces the rotation-axis side of the rotating drum 2, and includes a space portion that is formed between the outer surface 28a and the inner surface 28b and has a shape corresponding to the three-dimensional shape of the collecting/stacking recess 22. Portions of the pattern forming plate 28 except for the space portion have air impermeability where air does not pass. Here, "air impermeability" includes both of "air impermeability where air does not pass completely" and "sparing air permeability where minute amount of air passes but air does not pass substantially" and means a substantially air-impermeable property. For example, a plate in which an opening (a space portion having a shape corresponding to the three-dimensional shape of the recess 22) is formed by performing machining on a plate made of metal, such as stainless steel or aluminum, or a resin, a plate in which the opening is integrally formed by using a mold, a plate that has been subjected to punching or etching, a plate in which these plates overlap, or the like can be used as the pattern forming plate 28.

The porous plate 27 is an air-permeable plate which conveys an air stream (vacuum air), which is generated by suction from the inside of the device (the inside of the rotating drum 2), to the outside of the device (the outside of the rotating drum 2), holds a shaped-product material carried on the air stream without allowing the shaped-product material to pass therethrough, and allows only air to pass therethrough. A plurality of (many) suction holes (fine holes), which penetrate the plate 27 in a thickness direction, are formed with a uniform distribution in the entire porous plate 27, and the suction holes function as transmission holes for an air stream while the collecting/stacking recess 22 passes over a space in the rotating drum 2 that is maintained at negative pressure. For example, a mesh plate that is made of metal or a resin, a plate in which a plurality of (many) fine holes are formed by performing etching or punching on a plate made of metal or a resin, or the like can be used as the porous plate 27.

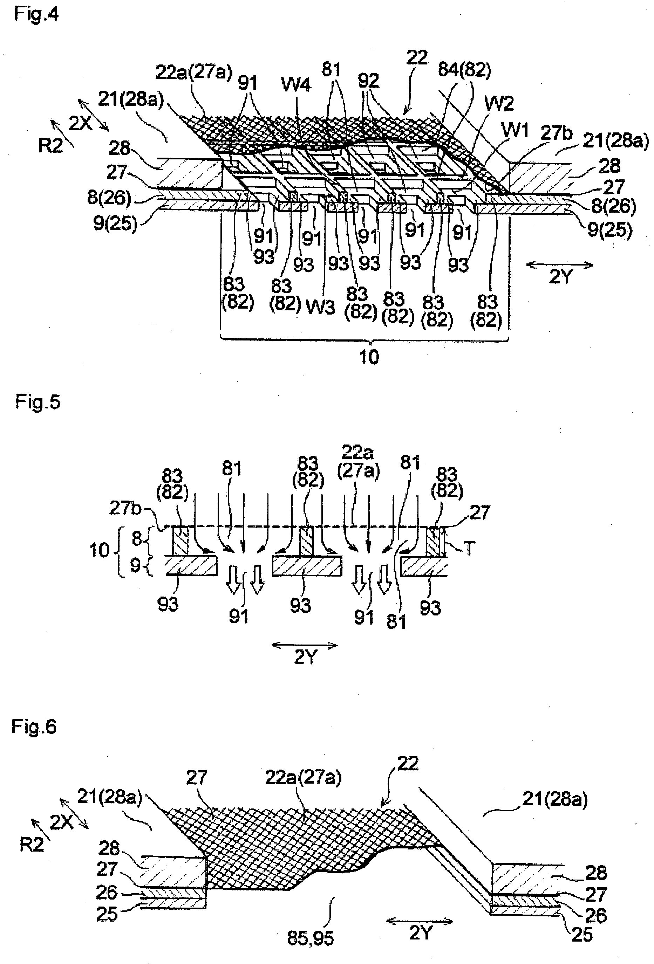

As illustrated in Figs. 3 and 4, an adjuster 10, which rectifies the air stream, is arranged on an inner surface 27b side of the porous plate 27 so as to overlap a part of the inner surface 27b of the plate 27. The adjuster 10 includes a first layer 8 and a second layer 9 that are laminated in this order from the porous plate 27. In the first embodiment, the adjuster 10 includes the suction adjusting plate 25 and the space plate 26. In a planar view of the collecting/stacking recess 22 as illustrated in Fig. 2, a part of a portion of the space plate 26 overlapping the bottom surface 22a of the collecting/stacking recess 22 forms the first layer 8, and a portion of the suction adjusting plate 25 overlapping the first layer 8 forms the second layer 9. Here, "planar view" refers to a view in which an object (the collecting/stacking recess, or the like) is viewed from the outside in a direction of a normal to the outer peripheral surface 21 of the rotating drum 2 (a direction orthogonal to the direction of the rotation axis of the rotating drum 2).

The first layer 8 includes a plurality of first openings 81 that penetrate the first layer 8 in the thickness direction and an opening defining section 82 that partitions and forms the first openings 81. The opening defining section 82 includes a plurality of MD defining members 83 that extend in a transporting direction R2 of the collecting/stacking recess 22 (the circumferential direction of the rotating drum 2 and the 2X direction), and a plurality of CD defining members 84 that extend in a direction orthogonal to the transporting direction (the width direction of the rotating drum 2 and the 2Y direction). Here, in relation to the MD defining members, "extend in a transporting direction of the collecting/stacking recess" means that the MD defining members have only to extend substantially in the transporting direction, is not limited to a case in which the MD defining members have the shape of a straight line parallel to the transporting direction in a planar view of the collecting/stacking recess 22, and includes a case in which the MD defining members are formed in the shape of a curve along the transporting direction and also includes a case in which the MD defining members are not parallel to the transporting direction but extend in a direction intersecting with the transporting direction at an angle of 45° or less. Further, in relation to the CD defining members, "extend in a direction orthogonal to the transporting direction of the collecting/stacking recess (an orthogonal direction)" means that the CD defining members have only to extend substantially in the orthogonal direction, is not limited to a case in which the CD defining members have the shape of a straight line parallel to the orthogonal direction in a planar view of the collecting/stacking recess 22, and includes a case in which the CD defining members are formed in the shape of a curve along the orthogonal direction and also includes a case in which the CD defining members are not parallel to the orthogonal direction but extend in a direction intersecting with the orthogonal direction at an angle of 45° or less.

In the first embodiment, as illustrated in Figs. 2 to 4, the opening defining section 82 includes six MD defining members 83 that are parallel to the transporting direction of the collecting/stacking recess 22 (the 2X direction) and have the shape of a straight line in planar view, and five CD defining members 84 that are parallel to the direction orthogonal to the transporting direction of the collecting/stacking recess 22 (the 2Y direction) and have the shape of a straight line in planar view. The opening defining section 82 is formed in the shape of a lattice in planar view by a total of ten defining members 83 and 84 that have the shape of a straight line in planar view. The first openings 81 are positioned at the respective cells of the lattice of the lattice-shaped opening defining section 82, and have a quadrangular shape in planar view. The opening defining section 82 (the defining members 83 and 84) has air impermeability where air does not pass. Here, "air impermeability" has the above-mentioned meaning. Metal, such as stainless steel, aluminum, or iron, a resin, or the like can be used as a material of the opening defining section 82 (the defining members 83 and 84) having air impermeability.

Second openings 91, which penetrate the second layer 9 in the thickness direction, are formed at portions of the second layer 9, which overlap the plurality of first openings 81 in a planar view of the collecting/stacking recess 22 as illustrated in Fig. 2, so as to correspond to the respective first openings 81. Accordingly, both the openings 81 and 91, which are in this correspondence, overlap each other in the planar view. In the first embodiment, as illustrated in Figs. 3 and 4, one second opening 91 of the second layer 9 corresponds to one first opening 81 of the first layer 8. Accordingly, the first openings 81 are in one-to-one correspondence with the second openings 91.

One of main features of the first embodiment is that the second opening 91 is present at a position separated from the CD defining members 84 in each of the plurality of first openings 81 in a planar view of the collecting/stacking recess 22 as illustrated in Fig. 2, and portions of the second layer 9 overlapping the CD defining members 84 (hereinafter, referred to as CD-defining-member corresponding sections) and the vicinity thereof have air impermeability where air does not pass. Here, "air impermeability" has the above-mentioned meaning. Reference numeral 92 in Fig. 3 denotes the CD-defining-member corresponding sections of the second layer 9 and the vicinity thereof. Like the other portions of the suction adjusting plate 25, the CD-defining-member corresponding sections and the vicinity thereof 92 are made of an air-impermeable material such as metal (for example, stainless steel, aluminum, or iron), or a resin, include no openings (through holes penetrating the second layer 9 in the thickness direction), and have air impermeability.

Here, "the vicinity of the CD-defining-member corresponding sections" is a region that extends along one CD defining member 84 over the total length of the one CD defining member 84 in the 2Y direction (the width direction of the rotating drum 2) in a planar view of the collecting/stacking recess 22 and has a constant width (length in the 2X direction) W1 (see Fig. 4). Further, the width of the CD-defining-member corresponding section (the length of the CD-defining-member corresponding section in the 2X direction) is the same as the width W2 of the corresponding CD defining member 84 (see Fig. 4).

Furthermore, the second layer 9 of the first embodiment includes air-impermeable sections through which air does not pass and which are formed at portions of the second layer 9 overlapping not only the CD defining members 84 but also the MD defining members 83 and in the vicinity thereof. That is, the second opening 91 is present at a position separated from the MD defining members 83 in each of the plurality of first openings 81 in a planar view of the collecting/stacking recess 22 as illustrated in Fig. 2, and portions of the second layer 9 overlapping the MD defining members 83 (hereinafter, referred to as MD-defining-member corresponding sections) and the vicinity thereof have air impermeability where air does not pass. Here, "air impermeability" has the above-mentioned meaning. Reference numeral 93 in Fig. 3 denotes the MD-defining-member corresponding sections of the second layer 9 and the vicinity thereof. Like the other portions of the suction adjusting plate 25, the MD-defining-member corresponding sections and the vicinity thereof 93 are made of an air-impermeable material such as metal (for example, stainless steel, aluminum, or iron), or a resin, include no openings (through holes penetrating the second layer 9 in the thickness direction)and have air impermeability.

Here, "the vicinity of the MD-defining-member corresponding sections" is a region that extends along one MD defining member 83 over the total length of the one MD defining member 83 in the 2X direction (the circumferential direction of the rotating drum 2) in a planar view of the collecting/stacking recess 22 and has a constant width (length in the 2Y direction) W3 (see Fig. 4). Further, the width of the MD-defining-member corresponding section (the length of the MD-defining-member corresponding section in the 2Y direction) is the same as the width W4 of the corresponding MD defining member 83 (see Fig. 4).

As described above, in the first embodiment, the second opening 91 is present at a position, which is separated from two CD defining members 84 and 84 facing each other and two MD defining members 83 and 83 facing each other, in each of the plurality of first openings 81 in a planar view of the collecting/stacking recess 22. Accordingly, the area of the second opening 91, which is present in one first opening 81 in a planar view of the recess 22, is smaller than that of the first opening 81. Here, the "areas" of both the openings 81 and 91 mean the areas of opening end portions of the openings (the first opening 81 and the second opening 91) that are closest to the porous plate 27 (the areas of portions of the openings coming into contact with the porous plate when the openings come into contact with the porous plate).

In the first embodiment, the first and second openings 81 and 91 overlap each other in a planar view of the collecting/stacking recess 22. Accordingly, it can be said that one opening, which penetrates the adjuster 10 (the first layer 8 and the second layer 9) in the thickness direction, is formed of both the openings 81 and 91 overlapping each other. Further, the area of the second opening 91, which is present in one first opening 81 in a planar view of the recess 22, is smaller than that of the first opening 81 as described above. Accordingly, considering this dimensional relationship between both the openings 81 and 91, it can be said that the adjuster 10 of the first embodiment includes a plurality of openings (openings formed of the first and second openings 81 and 91 that overlap each other in a planar view of the recess 22) penetrating the adjuster 10 in the thickness direction and the area of the opening end portion of the opening relatively distant from the porous plate 27 (the opening end portion of the second opening 91) is smaller than the area of the opening end portion thereof relatively close to the porous plate 27 (the opening end portion of the first opening 81).

Furthermore, in the first embodiment, the second opening 91 is formed at the center of each of the plurality of first openings 81 in a planar view of the collecting/stacking recess 22.

Moreover, in the first embodiment, in a planar view of the collecting/stacking recess 22, the shape of one first opening 81 in planar view and the shape of a second opening 91, which is present in the first opening 81, in planar view are similar to each other, and both the openings 81 and 91 have a quadrangular shape in planar view. That is, the ratio of similitude of the second opening 91 to the corresponding first opening 81 is lower than 1 in terms of planar-view shape of the openings 81 and 91.

When the adjuster 10 (the first and second layers 8 and 9) having the above-mentioned structure is arranged on the inner surface 27b side of the porous plate 27 that forms the bottom surface 22a of the collecting/stacking recess 22, the volume of air, which is obtained when an air stream (vacuum air) generated by suction from the inside of the device and sucking a shaped-product material flows through the porous plate 27, is reduced as compared to a case where the adjuster 10 is not arranged as in an adjuster-non-arrangement region (a high basis-weight fiber-stack region) 23 of the recess 22 to be described below. That is, as illustrated in Fig. 5, the air stream (illustrated by arrows in Fig. 5), which flows toward the inside from the outside of the rotating drum 2 through the porous plate 27, passes through the respective components of the rotating drum 2 in the order of the porous plate 27, the first layer 8, and the second layer 9 in the region, where the adjuster 10 is arranged, of the bottom section of the recess 22. However, since the area of the second opening 91 of the second layer 9, which is positioned on the leeward side on the air stream and substantially functions as a suction section for the shaped-product material, is smaller than that of the first opening 81 of the first layer 8 that is positioned on the windward side of the second opening 91 on the air stream, air permeability inherent in the porous plate 27 is inhibited in the region where the adjuster 10 is arranged. Accordingly, the volume of air of the air stream is reduced.

Further, in a fiber stacking step of sucking and stacking a shaped-product material, which is supplied on the air stream, in the collecting/stacking recess 22, the basis weight of the shaped-product material depends on the volume of air flowing through the porous plate 27. Accordingly, it is possible to manufacture a shaped product, of which the basis weight of a desired portion is reduced, with simple facilities by arranging the adjuster 10 in a region, which corresponds to a portion in which the basis weight of a shaped-product material to be stacked is intended to be smaller than those of other portions, of the inner surface 27b side of the porous plate 27. For example, when an absorbent member used for an absorbent article, such as a disposable diaper or a sanitary napkin, is manufactured as a shaped product, it is possible to obtain an absorbent member, which is excellent in both absorption performance and the reduction of inconvenience and discomfort in wearing, by concentrately stacking an absorbent-core material, such as pulp or a water-absorbent polymer, on a portion requiring high absorption capacity and reducing the basis weight of the other portions as much as possible by using the adjuster 10.

When the volume of air of the air stream flowing through the porous plate 27 forming the bottom surface 22a of the collecting/stacking recess 22 is reduced by the adjuster 10 as described above, a difference between the volume of air of the air stream (a suction force for sucking a shaped-product material) at a portion of the bottom surface 22a corresponding to the second opening 91 substantially functioning as the suction section for the shaped-product material (a portion of the bottom surface 22a overlapping the openings 91 in a planar view of the recess 22) and the vicinity thereof and the volume of air of the air stream at the other portions of the bottom surface 22a becomes significant. For this reason, since a difference in the amount of a shaped-product material to be stacked between both the portions becomes significant, there is a concern that fiber-stacking irregularity caused by a difference in the amount of a shaped-product material to be stacked may occur in a region where the adjuster 10 is arranged and the amount of a shaped-product material to be stacked should be originally uniform. However, in the first embodiment, the first openings 81, each of which has an area larger than the area of the second opening 91, are provided on the windward side of the second opening 91 on the air stream. Accordingly, since a space, which has a predetermined size and is formed by the first openings 81, is formed between the porous plate 27 and the second opening 91, an air stream having passed through the porous plate 27 is rectified while passing this space before passing through the second openings 91. As a result, a variation in the volume of air of an air stream flowing through the porous plate 27 is reduced and the occurrence of fiber-stacking irregularity is effectively prevented.

Further, as described below, a plurality of spaces (spaces B to D) partitioned from one another are formed in the rotating drum 2, a part (the space B) of the plurality of spaces is maintained at negative pressure, the collecting/stacking recess 22 is transported in an R2 direction by the rotation of the rotating drum 2 in the R2 direction, and an air stream flows through the bottom surface 22a of the recess 22 toward the inside from the outside of the drum while the collecting/stacking recess 22 passes over the space B maintained at negative pressure. If a structure in which the second openings 91 overlap the CD defining members 84 in a planar view of the recess 22, a structure in which the CD-defining-member corresponding sections of the second layer 9 and the vicinity thereof 92 form air-permeable sections through which air passes, or the like is employed, the plurality of first openings 81 communicate with one another so that an air stream can flow in the transporting direction R2 of the recess 22 (the circumferential direction of the rotating drum 2 and the 2X direction) through the second openings 91 overlapping the CD defining members 84 or the air-permeable sections. In this case, before the first openings 81 positioned on the relatively rear side in the transporting direction R2 (rear first openings 81) pass over a space maintained at negative pressure, turbulence is generated in the rear first openings 81 by the influence of an air stream flowing through the front first openings 81 when the first openings 81 positioned on the relatively front side in the transporting direction R2 (front first openings 81) pass over the space. Accordingly, a rectification effect obtained from the above-mentioned first openings 81 is impaired. For this reason, there is a concern that the occurrence of fiber-stacking irregularity may not be prevented.

In contrast, in the first embodiment, as described above, the second opening 91 is present at a position, which is separated from the CD defining members 84, in each of the plurality of first openings 81 in a planar view of the collecting/stacking recess 22, and the CD-defining-member corresponding sections of the second layer 9 and the vicinity thereof 92 form the air-impermeable sections through which air does not pass. Accordingly, the plurality of first openings 81 do not communicate with one another so that an air stream cannot flow in the transporting direction R2 of the recess 22. For this reason, before the rear first openings 81 pass over a space of the rotating drum 2 maintained at negative pressure, turbulence is not generated in the rear first openings 81. Therefore, a rectification effect obtained from the above-mentioned first openings 81 is reliably achieved.

Particularly, in the first embodiment, as described above, the second openings 91 are present at positions that are separated from not only the CD defining members 84 but also the MD defining members 83 extending in a direction orthogonal to the CD defining members 84, and the portions of the second layer 9 overlapping the opening defining sections 82 and the vicinity thereof (92, 93) form air-impermeable sections. Accordingly, the plurality of first openings 81 do not communicate with one another so that an air stream cannot flow in both the transporting direction R2 of the collecting/stacking recess 22 and a direction orthogonal to the transporting direction R2, and the independence of the space formed by each of the respective first openings 81 is ensured. Therefore, a rectification effect obtained from the above-mentioned first openings 81 is achieved more reliably.

Further, in the first embodiment, as described above, the second opening 91 is formed at the center of each of the plurality of first openings 81 in a planar view of the collecting/stacking recess 22 and the shape of one first opening 81 in planar view and the shape of the second opening 91, which is present in the first opening 81, in planar view are similar to each other in a planar view of the recess 22. The structure of these openings is effective in stably achieving a rectification effect obtained from the above-mentioned first openings 81.

From the viewpoint of more effectively preventing fiber-stacking irregularity by reliably achieving a rectification effect that is obtained from the above-mentioned first openings 81, it is preferable that dimensions and the like of the respective portions of the device be set in the following ranges.

The width W1 (see Fig. 4) of a portion of the second layer 9 in the vicinity of the CD-defining-member corresponding section (a portion of the second layer 9 overlapping the CD defining member 84 in a planar view of the collecting/stacking recess 22) is preferably 1 mm or greater and more preferably 2 mm or greater, and preferably 10 mm or less and more preferably 5 mm or less. More specifically, the width W1 of a portion of the second layer 9 in the vicinity of the CD-defining-member corresponding section is preferably in the range of 1 to 10 mm and more preferably in the range of 2 to 5 mm.

The width W2 (see Fig. 4) of the CD defining member 84 of the first layer 8 is preferably 0.5 mm or greater and more preferably 1 mm or greater, and preferably 5 mm or less and more preferably 2 mm or less. More specifically, the width W2 (see Fig. 4) of the CD defining member 84 is preferably in the range of 0.5 to 5 mm and more preferably in the range of 1 to 2 mm.

The width W3 (see Fig. 4) of a portion of the second layer 9 in the vicinity of the MD-defining-member corresponding section (a portion of the second layer 9 overlapping the MD defining member 83 in a planar view of the collecting/stacking recess 22) is preferably 1 mm or greater and more preferably 2 mm or greater, and preferably 10 mm or less and more preferably 5 mm or less. More specifically, the width W3 of a portion of the second layer 9 in the vicinity of the MD-defining-member corresponding section is preferably in the range of 1 to 10 mm and more preferably in the range of 2 to 5 mm.

The width W4 (see Fig. 4) of the MD defining member 83 of the first layer 8 is preferably 0.5 mm or greater and more preferably 1 mm or greater, and preferably 5 mm or less and more preferably 2 mm or less. More specifically, the width W4 of the MD defining member 83 is preferably in the range of 0.5 to 5 mm and more preferably in the range of 1 to 2 mm.

The length W5 (see Fig. 2) of the first opening 81 in the 2X direction is preferably 5 mm or greater and more preferably 10 mm or greater, and preferably 30 mm or less and more preferably 25 mm or less. More specifically, the length W5 of the first opening 81 in the 2X direction is preferably in the range of 5 to 30 mm and more preferably in the range of 10 to 25 mm.

The length W6 (see Fig. 2) of the first opening 81 in the 2Y direction is preferably 5 mm or greater and more preferably 10 mm or greater, and preferably 20 mm or less and more preferably 15 mm or less. More specifically, the length W6 of the first opening 81 in the 2Y direction is preferably in the range of 5 to 20 mm and more preferably in the range of 10 to 15 mm.

The length W7 (see Fig. 2) of the second opening 91 in the 2X direction is preferably 2 mm or greater and more preferably 5 mm or greater, and preferably 30 mm or less and more preferably 25 mm or less. More specifically, the length W7 of the second opening 91 in the 2X direction is preferably in the range of 2 to 30 mm and more preferably in the range of 5 to 25 mm.

The length W8 (see Fig. 2) of the second opening 91 in the 2Y direction is preferably 2 mm or greater and more preferably 5 mm or greater, and preferably 20 mm or less and more preferably 15 mm or less. More specifically, the length W8 of the second opening 91 in the 2Y direction is preferably in the range of 2 to 20 mm and more preferably in the range of 5 to 15 mm.

A ratio (S2/S1) of the area S2 of the second opening 91 to the area S1 of the first opening 81 is preferably in the range of 5 to 50% and more preferably in the range of 7 to 15%.

The thickness T (see Fig. 5) of the opening defining section 82 (the MD defining member 83 and the CD defining member 84) of the first layer 8 is preferably 2 mm or greater and more preferably 3 mm or greater, and preferably 10 mm or less and more preferably 5 mm or less. More specifically, the thickness T of the opening defining section 82 is preferably in the range of 2 to 10 mm and more preferably in the range of 3 to 5 mm.

In the first embodiment, the adjuster 10 (the first and second layers 8 and 9) is not arranged so as to correspond to the entire region of the porous plate 27, is arranged only in a region, which corresponds to the rear portion of the collecting/stacking recess 22 in the transporting direction R2, of the inner surface 27b side of the porous plate 27 as illustrated in Fig. 2, and is not arranged in the other regions including the front portion of the porous plate 27. That is, the collecting/stacking recess 22 includes an adjuster-arrangement region 24 where the adjuster 10 is arranged on the inner surface 27b side of the porous plate 27 forming the bottom surface 22a of the recess 22 and the adjuster-non-arrangement region 23 where the adjuster 10 is not arranged on the inner surface 27b side of the porous plate 27.

The adjuster-non-arrangement region 23 of the recess 22 will be further described. As illustrated in Fig. 3, a first large opening 85, which penetrates the space plate 26 in the thickness direction and has an area larger than the area of the first opening 81 and a rectangular shape in planar view, is formed at a portion of the space plate 26 except for the first layer 8, and a second large opening 95, which penetrates the suction adjusting plate 25 in the thickness direction and has a rectangular shape in planar view, is formed at the suction adjusting plate 25 so as to correspond to the first large opening 85. The shapes of both the openings 85 and 95 in planar view are congruent with each other, and a ratio of similitude of the second large opening 95 to the first large opening 85 is 1.

Further, since each of both the openings 85 and 95 have a size overlapping the entire adjuster-non-arrangement region 23 of the recess 22 in a planar view of the collecting/stacking recess 22, substantially the entire region of the inner surface 27b of the porous plate 27 corresponds to both the openings 85 and 95 as illustrated in Fig. 6 in the adjuster-non-arrangement region 23. Accordingly, in the adjuster-non-arrangement region 23 of the recess 22, air permeability inherent in the porous plate 27 is not inhibited and the volume of air of an air stream (vacuum air), which is generated by suction from the inside of the device and sucks a shaped-product material, is not reduced. For this reason, the shaped-product material is stacked in the adjuster-non-arrangement region 23 with a basis weight that is higher than the basis weight of the shaped-product material stacked in the adjuster-arrangement region 24 of the recess 22.

As described above, the collecting/stacking recess 22 of the first embodiment includes the adjuster-non-arrangement region (the high basis-weight fiber-stack region) 23 in which a shaped-product material is stacked with a relatively high basis weight, and the adjuster-arrangement region (a low basis-weight fiber-stack region) 24 in which a shaped-product material is stacked with a relatively low basis weight, and the adjuster-non-arrangement region (the high basis-weight fiber-stack region) 23 and the adjuster-arrangement region (the low basis-weight fiber-stack region) 24 are arranged in the transporting direction R2 (a longitudinal direction) of the recess 22 (see Fig. 2). In the adjuster-non-arrangement region (the high basis-weight fiber-stack region) 23, the adjuster 10 is not arranged on the inner surface 27b side of the porous plate 27 forming the bottom surface 22a of the collecting/stacking recess 22. In the adjuster-arrangement region (the low basis-weight fiber-stack region) 24, the adjuster 10 is arranged on the inner surface 27b side of the porous plate 27.

The fiber stacking device 100 of the first embodiment will be further described. As illustrated in Fig. 1, the spaces B, C, and D, which are partitioned from one another in the circumferential direction of the rotating drum 2 (the 2X direction), are formed on the inside (rotation-axis side) of the rotating drum 2. A known exhaust device (not illustrated), such as an air-suction fan, is connected to the space B, and the space B can be maintained at negative pressure through the actuation of the exhaust device. Outside air flows into the space C by suction from the vacuum box 13 to be described below, and outside air flows into the space D by suction from the transfer roller 12. For the successful transfer performed on the space C (the transfer of the fiber stack, which is present in the recess 22, onto the transfer roller 12 and the like), the space C is divided from the space D that corresponds to a region having been subjected to transfer. The rotating drum 2 may include means for generating blow (air stream), which is directed to the bottom surface 22a of the recess 22 (the porous plate 27) from the inside of the rotating drum 2, in a space (a space C) corresponding to a transfer position where the fiber stack present in the recess 22 is transferred onto the transfer roller 12. In this case, it is possible to facilitate the release of the fiber stack from the recess 22 by actively blowing air to the vacuum box 13 from the space C with the means for generating blow. Meanwhile, one end of the rotating drum 2 in the direction of the rotation axis of the rotating drum 2 is closed by a plate that rotates integrally with the rotating drum 2, and the other end thereof is airtightly closed by a plate that does not rotate. Further, the spaces B to D are partitioned from one another by plates that are provided from the rotation-axis side of the rotating drum 2 toward the inner surface of the rotating drum 2.

Meanwhile, the pressure of the space C is generally set to negative pressure, which is lower than the pressure of the space B, or zero pressure (atmospheric pressure). Until a fiber stack, which is present in the collecting/stacking recess 22, is transferred onto the transfer roller 12, from the viewpoint of the transport property of the fiber stack, it is preferable that the space C be maintained at low negative pressure and the fiber stack be sucked and held in the recess 22. However, if there is no particular problem in a transport property, it is preferable that the space C be maintained at zero pressure in consideration of a transfer property. Further, since the space D is a region over which the recess 22 passes after the fiber stack present in the recess 22 is transferred onto the transfer roller 12, it is preferable that the space D be maintained at zero pressure or positive pressure.

As illustrated in Fig. 1, one end side of the duct 4 covers the outer peripheral surface of the rotating drum 2 positioned on the space B, and the duct 11 includes a shaped-product material introduction device on the other side thereof (not illustrated). The shaped-product material introduction device includes, for example, a pulverizer that pulverizes a wood pulp sheet into fibrillated pulp and sends the fibrillated pulp (a fiber material) into the duct 11. A water-absorbent polymer introduction unit, which introduces water-absorbent polymer particles, may be provided on the duct 11.

The transfer roller 12 has an air-permeable cylindrical outer peripheral portion, and the outer peripheral portion rotates about a horizontal axis by receiving power from a prime mover, such as a motor. A space E of which inside pressure can be reduced is formed in the non-rotating section inside the transfer roller 512 (the rotation-axis side). A known exhaust device (not illustrated), such as an air-suction fan, is connected to the space E, and the space E can be maintained at negative pressure through the actuation of the exhaust device.

A plurality of (many) suction holes for communication between the inside and the outside of the transfer roller are formed in the outer peripheral surface of the transfer roller 12. While passing over the space E maintained at negative pressure, the suction holes suck air to the inside from the outside and the fiber stack present in the recess 22 is smoothly transferred from the rotating drum 2 onto the transfer roller 12 by this suction force of the air.

The vacuum conveyor 17 includes an endless air-permeable belt 173 that is spanned between a drive roller 171 and driven rollers 172 and 172, and a vacuum box 174 that is arranged at a position facing the transfer roller 12 across the air-permeable belt 173.

The vacuum box 13 has a box-like shape having upper and lower surfaces, left and right side surfaces, and a rear surface, and includes an opening that opens toward the rotating drum 2. A known exhaust device (not illustrated), such as an air-suction fan, is connected to the vacuum box 13 through an exhaust pipe (not illustrated), and the inside of the vacuum box 13 can be maintained at negative pressure through the actuation of the exhaust device. The mesh belt 14 is a member that is formed by endlessly connecting a band-shaped air-permeable belt having meshes, and moves continuously along a predetermined route by being guided by a plurality of free rollers 16 and the transfer roller 12. The mesh belt 14 is driven by the rotation of the transfer roller 12. As illustrated in Fig. 1, the mesh belt 14 is arranged so as to sequentially pass between the vacuum box 13 and the rotating drum 2 and between the transfer roller 12 and the rotating drum 2 after being introduced onto the outer peripheral surface of the rotating drum 2 in the vicinity of a downstream end 11a of the duct 11. The mesh belt 14 comes into contact with the outer peripheral surface of the rotating drum 2 while passing the front of the opening of the vacuum box 13, and the mesh belt is separated from the outer peripheral surface of the rotating drum 2 and moves onto the transfer roller 12 in the vicinity of a portion where the transfer roller 12 and the rotating drum 2 come nearest to each other.

The mesh belt 14 includes fine holes that are smaller than the suction holes of the transfer roller 12, and suction from the fine holes of the mesh belt 14, which overlap the suction holes of the transfer roller 12, is also performed with suction from the suction holes of the transfer roller 12. The pair of windshield plates 15 is provided on both sides of a region, in which the suction holes are formed, of the outer peripheral surface of the transfer roller 12 in the width direction. The windshield plates 15 prevent the fiber stack, which has been released from the recess 22, from losing its shape by preventing or reducing the inflow of air from the sides. The material of the windshield plate 15 is not particularly limited, but it is preferable that the material of the windshield plate 15 be metal or a synthetic resin and the thickness of the windshield plate 15 be in the range of about 0.5 to 10 mm from the viewpoint of stiffness capable of resisting air.

Devices that have been conventionally used to cut a continuous absorbent member body in the manufacture of absorbent articles, such as sanitary napkins and diapers, and the like can be used as the cutter 7 without limitation. The cutter 7 illustrated in Fig. 1 includes a cutter roller 72 that includes cutting blades 71 on the peripheral surface thereof and an anvil roller 73 which receives the cutting blades and of which the peripheral surface is smooth.

Next, a method of continuously manufacturing absorbent members by using the above-mentioned fiber stacking device 100, that is, an embodiment of a method of manufacturing an absorbent member of the invention (the first aspect of the invention) will be described. The manufacturing method of this embodiment includes a fiber stacking step of sucking and stacking an absorbent-core material (a shaped-product material), which is supplied on an air stream, in the collecting/stacking recess 2 of the rotating drum 2 in the fiber stacking device 100.

Before the fiber stacking step is performed, first, the pressure of the space B formed in the rotating drum 2, the pressure of the space E formed in the transfer roller 12, and the inside pressure of the vacuum box 13 are reduced to negative pressure through the actuation of the exhaust devices connected thereto. When the pressure of the space B is reduced to negative pressure, an air stream (vacuum air) for transporting the absorbent-core material onto the outer peripheral surface 21 of the rotating drum 2 is generated in the duct 11. Further, the vacuum conveyor 17 is actuated by the rotation of the rotating drum 2 and the transfer roller 12.

Then, when the shaped-product material introduction device is actuated and the absorbent-core material is supplied into the duct 11, the absorbent-core material floats on the air stream flowing in the duct 11 and is supplied toward the outer peripheral surface 21 of the rotating drum 2 in a dispersed airborne state.

While the collecting/stacking recess 22 of the rotating drum 2 is being transported along the portion covered with the duct 11, the absorbent-core material is sucked and stacked in the collecting/stacking recess 22 of the rotating drum 2. While each of the recesses 22 of the rotating drum 2 passes over the space B maintained at negative pressure, suction from the bottom surface 22a is performed. An air stream for transporting the absorbent-core material, which is introduced from the shaped-product material introduction device or the water-absorbent polymer introduction unit, onto the outer peripheral surface 21 of the rotating drum 2 is generated in the duct 11 by suction from the suction holes of the porous plate 27 forming the bottom surface 22a; and the absorbent-core material transported on the air stream is stacked in the recess 22. As described above, the amount of absorbent-core material to be stacked in the recess 22 is set to be different according to whether or not the adjuster 10 is arranged. Accordingly, the absorbent-core material is stacked in the adjuster-non-arrangement region (the high basis-weight fiber-stack region) 23 with a relatively high basis weight and is stacked in the adjuster-arrangement region (the low basis-weight fiber-stack region) 24 with a relatively low basis weight (see Fig. 2). When the recess 22 passes over the downstream end 11a of the duct 11, the adjuster-non-arrangement region 23 is completely covered with the absorbent-core material and the adjuster-arrangement region 24 is not completely covered with the absorbent-core material. For this reason, a difference in level, which is caused by a difference in the stacked amount of the absorbent-core material, is formed at a boundary portion between the adjuster-non-arrangement region 23 and the adjuster-arrangement region 24 on the surface of the fiber stack that is present in the recess 22 (the surface of the fiber stack that is opposite to the surface of the fiber stack coming into contact with the bottom surface 22a of the recess 22). Accordingly, the surface of the adjuster-arrangement region 24 is present at a position lower than the surface of the adjuster-non-arrangement region 23.

After the absorbent-core material is stacked in the collecting/stacking recess 22 as described above and a fiber stack 300 is obtained, the rotating drum 2 is further rotated. Further, when the fiber stack 300 present in the recess 22 reaches a position facing the vacuum box 13, the fiber stack 300 is sucked onto the mesh belt 14 by suction from the vacuum box 13. In this state, the fiber stack 300 is transported to a portion where the transfer roller 12 and the rotating drum 2 come nearest to each other or to the vicinity thereof. Then, the fiber stack 300, which has been sucked onto the mesh belt 14, is released from the recess 22 by suction from the transfer roller 12, and is transferred onto the transfer roller 12 together with the mesh belt 13.

Fig. 7 illustrates the fiber stack 300 that has just been released from the collecting/stacking recess 22 of the first embodiment. As illustrated in Fig. 7, a portion of the fiber stack 300 corresponding to the adjuster-non-arrangement region (the high basis-weight fiber-stack region) 23 of the recess 22 is a high basis-weight section (a thick section) 330 in which the stacked amount of the absorbent-core material is relatively large, and a portion of the fiber stack 300 corresponding to the adjuster-arrangement region (the low basis-weight fiber-stack region) 24 of the recess 22 is a low basis-weight section (a thin section) 340 in which the stacked amount of the absorbent-core material is relatively small. Further, the entire one surface 300b of the fiber stack 300 (the surface of the fiber stack 300 coming into contact with the bottom surface 22a of the recess 22) is substantially flat. On the other hand, the other surface 300a (the surface of the fiber stack 300 that is opposite to the surface of the fiber stack 300 coming into contact with the bottom surface 22a of the recess 22) has a difference in level at a boundary portion between the high basis-weight section 330 and the low basis-weight section 340, and the other surface 300a is not flat.

The fiber stack 300 transferred onto the transfer roller 12 is transported while being sucked from the transfer roller 12, and is then passed on to a core-wrap sheet 500 that has been introduced onto the vacuum conveyor 17 arranged below the transfer roller 12 and is made of tissue paper, liquid-permeable nonwoven fabric, or the like. After that, as illustrated in Fig. 1, both side portions of the core-wrap sheet 500 extending in the transporting direction are folded back, and both the upper and lower surfaces of the fiber stack 300 are covered with the core-wrap sheet 500. Then, the fiber stack 300, which is covered with the core-wrap sheet 500, is cut to a predetermined size together with the core-wrap sheet 500 by the cutter roller 72 of the cutter 7. As a result, an absorbent member 30 covered with the core-wrap sheet 500 is obtained.

The absorbent member 30 is an absorbent member which includes the high basis-weight section 330 and the low basis-weight section 340 and in which the stacked amount of the absorbent-core material is partially different. The high basis-weight section 330 is stacked in the adjuster-non-arrangement region (the high basis-weight fiber-stack region) 23 of the recess 22 and has a relatively high basis weight, and the low basis-weight section 340 is stacked in the adjuster-arrangement region (the low basis-weight fiber-stack region) 24 and has a relatively low basis weight. Since fiber-stacking irregularity does not occur in the absorbent member 30 (particularly, the low basis-weight section 340) due to the operation of the above-mentioned adjuster 10, the absorbent member 30 constitutes a high-quality absorbent member suitable as an absorbent member used for absorbent articles, such as disposable diapers, sanitary napkins, and incontinence pads. Particularly, from the viewpoint of the exhibition of the maximum performance of the absorbent member 30, it is preferable that the absorbent member 30, which is built in an absorbent article so that the high basis-weight section 330 of the absorbent member 30 forms a ventral (front) portion of the absorbent article and the low basis-weight section 340 forms a dorsal (rear) portion of the absorbent article, be used as an absorbent member used for a disposable diaper. When the high basis-weight section and the low basis-weight section having different basis weights (the stacked amount of a material) are formed in the absorbent member as described above, an advantage of obtaining an absorbent member which is flexible and of which a wearing feeling is improved, and the like are obtained. Particularly, an absorbent member in which low basis-weight sections are formed on the front and rear of or around a high basis-weight section is flexible and is excellent in a wearing feeling.

Another embodiment of the invention (the first aspect of the invention) will be described below. Components of another embodiment to be described below, which are different from the components of the above-mentioned first embodiment, will be mainly described. The same components of another embodiment as those of the first embodiment will be denoted by the same reference numerals and the description thereof will be omitted. The description given for the above-mentioned first embodiment is appropriately applied to components that are not particularly described below.

Fig. 8 illustrates a main part of a second embodiment of the fiber stacking device of the invention, and Fig. 9 illustrates an exploded perspective view of the main part. In a fiber stacking device of the second embodiment, a recess partitioning member 35, which partitions a collecting/stacking recess 221 into a plurality of regions in a direction parallel to a bottom surface 22a, is arranged so as to overlap at least a part of an outer surface 27a of a porous plate 27 that forms the bottom surface 22a of the collecting/stacking recess 221.

A space plate 26A of the second embodiment includes a narrow section 29 and a wide section arranged in the 2X direction (the circumferential direction of the rotating drum 2). The narrow section 29 has a relatively short length (width) in the 2Y direction (the width direction of the rotating drum 2), and the wide section has a relatively long length (width) in the 2Y direction. The wide section forms the first layer 8 of the adjuster 10. The narrow section 29 is formed in the shape of a lattice so as to have the same opening pattern as that of the first layer 8 (the wide section), and includes a plurality of (many) openings 81A that have the same shape and dimensions as those of a first opening 81 of the first layer 8. The narrow section 29 has a rectangular shape in planar view, and is positioned at the middle of the space plate 26A in the 2Y direction. The entire narrow section 29 overlaps the second large opening 95 of the suction adjusting plate 25 in a planar view of the collecting/stacking recess 221.

The recess partitioning member 35 includes a plurality of openings 36 that penetrate the recess partitioning member 35 in the thickness direction, and an opening defining section 37 that partitions and forms the openings 36. The opening defining section 37 is formed in the shape of a lattice in planar view. The respective openings 36 are positioned at the respective cells of the lattice of the lattice-shaped opening defining section 37, and have a quadrangular shape in planar view. The opening defining section 37 has air impermeability where air does not pass. Here, "air impermeability" has the above-mentioned meaning. Metal, such as stainless steel, aluminum, or iron, a resin, the combination thereof, or the like can be used as a material of the opening defining section 37 having air impermeability. The opening defining section 37 has a rectangular shape in planar view, and is arranged at the middle portion of the bottom surface 22a of the collecting/stacking recess 221 in the 2Y direction so that the longitudinal direction of the opening defining section 37 corresponds to the 2X direction. The total length of the recess partitioning member 35 in the 2X direction is shorter than that of the space plate 26A in the 2X direction, and the length (width) of the recess partitioning member 35 in the 2Y direction is the same as that of the narrow section 29 of the space plate 26A in the 2Y direction. The opening pattern of the recess partitioning member 35 is the same as that of the space plate 26A [the narrow section 29, and the first layer (the wide section)], and the shapes and dimensions of the openings 36 are the same as those of the openings 81 and 81A of the space plate 26A. The recess partitioning member 35 is fixed onto the outer surface 27a of the porous plate 27 by known fixing means, such as bolts or an adhesive, so as to completely correspond to the narrow section 29 in a planar view of the collecting/stacking recess 221.

As illustrated in Fig. 10(a), one opening 36 of the recess partitioning member 35 is in one-to-one correspondence with one opening 81A of the narrow section 29 of the space plate 26A at a part (a middle portion thereof in the 2Y direction) of an adjuster-non-arrangement region (the high basis-weight fiber-stack region) 23, in which the adjuster 10 is not arranged and a shaped-product material is stacked with a relatively high basis weight, of the collecting/stacking recess 221. Further, as illustrated in Fig. 10(b), one opening 36 of the recess partitioning member 35 is in one-to-one correspondence with each of one first opening 81 of the first layer 8 (the wide section) of the space plate 26A and one second opening 91 of the second layer 9 of the suction adjusting plate 25 in an adjuster-arrangement region (the low basis-weight fiber-stack region) 24 in which the adjuster 10 is arranged and a shaped-product material is stacked with a relatively low basis weight. That is, in the adjuster-arrangement region 24, the openings 36 of the recess partitioning member 35 and openings, which penetrate the adjuster 10 in the thickness direction, (openings formed of both the openings 81 and 91 that overlap each other) overlap each other in a planar view of the recess 221.

The fiber stacking device of the second embodiment, which includes the rotating drum including the collecting/stacking recesses 221 having the above-mentioned structure, can be used in the same manner as the above-mentioned fiber stacking device of the first embodiment, and can continuously manufacture absorbent members according to the above-mentioned manufacturing method. While the recess 221 passes over the space B (see Fig. 1) of the rotating drum 2 maintained at negative pressure, suction from the bottom surface 22a is performed as usual at portions of the bottom surface 22a of the recess 221 that correspond to the openings 36 of the recess partitioning member 35, but suction from the bottom surface 22a is not performed at a portion of the bottom surface 22a that corresponds to the opening defining section 37 of the recess partitioning member 35 (a portion of the bottom surface 22a coming into contact with the opening defining section 37) since the opening defining section 37 has air impermeability. However, in the second embodiment, an absorbent-core material is stacked not only in the opening 36 but also on the opening defining section 37. On the upstream side of a duct 11, the absorbent-core material is stacked only in the openings 36, but the absorbent-core material starts to be also stacked on the opening defining section 37 in accordance with the intertwining among pieces of the absorbent-core material and the flow of air, which is present in the duct 11 and transports the absorbent-core material, when the height of the stacked absorbent-core material reaches the thickness of the opening defining section 37 (the recess partitioning member 35). When the recess 221 passes over the downstream end 11a of the duct 11, the opening defining section 37 is completely covered with the absorbent-core material.

Fig. 11 illustrates the fiber stack 300A that has just been released from the collecting/stacking recess 221 in the above-mentioned manufacturing method. As illustrated in Fig. 11, a portion of the fiber stack 300A corresponding to the adjuster-non-arrangement region (the high basis-weight fiber-stack region) 23 of the recess 331 constitutes a high basis-weight section (a thick section) 330 in which the stacked amount of the absorbent-core material is relatively large, and a portion of the fiber stack 300A corresponding to the adjuster-arrangement region (the low basis-weight fiber-stack region) 24 of the recess 221 constitutes a low basis-weight section (a thin section) 340 in which the stacked amount of the absorbent-core material is relatively small. Further, as illustrated in Fig. 11, a plurality of grooves (recesses) 380, which extend in both the 2X direction and the 2Y direction and have the shape of continuous straight lines in planar view, are arranged in the shape of a lattice on one surface 300Ab of the fiber stack 300A (the surface of the recess 221 coming into contact with the bottom surface 22a) so as to correspond to the lattice-shaped opening defining section 37 of the recess partitioning member 35, and protrusions 390, which have a quadrangular shape in planar view, are arranged at cells of the lattice so as to correspond to the plurality of openings 36 of the recess partitioning member 35. Accordingly, one surface 300Ab includes a projecting-and-recessed section with large undulations. A region of one surface 300Ab on which the projecting-and-recessed section including the grooves 380 and the protrusions 390 is not formed (a region present around the projecting-and-recessed section) is substantially flush with the tops of the protrusions 390. Meanwhile, the other surface 300Aa of the fiber stack 300A (the surface of the fiber stack 300A that is opposite to the surface of the fiber stack 300A coming into contact with the bottom surface 22a of the recess 221) has a difference in level at a boundary portion between the high basis-weight section 330 and the low basis-weight section 340 like the other surface 300a of the fiber stack 300 illustrated in Fig. 7, and the other surface 300Aa is not flat.

As described above, the fiber stack 300A includes the thick section (a high basis-weight section) 33 and the thin section (a low basis-weight section) 34 formed according to whether or not the adjuster 10 is arranged, and includes the projecting-and-recessed section, which includes the protrusions 390 (high basis-weight section) and the grooves 380 (a low basis-weight section) and corresponds to the opening pattern of the recess partitioning member 35, on one surface 300Ab of each of the thick section 33 and the thin section 34 (the surface of each of the thick section 33 and the thin section 34 coming into contact with the bottom surface 22a of the recess 221). When the absorbent member including the projecting-and-recessed section (an absorbent member having a surface that is not flat) is pressed by a press machine or the like and the thickness of the absorbent member, particularly, the thickness of the protrusions (a high basis-weight section) is actively reduced, a difference and/or a ratio between the thickness of the protrusion (a high basis-weight section) and the thickness of the groove (a low basis-weight section) is reduced. Accordingly, the absorbent member in which the protrusions (high basis-weight section) constitute a high-density section and the grooves (a low basis-weight section) constitute a low-density section is obtained. Since the absorbent member in which density is partially different, for example, allows body fluid to quickly pass through the low-density section and allows the high-density section to absorb and hold body fluid, the absorption performance of the absorbent member is improved. Further, since fiber-stacking irregularity does not occur in the fiber stack 300A (particularly, the low basis-weight section 340) due to the operation of the above-mentioned adjuster 10, the fiber stack 300A can be suitably used as an absorbent member used for absorbent articles, such as disposable diapers, sanitary napkins, and incontinence pads.

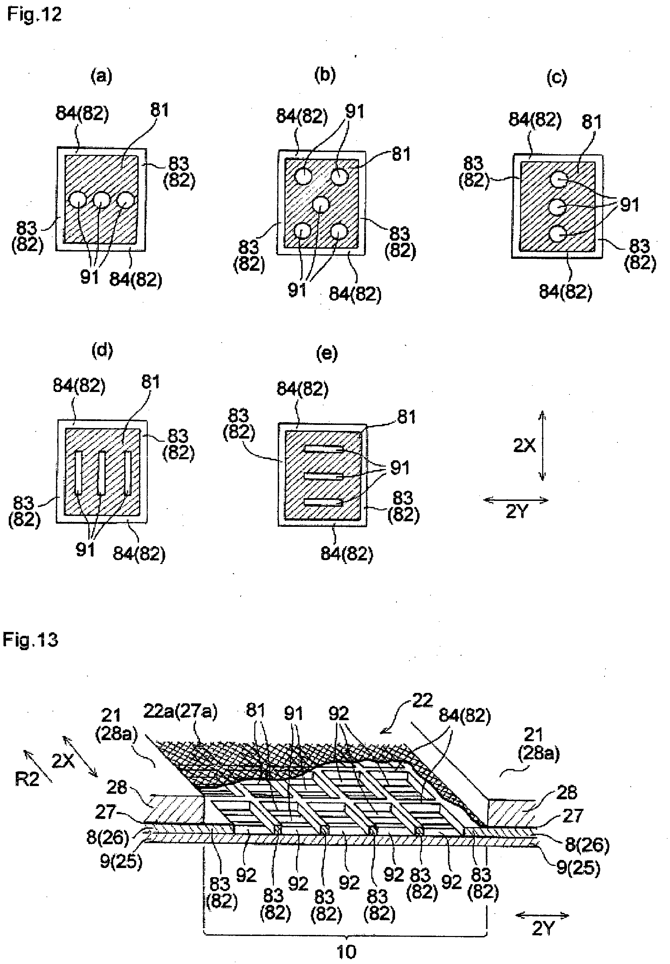

Incidentally, in relation to the adjuster 10, one first opening 81 of the first layer 8 has corresponded to one second opening 91 of the second layer 9 in the above-mentioned embodiments (the first and second embodiments) so that the first openings 81 are in one-to-one correspondence with the second openings 91. However, as illustrated in Fig. 12, the plurality of second openings 91 of the second layer 9 may correspond to one first opening 81 of the first layer 8. In embodiments illustrated in Figs. 12(a) to 12(c), a plurality of second openings 91 having a circular shape in planar view correspond to one first opening 81 having a rectangular shape in planar view. In embodiments illustrated in Figs. 12(d) to 12(e), a plurality of second openings 91 having a rectangular shape in planar view correspond to one first opening 81 having a rectangular shape in planar view. Accordingly, the shape of the first opening 81 in planar view is not similar to the shape of the second opening 91, which corresponds to the first opening 81, in planar view in any embodiment illustrated in Fig. 12. In Fig. 12, a hatched portion is an air-impermeable section through which air does not pass. Meanwhile, for easy description, the porous plate 27 is not illustrated in Fig. 12.

In a case in which a plurality of second openings 91 correspond to one first opening 81 as in the embodiments illustrated in Fig. 12, it is preferable to arrange the plurality of second openings 91 in a line in a direction (the 2Y direction) orthogonal to the transporting direction of the recess 22 as illustrated in Figs. 12(a) and 12(d) in consideration of the transporting direction of the collecting/stacking recess 22 (a direction parallel to the 2X direction) from the viewpoint of more reliably achieving a rectification effect, which is obtained from the above-mentioned first openings 81, by making an air stream flow in the plurality of second openings 91 present in the first opening 81 substantially in the same time when the first opening 81 enters a region above the space of the rotating drum 2 maintained at negative pressure.

The invention (the first aspect of the invention) is not limited to the above-mentioned embodiments and may be appropriately modified. For example, each second opening 91 is present at the position separated from both the CD defining members 84 and the MD defining members 83, and the portions of the second layer 9 overlapping both the defining members 83 and 84 and the vicinity thereof (92, 93) form air-impermeable sections in the above-mentioned first and second embodiments, but at least each second opening 91 may be positioned at a position separated from the CD defining members 84, and at least the portions of the second layer 9 overlapping the CD defining members 84 and the vicinity thereof may form air-impermeable sections in the invention. Accordingly, each second opening 91 may continue so as to cross over the plurality of first openings 81 in the 2Y direction as illustrated in Fig. 13. In an embodiment illustrated in Fig. 13, each of the plurality of second openings 91 continue over substantially the total length of the recess 22 in the 2Y direction. For this reason, the plurality of first openings 81 communicate with one another so that an air flow can flow in the 2Y direction through the second openings 91 continuing in the 2Y direction.

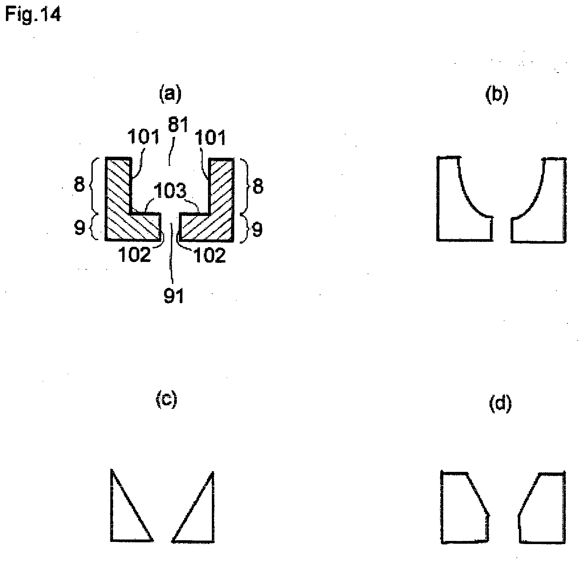

The adjuster according to the invention only has to include a plurality of openings that penetrate the adjuster in the thickness direction, and the area of the opening end portion of the opening relatively distant from the porous member only has to be smaller than the area of the opening end portion thereof relatively close to the porous member, and the structure of the adjuster is not limited to the above-mentioned embodiments. For example, the first and second layers 8 and 9 of the adjuster 10 are formed separately from each other in the first embodiment (see Fig. 5), but both the layers 8 and 9 may be formed integrally with each other (the adjuster 10 may be formed of one plate) (see Fig. 14(a)).

Further, in the first and second embodiments, as illustrated in Fig. 14(a), the shape of each of the plurality of openings, which penetrate the adjuster 10 in the thickness direction, in cross-sectional view (the shape of the cross-section of each of the plurality of openings taken in a direction parallel to the rotation axis of the rotating drum 2) is formed by vertical wall portions 101 (a part of the first layer 8), vertical wall portions 102 (a part of the second layer 9), and horizontal wall portions 103 (portions of the second layer 9 in the vicinity of the MD-defining-member corresponding section). The vertical wall portions 101 and 102 are parallel to a direction of a normal to the outer peripheral surface 21 of the rotating drum 2 (the thickness direction of the adjuster 10; a vertical direction in Fig. 14), and the horizontal wall portions 103 are parallel to a direction (a horizontal direction in Fig. 14) orthogonal to the direction of the normal (the thickness direction of the adjuster 10). Each of the plurality of openings which penetrate the adjuster 10 in the thickness direction, includes a flat stepped portion, which is positioned at a middle portion thereof in the thickness direction or in the vicinity thereof and is formed of the horizontal wall portions 103. The shape of the opening according to the invention in cross-sectional view is not limited to the shape illustrated in Fig. 14(a), and may be, for example, a shape that includes a part of curved wall portions as illustrated in Fig. 14(b), a shape that is formed by only diagonal wall portions extending in a direction (except for a direction orthogonal to the direction of the normal) intersecting with the direction of the normal (the thickness direction of the adjuster 10) as illustrated in Fig. 14(c), or a shape that includes a part of the diagonal wall portions as illustrated in Fig. 14(d).

Meanwhile, each of the adjusters 10 illustrated in Figs. 14(a) to 14(d) may be integrally formed (the adjuster 10 may be formed of one plate) as illustrated, or may be formed of a plurality of layers, which are separate bodies, as in the first and second embodiments. Further, in the embodiments illustrated in Figs. 14(a) to 14(d), the opening end portion relatively close to the porous member (the porous plate 27) corresponds to the above-mentioned first opening 81 and the opening end portion relatively distant from the porous member corresponds to the above-mentioned second opening 91. Accordingly, since the adjuster 10 includes the first and second openings 81 and 91 as one opening end portions and the other opening end portions of the adjuster 10 in the thickness direction even in embodiments in which the adjusters 10 are integrally formed (embodiments in which the adjuster 10 is formed of one plate) and have the shapes of Figs. 14(a) to 14(d) in cross-sectional view, the description of the arrangement or the sizes of the above-mentioned first and second openings 81 and 91 is also applied to these embodiments in which the adjusters 10 are integrally formed.

Furthermore, the adjuster 10 includes two layers, that is, the first and second layers 8 and 9 in the first embodiment, but may further include a plurality of layers, such as third and fourth layers.

Moreover, the collecting/stacking recesses 22 are intermittently formed on the outer peripheral surface 21 of the rotating drum 2 in the circumferential direction in the first and second embodiments, but may be continuously formed on the outer peripheral surface 21 of the rotating drum 2 over the total length of the rotating drum 2 in the circumferential direction. Further, the recess partitioning member 35 is arranged so as to overlap only a part of the outer surface 27a of the porous plate 27 in the second embodiment, but may be arranged so as to overlap the entire outer surface 27a.

Portions, which are included in only one embodiment described above, may be appropriately used in the all embodiments.

Furthermore, the porous plate 27 forming the bottom surface 22a of the collecting/stacking recess 22 is flat in the first and second embodiments, but a difference in level may be formed on the bottom surface 22a so that the depth of the recess 22 in the adjuster-non-arrangement region 23 is different from the depth of the recess 22 in the adjuster-arrangement region 24. In this case, a difference in level is formed on the bottom surface 22a so that the bottom surface 22a of the adjuster-non-arrangement region 23 is lower than the bottom surface 22a of the adjuster-arrangement region 24, and the depth of the recess 22 in the adjuster-non-arrangement region 23 is made to be larger than the depth of the recess 22 in the adjuster-arrangement region 24 by this difference in level. Further, in order to cope with a case in which a difference in level is formed on the bottom surface 22a as described above and the recess partitioning member 35 is disposed so as to cross over the difference in level (so as to cross over two regions 23 and 24 that are different in the depth of the recess 22) in the second embodiment, it is preferable that the shape of the recess partitioning member 35 be modified to a shape along the bottom surface of the collecting/stacking recess 22 or the thickness of the adjuster-non-arrangement region 23 in which the bottom surface 22a is present at a relatively low position be increased in the recess partitioning member 35.

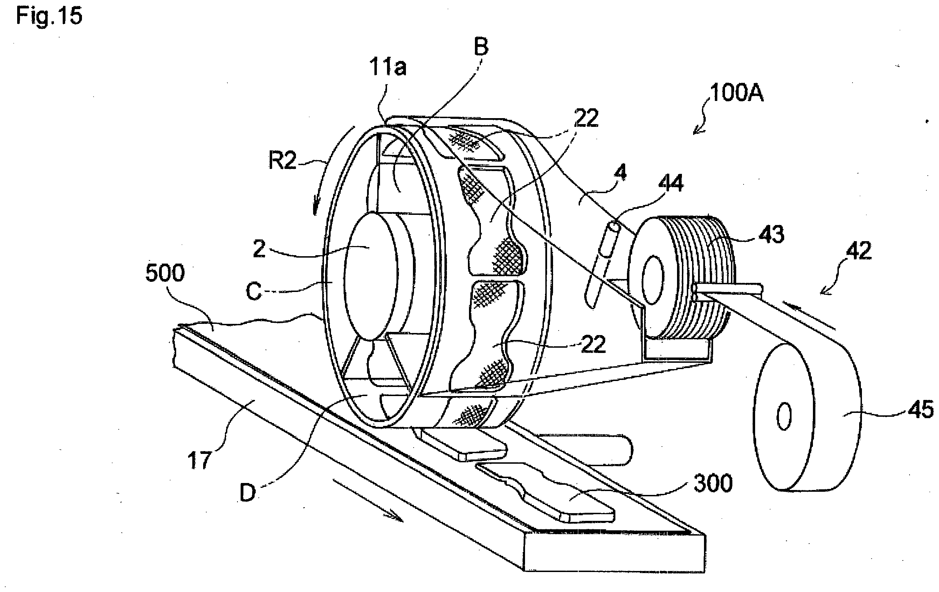

Furthermore, the fiber stacking device of the invention (the first aspect of the invention) is not limited to the fiber stacking device 100 illustrated in Fig. 1, and may be formed like a fiber stacking device 100A illustrated in Fig. 15. Meanwhile, for easy description, a part of one side plate of the rotating drum 2 in an axial direction (a plate that defines the spaces B, C, and D in the rotating drum 2 and has a circular shape in planar view) is transparently illustrated in Fig. 15. The fiber stacking device 100 illustrated in Fig. 1 includes the vacuum box 13 and the windshield plates 15 as means for stably transferring the fiber stack 300, which is present in the collecting/stacking recess 22, without causing the fiber stack 300 to lose its shape. However, the fiber stacking device 100A does not include this means as illustrated in Fig. 15. Moreover, in the fiber stacking device 100A, the fiber stack 300 present in the collecting/stacking recess 22 is directly transferred onto a vacuum conveyor 17, which is arranged below a rotating drum 2, (onto a core-wrap sheet 500 that is introduced onto the conveyor 17) at the lowest point of the rotating drum 2. For this reason, the fiber stacking device 100A also does not include the transfer roller 12 and the mesh belt 14 that have been included in the fiber stacking device 100. In Fig. 15, reference numeral 42 denotes a shaped-product material introduction device that can also be applied to the fiber stacking device 100, and the shaped-product material introduction device 42 is adapted to pulverize a wood pulp sheet 45 into fibrillated pulp by a pulverizer 43 and to send the fibrillated pulp (a fiber material) into the duct 11. Further, a water-absorbent polymer introduction unit 44, which introduces water-absorbent polymer particles, is provided on the duct 11.

In the fiber stacking device 100A illustrated in Fig. 15, the pressures of a space B, a space C, and a part of a space D (a region of the space D that is positioned on the upstream side of a transfer position of the fiber stack 300 in a rotating direction R2 of the rotating drum 2) are set to negative pressure, the fiber stack 300 formed in the collecting/stacking recess 22 is transported to a predetermined transfer position (a position which is present above the space D and at which the rotating drum 2 and the vacuum conveyor 17 face each other: the lowest point of the rotating drum 2) by the rotation of the rotating drum 2 while being sucked in the recess 22 above the space B (in the duct 11), the fiber stack 300 is released from the recess 22 at the transfer position by suction from the conveyor 17, and the fiber stack 300 is transferred onto the core-wrap sheet 500 that is introduced onto the conveyor 17. At the transfer position, it is possible to facilitate the release of the fiber stack 300 from the recess 22 by actively blowing air to the vacuum conveyor 17 from the space D. When a fiber stack, which is relatively less likely to lose its shape like the fiber stack 300 illustrated in Fig. 7, is obtained, this so-called transfer blow is particularly effective in facilitating the release of the fiber stack from the collecting/stacking recess.

The absorbent member manufactured in the invention (the first aspect of the invention) is preferably used as an absorbent member of an absorbent article. An absorbent article is used to mainly absorb and hold body fluid, such as urine and menstrual blood, excreted from the body. An absorbent article includes, for example, a disposable diaper, a sanitary napkin, an incontinence pad, a panty liner, and the like. However, the absorbent article is not limited thereto, and widely includes articles that are used to absorb fluid discharged from a human body.

An absorbent article typically includes a topsheet, a backsheet, and a liquid-retaining absorbent member that is interposed between both the sheets. Upper and lower surfaces of the absorbent member may be covered with one or a plurality of core-wrap sheets. The backsheet may have or may not have vapor permeability. The absorbent article may further include various members that correspond to specific uses of the absorbent article. Such the members are known to those skilled in the art. For example, when the absorbent article is applied to a disposable diaper or a sanitary napkin, a pair of or two or more pairs of three-dimensional guards may be disposed outside both side portions, which stand up, of the absorbent member.

In relation to the above-mentioned embodiments of the invention (the first aspect of the invention), the following additional features (for the fiber stacking device, the method of manufacturing an absorbent member, and the absorbent member) are disclosed.
<1> A fiber stacking device including a collecting/stacking recess in which a shaped-product material is stacked and which is formed on an outer surface thereof, and stacking a shaped-product material, which is transported on an air stream generated by suction from inside, on a bottom surface of the collecting/stacking recess, which is formed on a porous member including a plurality of suction holes, while transporting the collecting/stacking recess in one direction,
   wherein an adjuster, which adjusts the air stream, is arranged on an inner surface of the porous member so as to overlap at least a part of the inner surface of the porous member, and
   the adjuster includes a plurality of openings that penetrates the adjuster in a thickness direction, and the area of an opening end portion of each opening relatively distant from the porous member is smaller than the area of an opening end portion thereof relatively close to the porous member.
<2> The fiber stacking device as set forth in clause <1>,
   wherein the adjuster includes a first layer and a second layer that are laminated in this order from the porous member,
   the first layer includes a plurality of first openings that penetrate the first layer in the thickness direction and an opening defining section that partitions and forms the first openings, and the opening defining section includes MD defining members that extend in a transporting direction of the collecting/stacking recess, and CD defining members that extend in a direction orthogonal to the transporting direction,
   second openings, which penetrate the second layer in the thickness direction, are formed at portions of the second layer, which overlap the plurality of first openings in a planar view of the collecting/stacking recess, so as to correspond to the respective first openings, and the plurality of openings are formed of the first and second openings that overlap each other in the planar view, and
   the second opening is present at a position separated from the CD defining members in each of the plurality of first openings in a planar view of the collecting/stacking recess, and portions of the second layer overlapping the CD defining members and the vicinity thereof have air impermeability where air does not pass.
<3> The fiber stacking device as set forth in clause <1>,
   wherein the adjuster is formed of one plate, and includes first openings that are the opening end portions relatively close to the porous member and second openings that are the opening end portions relatively distant from the porous member.
<4> The fiber stacking device as set forth in clause <2> or <3>,
   wherein a ratio (S2/S1) of the area (S2) of the second opening to the area (S1) of the first opening is preferably in the range of 5 to 50% and more preferably in the range of 7 to 15%.
<5> The fiber stacking device as set forth in any one of clauses <2> to <4>,
   wherein the second opening is present at a position separated from the MD defining members in each of the plurality of first openings in a planar view of the collecting/stacking recess, and portions of the second layer overlapping the MD defining members and the vicinity thereof have air impermeability where air does not pass.
<6> The fiber stacking device as set forth in any one of clauses <2> to <5>,
   wherein the second opening is formed at the center of each of the plurality of first openings in a planar view of the collecting/stacking recess.
<7> The fiber stacking device as set forth in any one of clauses <2> to <6>,
   wherein the shape of one of the first openings in planar view and the shape of the second opening, which is present in the one first opening, in planar view are similar to each other in a planar view of the collecting/stacking recess.
<8> The fiber stacking device as set forth in any one of clauses <1> to <7>,
   wherein the shape of each of the plurality of openings, which penetrate the adjuster in the thickness direction, in cross-sectional view is formed by vertical wall portions that are parallel to the thickness direction of the adjuster, and horizontal wall portions that are parallel to a direction orthogonal to the thickness direction, and
   each of the plurality of openings includes a flat stepped portion, which is positioned at a middle portion thereof in the thickness direction or in the vicinity thereof and is formed of the horizontal wall portions.
<9> The fiber stacking device as set forth in any one of clauses <1> to <8>,
   wherein the shape of each of the plurality of openings, which penetrate the adjuster in the thickness direction, in cross-sectional view is a shape that includes a part of curved wall portions, a shape that is formed by only diagonal wall portions extending in a direction intersecting with the thickness direction of the adjuster, or a shape that includes a part of the diagonal wall portions.
<10> The fiber stacking device as set forth in any one of clauses <1> to <9>,
   wherein the collecting/stacking recess includes an adjuster-arrangement region where the adjuster is arranged on an inner surface of the porous member forming the bottom surface of the collecting/stacking recess and an adjuster-non-arrangement region where the adjuster is not arranged on the inner surface of the porous member, and the basis weight of the shaped-product material stacked in the adjuster-non-arrangement region is higher than that of the shaped-product material stacked in the adjuster-arrangement region.
<11> The fiber stacking device as set forth in any one of clauses <1> to <10>,
   wherein a recess partitioning member, which partitions the recess into a plurality of regions in a direction parallel to the bottom surface, is arranged so as to overlap at least a part of an outer surface of the porous member that forms the bottom surface of the collecting/stacking recess.
<12> The fiber stacking device as set forth in clause <11>,
   wherein the recess partitioning member includes a plurality of openings that penetrate the recess partitioning member in the thickness direction, and
   the openings of the recess partitioning member and the openings, which penetrate the adjuster in the thickness direction, overlap each other in a planar view of the collecting/stacking recess.
<13> The fiber stacking device as set forth in any one of clauses <1> to <12>, further including:
   a rotating drum; and
   a duct that supplies the shaped-product material to an outer peripheral surface of the rotating drum,
   wherein the rotating drum includes the collecting/stacking recess on the outer peripheral surface thereof.
<14> The fiber stacking device as set forth in clause <13>,
   wherein the rotating drum includes a cylindrical drum body, a suction adjusting plate that is fixed so as to overlap an outer peripheral portion of the drum body, a space plate that is fixed so as to overlap an outer surface of the suction adjusting plate, a porous plate that is the porous member fixed so as to overlap an outer surface of the space plate, and a pattern forming plate that is fixed so as to overlap an outer surface of the porous plate, and
   the adjuster includes the suction adjusting plate and the space plate.
<15> A method of manufacturing an absorbent member by using the fiber stacking device as set forth in any one of clauses <1> to <14>, the method including:
   a fiber stacking step of sucking and stacking a shaped-product material, which is an absorbent-member material supplied on an air stream, in the collecting/stacking recess of the fiber stacking device.
<16> The method as set forth in clause <15>,
   wherein the shaped-product material is stacked in a region, in which the adjuster is not arranged on an inner surface of the porous member, (the adjuster-non-arrangement region) of the collecting/stacking recess with a relatively high basis weight and is stacked in a region, in which the adjuster is arranged on the inner surface of the porous member, (the adjuster-arrangement region) of the collecting/stacking recess with a relatively low basis weight.
<17> An absorbent member that is manufactured by using the fiber stacking device as set forth in any one of clauses <1> to <14>,
   a portion corresponding to a region, in which the adjuster is not arranged on an inner surface of the porous member, (the adjuster-non-arrangement region) of the collecting/stacking recess is a high basis-weight section in which the amount of an absorbent-member material (a shaped-product material) to be stacked is relatively large, and
   a portion corresponding to a region, in which the adjuster is arranged on the inner surface of the porous member, (the adjuster-arrangement region) of the collecting/stacking recess is a low basis-weight section in which the amount of an absorbent-member material (a shaped-product material) to be stacked is relatively small.
<18> The absorbent member as set forth in clause <17>,
   wherein the high basis-weight section is a thick section, and the low basis-weight section is a thin section.
<19> The absorbent member as set forth in clause <17> or <18>,
   wherein the entire one surface of the absorbent member is substantially flat, and the other surface of the absorbent member has a difference in level at a boundary portion between the high basis-weight section and the low basis-weight section.
<20> The absorbent member as set forth in any one of clauses <17> to <19>,
   wherein the absorbent member includes a projecting-and-recessed section that is formed on one surface thereof and includes a plurality of grooves having the shape of continuous straight lines in planar view and arranged in the shape of a lattice and protrusions having a quadrangular shape in planar view and arranged at cells of the lattice.
<21> The absorbent member as set forth in clause <20>,
   wherein a region of one surface of the absorbent member on which the projecting-and-recessed section is not formed is substantially flush with tops of the protrusions of the projecting-and-recessed section.

A fiber stacking device of the invention (the second aspect of the invention) will be described below on the basis of a preferred embodiment thereof with reference to the drawings. Fig. 16 illustrates a fiber stacking device 200 that is an embodiment of the fiber stacking device of the invention, and Fig. 17 illustrates a rotating drum 1 that is included in the fiber stacking device 200. The fiber stacking device 200 is a device that includes the rotating drum 1 including a collecting/stacking recess 22 on the outer peripheral surface thereof and forms a shaped product by stacking a shaped-product material, which is transported on an air stream generated by suction from the inside of the rotating drum 1, on a bottom section 22A of the collecting/stacking recess 22. When the shaped product manufactured by the fiber stacking device 200 is an absorbent member used for an absorbent article, such as a disposable diaper or a sanitary napkin, the shaped-product material is an absorbent-member material.

The shaped-product material contains a fiber material. Various materials, which have been conventionally used for absorbent members of absorbent articles, such as a sanitary napkin or a panty liner and a disposable diaper, can be used as the shaped-product material, which serves as the absorbent-member material, without particular limitation. For example, pulp fiber such as fibrillated pulp, short fiber of cellulosic fiber, such as rayon fiber and cotton fiber, short fiber of synthetic fiber such as polyethylene, and the like are used. One of these fiber materials can be used alone or the combination of two or more thereof can be used. Further, a water-absorbent polymer and a fiber material may be used together as the absorbent-member material. Furthermore, as a fibrous material, a fibrous water-absorbent polymer can be used alone or can be used together with a fiber material. Moreover, a deodorizer, an antimicrobial agent, or the like can also be used together with a fiber material or the like, as necessary.

As illustrated in Fig. 16, the fiber stacking device 200 includes the rotating drum 1 that is driven to rotate in the direction of arrow R1, a duct 11 that supplies a shaped-product material to the outer peripheral surface of the rotating drum 1, a transfer roller 12 that is disposed obliquely below the rotating drum 1 and is driven to rotate in the direction of arrow R2, and a vacuum conveyor (not illustrated) that is arranged below the transfer roller 12. The vacuum conveyor of the fiber stacking device 200 is formed so as to have the same structure as that of a general vacuum conveyor of this type of fiber stacking device; and includes an endless air-permeable belt that is spanned between a drive roller and a driven roller, and a vacuum box that is arranged at a position facing the transfer roller 12 across the air-permeable belt.

The fiber stacking device 200 is further provided with a vacuum box 11 that is provided between the duct 11 and the transfer roller 12 in a circumferential direction of the rotating drum 1, a mesh belt 14 that is arranged so as to pass between the vacuum box 13 and the rotating drum 1 and between the transfer roller 12 and the rotating drum 1, and windshield plates 15 that are provided close to the outer peripheral surface of the transfer roller 12.

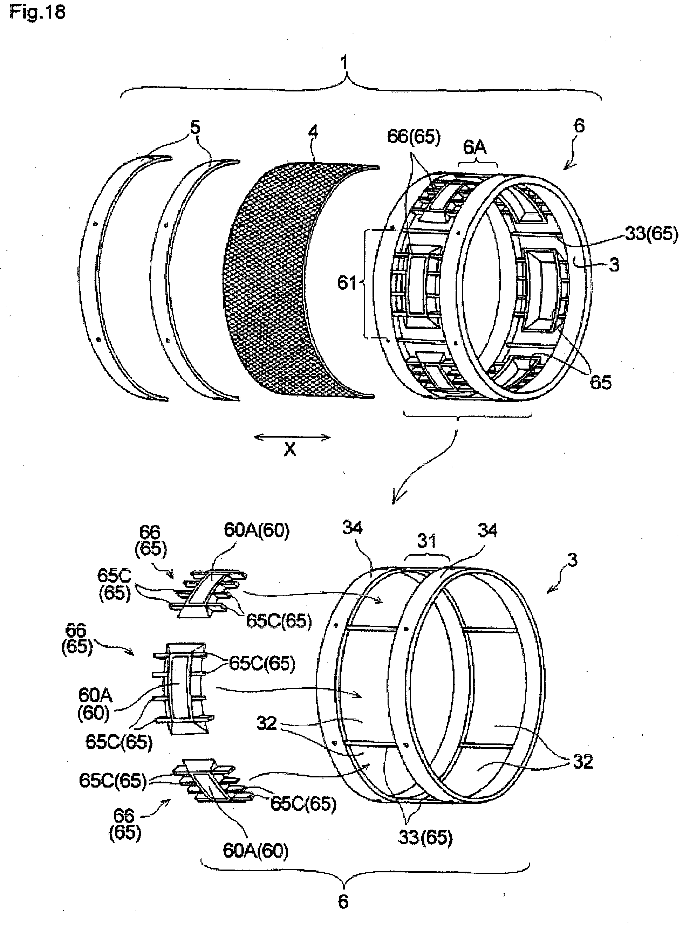

The rotating drum 1, which is a main feature of the fiber stacking device 200, will be described below. As illustrated in Figs. 17 and 18, the rotating drum 1 of this embodiment includes a collecting/stacking recess 22 which is formed on the outer peripheral surface thereof and in which a shaped-product material is stacked, and includes a drum body 3 and an air-permeable porous member 4 that forms a bottom section 22A of the collecting/stacking recess 22. The collecting/stacking recess 22 continues on the outer peripheral surface of the rotating drum 1 over the total length of the rotating drum in a circumferential direction. The shaped-product material is stacked on an outer surface 4a of the porous member 4 (the bottom surface of the collecting/stacking recess 22).

The drum body 3 is formed of a cylindrical body made of metal having stiffness, and includes a bottom-section corresponding section 31, which overlaps the bottom section 22A in a planar view of the collecting/stacking recess 22, at the middle portion thereof in a drum's width direction (the direction of a rotation axis of the rotating drum 1, a direction denoted by symbol X in Fig. 18) as illustrated in Fig. 18. Here, "planar view" refers to a view in which an object (the collecting/stacking recess, or the like) is viewed from the outside in a direction of a normal to the outer peripheral surface of the rotating drum 1 (a direction orthogonal to the direction of the rotation axis of the rotating drum 1). The drum body 3 includes a pair of annular sections 34 and 34 forming peripheral edge portions of the drum body 3, and the bottom-section corresponding section 31 is a portion that is interposed between the pair of annular sections 34 and 34. The bottom-section corresponding section 31 of the drum body 3 includes a plurality of (six in this embodiment) through openings 32 that penetrate the bottom-section corresponding section 31 in the thickness direction, and air-impermeable ribs 33 each of which is positioned between adjacent two through openings 32 and 32. Since the bottom-section corresponding section 31 includes the through openings 32, the bottom-section corresponding section 31 has air permeability as a whole. The plurality of through openings 32 are formed at a predetermined interval in the circumferential direction (the rotating direction) of the drum body 3, and an air-impermeable rib 33 is formed between two through openings 32 and 32, which are adjacent to each other in the circumferential direction, so as to extend in the drum's width direction X between the pair of annular sections 34 and 34. The ribs 33 originally serve to improve the strength of the drum body 3 itself and to improve the strength of the bottom section 22A of the collecting/stacking recess 22.

In the first embodiment, the drum body 3 functions as a base body that forms a basic framework of the rotating drum 1, and also functions as a part of a flow rate adjusting member that adjusts the flow rate of an air stream (vacuum air) generated by suction from the inside of the drum body 3. That is, the outer peripheral portion (a portion overlapping the porous member 4) of the drum body 3, specifically, the ribs 33 and the pair of annular sections 34 and 34 are also a part of a flow rate adjusting member 6 to be described below, and the ribs 33 that are positioned on a bottom-section corresponding section 6A of the flow rate adjusting member 6 are also a part (defining members) of opening defining sections 65 to be described below.

The porous member 4 conveys an air stream (vacuum air), which is generated by suction from inside the rotating drum 1, to the outside of the drum, and holds a shaped-product material, such as pulp, that are carried on the vacuum air. A plurality of fine air-permeation holes (not illustrated) are formed over the entire region of the porous member 4. Accordingly, the air-permeation holes function as suction holes for sucking the shaped-product material while the collecting/stacking recess 22 passes over a space that is formed in the rotating drum 1 and is maintained at negative pressure. The porous member 4 may be provided with, for example, circular holes, which have a diameter of about 0.2 to 0.6 mm and are formed at a pitch of about 0.4 to 1.5 mm in a staggered pattern, as the air-permeation holes. A metal or resin mesh, a porous metal plate or a resin plate in which a plurality of fine holes are formed in a metal or resin plate by etching or punching, or the like can be used as the porous member 4. For example, a plate, which is obtained by forming a plurality of fine holes in a metal or resin plate (for example, a stainless steel plate, or the like) having a thickness of about 0.1 to 0.5 mm with a method, such as punching or etching, is used as the porous metal plate or the resin plate that forms the porous member 4.

As illustrated in Figs. 17 to 19, the rotating drum 1 includes ring members 5 that form inner-side surfaces 22B of the collecting/stacking recess 22, in addition to the drum body 3 and the porous member 4. The ring members 5 define the length of the collecting/stacking recess 22 in the drum's width direction X (the width of the collecting/stacking recess 22), and are arranged on both side portions of the outer peripheral surface of the rotating drum 1 in the width direction with the collecting/stacking recess 22 between the ring members 5. A distance between the ring member 5, which is arranged on one side in the width direction, and the ring member 5, which is arranged on the other side, (a distance between the pair of right and left ring members) constitutes the width of the collecting/stacking recess 22. Further, the inner end face of the ring member 5 formed along the circumferential direction of the rotating drum 1 forms the inner-side surface 22B of the collecting/stacking recess 22, and is an element that determines the depth of the collecting/stacking recess 22. The mounting positions of the ring members 5 (the distance between the pair of right and left ring members) and the thickness of each ring member 5 (the height of the inner end face) are determined in consideration of the width of the shaped product (the fiber stack), the amount of a shaped-product material to be stacked, and the like. The ring member 5 is air-impermeable and is formed of, for example, a metal plate such as a stainless steel plate or a resin plate, and the thickness of the ring member 5 is, fot example, about 2 to 12 mm.

One of the main features of the rotating drum 1 of this embodiment is that the flow rate adjusting member 6 for adjusting the flow rate of an air stream (vacuum air) generated by suction from the inside of the rotating drum 1 is arranged on an inner surface 4b side of the porous member 4 (the side opposite to the outer surface 4a that is a surface on which the shaped-product material is stacked) as illustrated in Figs. 18 and 19. As described above, the flow rate adjusting member 6 of this embodiment includes the drum body 3 and forms a cylindrical body as a whole. The porous member 4 and the flow rate adjusting member 6 are integrally rotated by the rotation of the rotating drum 1 (the drum body 3). The porous member 4 and the flow rate adjusting member 6 directly overlap each other as illustrated in Figs. 18 and 19 in this embodiment, and other members are not arranged between the porous member 4 and the flow rate adjusting member 6.

The length (width) of the flow rate adjusting member 6 (the outer peripheral portion of the drum body 3) in the drum's width direction X is the same as the length (width) of the porous member 4 in the drum's width direction X, and the flow rate adjusting member 6 includes the bottom-section corresponding section 6A, which overlaps the bottom section 22A in a planar view of the collecting/stacking recess 22, at the middle portion thereof in the drum's width direction X. Here, "planar view" has the above-mentioned meaning. Further, portions of the flow rate adjusting member 6 except for the bottom-section corresponding section 6A, that is, the pair of annular sections 34 and 34 of the drum body 3 constitute ring-members corresponding sections that overlap the ring members 5 in a planar view of the outer peripheral surface of the rotating drum 1 in this embodiment. The length (width) of the annular section 34 of the flow rate adjusting member 6 (the drum body 3) in the drum's width direction X is the same as the length (width) of the ring members 5 in the drum's width direction X. As illustrated in Fig. 19, the inner end faces of the annular sections 34 formed along the circumferential direction (the rotating direction) of the rotating drum 1 form the inner-side surfaces 22B of the collecting/stacking recess 22 together with the ring members 5. Like the bottom-section corresponding section 6A (the opening defining section 65), the annular section 34 is formed of a sparingly air-permeable or air-impermeable member through which air does not pass substantially. Metal, such as stainless steel, aluminum, or iron, a resin, or the like can be used as the sparingly air-permeable or air-impermeable member.

As illustrated in Figs. 18 to 20, the bottom-section corresponding section 6A of the flow rate adjusting member 6 (the bottom-section corresponding section 31 of the drum body 3) includes a plurality of openings 60 (60A, 60B, and 60C) that penetrate the bottom-section corresponding section 6A in the thickness direction and the opening defining sections 65 that partition and form the respective openings 60. The opening defining section 65 is formed of a sparingly air-permeable or air-impermeable member through which air does not pass substantially. Since the bottom-section corresponding section 6A includes sparingly air-permeable or air-impermeable members (the opening defining sections 65) but includes the openings 60 through which air passes, the bottom-section corresponding section 6A has air permeability as a whole.

The flow rate adjusting member 6 of this embodiment includes three kinds of openings 60A, 60B, and 60C, which have different shapes, as the plurality of openings 60. More specifically, the flow rate adjusting member 6 of this embodiment includes first openings 60A at the middle portion of the bottom-section corresponding section 6A in the drum's width direction X, includes second openings 60B on both sides of the bottom-section corresponding section 6A in the drum's width direction X with the first openings 60A interposed between the second openings 60B, and includes third openings 60C on the front and rear sides of the bottom-section corresponding section 6A in a drum's circumferential direction with the first openings 60A interposed between the third openings 60C. The first opening 60A has a rectangular shape in planar view, and the longitudinal direction of the first opening 60A corresponds to the drum's circumferential direction. The length of the second opening 60B in the drum's circumferential direction is shorter than the length of the first opening 60A in the drum's circumferential direction, and the second opening 60B has a quadrangular shape in planar view. The third opening 60C has a rectangular shape in planar view, and the longitudinal direction of the third opening 60C corresponds to the drum's width direction X. Further, the flow rate adjusting member 6 of this embodiment includes a plurality of (six in this embodiment) opening groups 61 (see Fig. 18) that have a quadrangular shape in planar view and are arranged at a predetermined interval in the drum's circumferential direction. That is, the flow rate adjusting member 6 includes the opening groups 61 that are formed in the bottom-section corresponding section 6A thereof and each of which includes the plurality of openings 60 having different shapes or sizes. The plurality of opening groups 61 are present in the drum's circumferential direction. Specifically, the opening group 61 includes one first opening 60A, a plurality of (six in this embodiment) second openings 60B that are positioned on (are adjacent to) both sides of the one first opening 60A in the drum's width direction X, and a plurality of (two in this embodiment) third openings 60C that are positioned on (are adjacent to) the front and rear sides of the one first opening 60A in the drum's circumferential direction. The plurality of opening groups 61 are in one-to-one correspondence with the plurality of (six in this embodiment) through openings 32 of the bottom-section corresponding section 31 of the drum body 3 (see Fig. 17), and one opening group 61 and one through opening 32 overlap each other in a planar view of the collecting/stacking recess 22. "Having different shapes or sizes" also includes, for example, a case in which the shapes or sizes of opening end portions, which are relatively close to the porous member 4 (the opening end portion will be described below), of the first and second openings 60A and 60B are the same as each other but the shapes or sizes of opening end portions, which are relatively distant from the porous member 4, of the first and second openings 60A and 60B are different from each other.

As illustrated in Fig. 20, the opening defining section 65 (the bottom-section corresponding section 6A) of this embodiment includes four defining members 65A and 65B that partition and form the first opening 60A having a rectangular shape in planar view and have a trapezoid shape in planar view. Among the four defining members, two defining members 65A and 65A are arranged so as to extend in the drum's circumferential direction and face each other, and two defining members 65B and 65B are arranged so as to extend in the drum's width direction X (the direction of the rotation axis of the rotating drum 1) and face each other. Both end portions of the defining members 65A in the drum's circumferential direction are connected to both end portions of the defining members 65B in the drum's width direction X, so that the first opening 60A having a rectangular shape in planar view is partitioned and formed. Each of the defining members 65A and 65B is formed of a sparingly air-permeable or air-impermeable member through which air does not pass substantially.

Further, as illustrated in Fig. 20, the opening defining section 65 (the bottom-section corresponding section 6A) of the this embodiment includes a plurality of (eight in this embodiment) defining members 65C that are provided between the defining members 65A extending in the drum's circumferential direction and the annular sections 34 of the drum body 3 and extend in the drum's width direction X. The opening defining section 65 (the bottom-section corresponding section 6A) further includes the ribs 33 that are also a part of the drum body 3 as described above and extend in the drum's width direction X. Each of the plurality of second openings 60B is partitioned and formed by the two defining members 65C and 65C that are parallel to each other and the annular section 34 and the defining member 65A that extend in the drum's circumferential direction. Furthermore, each of the plurality of third openings 60C is partitioned and formed by the rib 33, the defining members 65B and 65C, and the pair of annular sections 34 and 34 extending the drum's circumferential direction. Each of the defining member 65C and the rib 33 is formed of a sparingly air-permeable or air-impermeable member through which air does not pass substantially.

The opening defining section 65 (the bottom-section corresponding section 6A) of this embodiment includes the defining members 65A, 65B, and 65C, and the ribs 33 that are also a part of the drum body 3 as described above, the defining members 65A, 65B, and 65C are integrated in advance so as to form a defining member unit 66 as illustrated in Fig. 18, and the defining member unit 66 is detachably fixed to the drum body 3. That is, in this embodiment, a part of the flow rate adjusting member 6 is detachably fixed to the body (the drum body 3) of the rotating drum 1. The length (width) of the defining member unit 66 in the drum's width direction X is substantially the same as the length of the through opening 32 of the bottom-section corresponding section 31 of the drum body 3 (the bottom section 22A of the collecting/stacking recess 22) in the drum's width direction X. The defining member unit 66 is arranged in the through opening 32 as illustrated in Figs. 18 and 19, and, at the respective end portions of the plurality of defining members 65C extending in the drum's width direction X, is detachably fixed to the pair of annular sections 34 and 34 of the drum body 3, which define the through opening 32, by fixing means (not illustrated), such as screws.

At least a part of the plurality of openings 60 (60A, 60B, and 60C) of the bottom-section corresponding section 6A of the flow rate adjusting member 6 are porous member-side-small-open-type openings of each which the area of an opening end portion relatively close to the porous member 4 is smaller than the area of an opening end portion relatively distant from the porous member 4. Meanwhile, the opening end portion of the opening 60 (60A, 60B, 60C) is one end portion or other portion of the opening 60 in a depth direction of the opening 60 (the thickness direction of the flow rate adjusting member 6). In this embodiment, the first opening 60A of which the area of an opening end portion close to the porous member 4 is relatively large as illustrated in Figs. 19 and 20 is a porous member-side-small-open-type opening. The defining member 65A, which partitions and forms the first opening 60A, extends so as to be inclined with respect to a normal 2L (see Fig. 19) to the bottom section 22A of the collecting/stacking recess 22 by a predetermined angle α (see Fig. 19) from the porous member 4 side toward the inside of the rotating drum 1 (the drum body 3) in side view, and has the shape of a straight line in side view (cross-sectional view). Like the defining member 65A, the other defining member 65B, which partitions and forms the first opening 60A, is also formed in the shape of a straight line that extends so as to be inclined with respect to the normal 2L to the bottom section 22A by a predetermined angle. As illustrated in Fig. 19, the area of the first opening 60A (a porous member-side-small-open-type opening), which is partitioned and formed by the defining members 65A and 65B formed in the shape of a straight line that extends so as to be inclined with respect to the normal 2L to the bottom section 22A as described above, is gradually increased as being apart from the porous member 4.

In this embodiment, the bottom-section corresponding section 6A of the flow rate adjusting member 6 further includes porous member-side-large-open-type openings each of which the area of an opening end portion relatively close to the porous member 4 is larger than the area of an opening end portion relatively distant from the porous member 4, as a part of the plurality of openings 60. That is, on the contrary to the first opening 60A, the plurality of second openings 60B are porous member-side-large-open-type openings each of which the area of an opening end portion relatively close to the porous member 4 is larger than the area of an opening end portion relatively distant from the porous member 4 as illustrated in Figs. 19 and 20. Among the defining members 65A and 65C and the annular section 34 of the drum body 3 that partition and form the second opening 60B, as described above, only the defining member 65A extends so as to be inclined with respect to the normal 2L to the bottom section 22A of the collecting/stacking recess 22, and each of the defining member 65C and the annular section 34 is formed so as to extend in parallel to the normal 2L and has the shape of a straight line in side view (cross-sectional view). As illustrated in Fig. 19, the area of the second opening 60B (a porous member-side-large-open-type opening), which is partitioned and formed by the defining member 65A formed in the shape of a straight line that extends so as to be inclined with respect to the normal 2L to the bottom section 22A and the defining members 65C and the annular section 34 formed in the shape of a straight line extending in parallel to the normal 2L as described above, is gradually reduced as being apart from the porous member 4.

Further, on the contrary to the first opening 60A, the plurality of third openings 60C are also porous member-side-large-open-type openings each of which the area of an opening end portion relatively close to the porous member 4 is larger than the area of an opening end portion relatively distant from the porous member 4 as illustrated in Fig. 20. Among the defining members 65B and 65C and the rib 33 and the pair of annular sections 34 and 34 also serving as a part of the drum body 3 that partition and form the third opening 60C, as described above, only the defining member 65B extends so as to be inclined with respect to the normal 2L to the bottom section 22A of the collecting/stacking recess 22, and each of the defining member 65C, the rib 33, and the annular section 34 is formed so as to extend in parallel to the normal 2L and has the shape of a straight line in side view (cross-sectional view). Like the second opening 60B, the area of the third opening 60C (a porous member-side-large-open-type opening), which is partitioned and formed by the defining member 65B formed in the shape of a straight line that extends so as to be inclined with respect to the normal 2L to the bottom section 22A and the defining members 65C, the rib 33, and the annular sections 34 formed in the shape of a straight line extending in parallel to the normal 2L as described above, is gradually reduced as being apart from the porous member 4.

If the area of an opening end portion relatively close to the porous member 4 is smaller than the area of an opening end portion relatively distant from the porous member 4 as in the first opening 60A (a porous member-side-small-open-type opening), the flow rate of air (the amount of air passing through the bottom section 22A per unit time), which is obtained when an air stream (vacuum air) for sucking a shaped-product material flows through the bottom section 22A (the porous member 4) of the collecting/stacking recess 22, is increased and a suction force for sucking a shaped-product material is increased as compared to a case where the area of an opening end portion relatively close to the porous member 4 is the same as the area of an opening end portion relatively distant from the porous member 4. On the other hand, if the area of an opening end portion relatively close to the porous member 4 is larger than the area of an opening end portion relatively distant from the porous member 4 as in the second and third openings 60B and 60C (porous member-side-large-open-type openings), the above-mentioned flow rate of air is reduced and a suction force for sucking a shaped-product material reduced increased as compared to a case where the area of an opening end portion relatively close to the porous member 4 is the same as the area of an opening end portion relatively distant from the porous member 4. Further, in the fiber stacking step of stacking a fiber material, such as pulp fiber or synthetic fiber, a water-absorbent polymer (SAP), and the like in the collecting/stacking recess 22 as the shaped-product material, the basis weights of the fiber material and the SAP depend on the flow rate of an air stream that flows through the bottom section 22A (the porous member 4) of the collecting/stacking recess 22.

Accordingly, when the flow rate adjusting member 6 including the first openings 60A, which are the porous member-side-small-open-type openings, is arranged on the inner surface 4b side of the porous member 4 (the side opposite to the outer surface 4a that is a surface on which the shaped-product material are stacked) forming the bottom section 22A of the collecting/stacking recess 22 as in this embodiment, the flow rate of an air stream at a portion of the bottom section 22A corresponding to the first opening 60A (a portion of the bottom section 22A overlapping the opening 60A in a planar view of the bottom section 22A) is larger than that at the other portions of the bottom section 22A (portions of the bottom section 22A corresponding to the second and third openings 60B and 60C) and a suction force for sucking a shaped-product material at the portion of the bottom section 22A corresponding to the first opening 60A is larger than that at the other portions of the bottom section 22A. For this reason, according to this embodiment, a high basis-weight section in which the stacked amount of a shaped-product material is relatively large can be formed on the portion of the bottom section 22A corresponding to the first opening 60A, and a low basis-weight section in which the stacked amount of a shaped-product material is relatively small can be formed on the other portions of the bottom section 22A. Accordingly, it is possible to accurately adjust the basis weight of each portion of a shaped product and to manufacture a shaped product (an absorbent member) that includes the high basis-weight section and the low basis-weight sections. Further, it is possible to obtain an absorbent member, which is excellent in both absorption performance and the reduction of inconvenience and discomfort in wearing, by concentrating the material (for example, pulp fiber, SAP, and the like) of the absorbent member on a portion requiring high absorption capacity and reducing the basis weight of the other portions.

The adjustment of the basis weight of each portion of a shaped product and the manufacture of a shaped product of which the basis weight is partially different can also be performed by a technique disclosed in JP 2000-234255 A. However, since the technique disclosed in JP 2000-234255 A requires a plurality of suction means for sucking air from a plurality of chambers as described above, the costs of manufacturing facilities are increased. For this reason, there is a concern that an increase in the manufacturing cost of a shaped product may be caused. In contrast, according to this embodiment, it is possible to adjust the basis weight of each portion of a shaped product by partially changing a suction force with the use of the flow rate adjusting member 6 even though single suction means is used.

A method of partially changing a suction force for sucking the bottom section by making an opening area ratio of a porous member, which forms a bottom section of a collecting/stacking recess, partially vary when single suction means is used is known as a method of manufacturing a shaped product of which the basis weight is partially different. However, the method of manufacturing a shaped product with the change of specifications of the porous member is inferior to this embodiment in terms of performance due to the following reasons. That is, when the opening area ratio of a portion of the porous member corresponding to a low-suction section in which a suction force is lower than suction forces in other portions is reduced to form a part of the bottom section of the collecting/stacking recess as the low-suction section, i) a method of increase the pitch of air-permeation holes of the method or ii) a method of reducing the diameter of the air-permeation hole is generally considered. In relation to the method of i), since a difference in a suction force for sucking a shaped-product material between the air-permeation hole and the vicinity thereof and other portions is large in a low-suction section formed by the method, a large difference in the stacked amount of a material between both the portions may be caused. As a result, there is a concern that fiber-stacking irregularity caused by this difference in the stacked amount of a shaped-product material may occur in a low basis-weight section of a shaped product that is to be stacked on the low-suction section so as to originally have a uniform stacked amount of a material. For example, when the shaped product is an absorbent member used for an absorbent article, there is a concern that the occurrence of fiber-stacking irregularity may cause the reduction of absorption performance, the deterioration of the wearing comfort of an absorbent article, or the like. Further, in relation to the method of ii), for example, when a shaped-product material is an absorbent material, such as pulp fiber and SAP, there is a concern that the air-permeation hole may be clogged by the absorbent material.

Moreover, in this embodiment, the area of each of the plurality of openings 60A, 60B, and 60C of the bottom-section corresponding section 6A (the opening group 61) of the flow rate adjusting member 6 is changed in the thickness direction. However, since the first openings 60A (porous member-side-small-open-type openings) are adjacent to the second and third openings 60B and 60C (porous member-side-large-open-type openings) with the defining members 65A and 65B interposed therebetween, the area of the entire bottom-section corresponding section 6A (the opening groups 61) (the sum of the areas of the plurality of openings 60A, 60B, and 60C) is constant in the thickness direction of the flow rate adjusting member 6 (the bottom-section corresponding section 6A) and the flow rate of an air stream generated by suction from the inside of the drum body 3 is not changed substantially between the inside and the outside of the rotating drum 1 across the bottom section 22A (the porous member 4). Accordingly, it is possible to effectively prevent the reduction of a suction force for sucking a shaped-product material that is caused by the employment of the flow rate adjusting member 6 and a disadvantage that a shaped-product material is likely to be changed into a mass (lump) due to the reduction of a suction force.

From the viewpoint of more reliably achieving the effects that are obtained from the above-mentioned flow rate adjusting member 6, it is preferable that dimensions and the like of the respective portions of the flow rate adjusting member 6 be set as described below.

When the area of an opening end portion, which is relatively close to the porous member 4, of the first opening 60A (the porous member-side-small-open-type opening) is denoted by S1, and the area of an opening end portion, which is relatively distant from the porous member 4, of the first opening 60A is denoted by S2, a ratio of S2 to S1 [(S2/S1) × 100] is preferably 150% or greater, more preferably 200% or greater, and even more preferably 500% or greater, and preferably 1000% or less and more preferably 800% or less. More specifically, the ratio of S2 to S1 is preferably in the range of 150 to 1000%, more preferably in the range of 200 to 1000%, and even more preferably in the range of 500 to 800%.

The angle α (see Fig. 19) between the defining member 65A, which partitions and forms the first opening 60A, and the normal 2L is preferably 5° or greater and more preferably 10° or greater, and preferably 80° or less and more preferably 45° or less. More specifically, the angle α is preferably in the range of 5 to 80° and more preferably in the range of 10 to 45°. Likewise, the angle between the defining member 65B, which partitions and forms the first opening 60A, and the normal 2L can also be set in the same range of the angle α. Meanwhile, when the angle between the defining member 65A and the normal 2L is the same as the angle between the defining member 65B and the normal 2L, the shape of a portion, which is close to the porous member 4, of the first opening 60A in planar view is similar to the shape of a portion, which is distant from the porous member 4, of the first opening 60A in planar view.

The thickness T1 of the defining member unit 66 (the defining members 65A, 65B, and 65C except for the rib 33) (see Fig. 19; corresponding to the depth of the openings 60A, 60B, and 60C) is preferably 30 mm or greater and more preferably 50 mm or greater, and preferably 150 mm or less and more preferably 100 mm or less. More specifically, the thickness T1 of the defining member unit 66 is preferably in the range of 30 to 150 mm and more preferably 50 to 100 mm. Further, a ratio of the thickness T1 of the defining member unit 66 to the thickness T2 (see Fig. 19) of the annular section 34 of the drum body 3 (the flow rate adjusting member 6) [= (T1/T2) × 100] is preferably 50% or greater and more preferably 80% or greater, and preferably 100% or less. More specifically, the ratio of the thickness T1 to the thickness T2 is preferably in the range of 50 to 100% and more preferably in the range of 80 to 100%.

The fiber stacking device 200 will be further described. The porous member 4 and the ring members 5, which have been described above, are detachably fixed to the outer peripheral portion of the drum body 3, which is a part of the flow rate adjusting member 6, in this order by bolts (not illustrated) or the like. In this embodiment, the length of each of these members fixed to the drum body 3 in the longitudinal direction (the drum's circumferential direction) is the same as substantially the half of the perimeter of the rotating drum 1 as illustrated in Fig. 18. Accordingly, the rotating drum 1 can be assembled by fixing two pieces of each member to the drum body 3.

In the fiber stacking device 200, a rotating plate, which has a circular shape in planar view and rotates by receiving power from a prime mover, such as a motor, is fixed to one end of the rotating drum 1 in the drum's width direction X (the direction of the rotation axis of the rotating drum 1), and the drum body 3 (the flow rate adjusting member 6), the porous member 4, and the ring members 5 are integrally rotated about a horizontal axis by the rotation of the rotating plate. On the other hand, a stationary plate, which has a circular shape in planar view, and is fixed to the other components of the fiber stacking device 200, and is not rotated, is fixed to the other end of the rotating drum 1 in the drum's width direction X. Plates, which partition the inside of the rotating drum 1 (the drum body 3) into a plurality of regions in the circumferential direction, are fixed to the stationary plate; and spaces A, B, and C, which are partitioned from one another in the drum's circumferential direction, are formed in the rotating drum 1 (the drum body 3) by the plates as illustrated in Fig. 16. That is, the spaces A to C are partitioned from one another by the plates that are provided from the stationary plate toward the rotating plate. Even when the drum body 3 and the like fixed to the rotating plate are rotated, the plates fixed to the stationary plate are not rotated. Accordingly, the positions of the spaces A, B, and C are constant without changing. Since a known exhaust device (suction means) (not illustrated), such as an air-suction fan, is connected to the space A, the space A can be maintained at negative pressure through the actuation of the exhaust device. While the collecting/stacking recess 22 passes over the space A maintained at negative pressure, fine air-permeation holes of the porous member 4 forming the bottom section 22A of the collecting/stacking recess 22 function as suction holes.

Meanwhile, the pressure of the space B is generally set to negative pressure, which is lower than the pressure of the space A, or zero pressure (atmospheric pressure). Until a fiber stack, which is present in the collecting/stacking recess 22, is transferred onto the transfer roller 12, from the viewpoint of the transport property of the fiber stack, it is preferable that the space B be maintained at low negative pressure and the fiber stack be sucked and held in the recess 22. However, if there is no particular problem in a transport property, it is preferable that the space B be maintained at zero pressure in consideration of a transfer property. Further, since the space C is a region over which the recess 22 passes after the fiber stack present in the recess 22 is transferred onto the transfer roller 12, it is preferable that the space C be maintained at zero pressure or positive pressure.

As illustrated in Fig. 16, one end side of the duct 11 covers the outer peripheral surface of the rotating drum 1 positioned on the space A and the duct 11 includes a shaped-product material introduction device on the other side thereof (not illustrated). The shaped-product material introduction device includes, for example, a pulverizer that pulverizes a wood pulp sheet into fibrillated pulp and sends the fibrillated pulp (the fiber material) into the duct 11. A water-absorbent polymer introduction unit, which introduces water-absorbent polymer particles, may be provided on the duct 11.

As described above, in this embodiment, the plurality of spaces A to C partitioned from one another in the circumferential direction are formed in the rotating drum 1, the space A (the specific space) of the plurality of spaces A to C is maintained at negative pressure by suction from the inside of the rotating drum 1, and a shaped-product material is supplied to the outer peripheral surface of the rotating drum 1, which is positioned on the space A, by the duct 11. The space A is a single space that is not partitioned into a plurality of regions, and is not partitioned into plurality of regions in the drum's width direction X unlike the suction chamber disclosed in, for example, Patent Literature 1. Accordingly, when a single exhaust device (suction means) is connected to the rotating drum 1 and a single space A is maintained at negative pressure by this single suction means as described below, the pressure of the space A is substantially uniform before a shaped-product material is stacked. In this case, if the flow rate adjusting member 6 is not arranged, a suction force generated from the bottom section 22A of the collecting/stacking recess 22 passing over the space A is substantially uniform over the entire bottom section 22A.

The transfer roller 12 has an air-permeable cylindrical outer peripheral portion, and the outer peripheral portion rotates about a horizontal axis by receiving power from a prime mover, such as a motor. A space D of which inside pressure can be reduced is formed in the non-rotating section inside the transfer roller 12 (the rotation-axis side). Since a known exhaust device (not illustrated), such as an air-suction fan, is connected to the space D, the space D can be maintained at negative pressure through the actuation of the exhaust device. A plurality of suction holes for communication between the inside and the outside of the transfer roller are formed in the outer peripheral surface of the transfer roller 12. While passing over the space D maintained at negative pressure, the suction holes suck air to the inside from the outside and the fiber stack (the shaped product) present in the collecting/stacking recess 22 is smoothly transferred from the rotating drum 1 onto the transfer roller 12 by this suction force of the air.

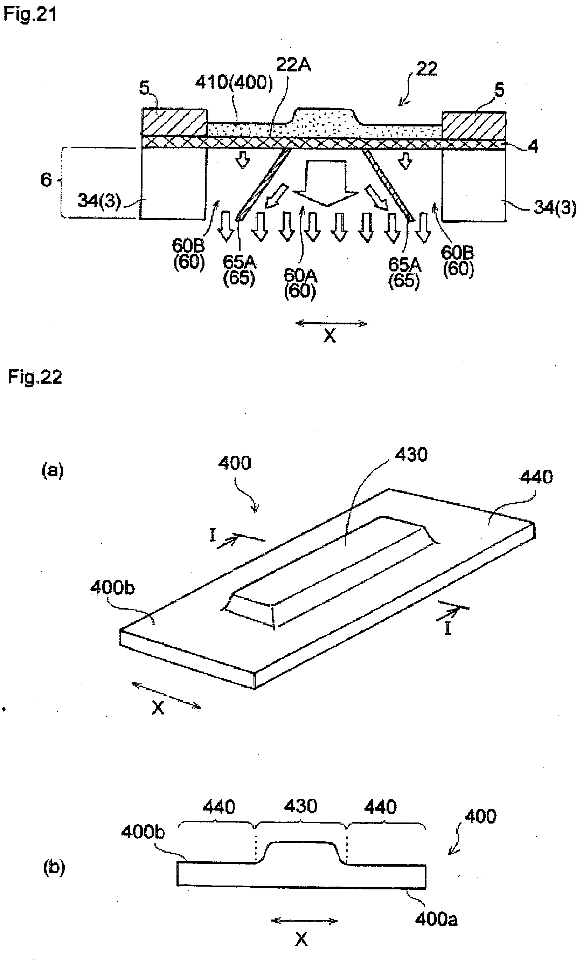

The vacuum box 13 has a box-like shape having upper and lower surfaces, left and right side surfaces, and a rear surface, and includes an opening that opens toward the rotating drum 1. Since a known exhaust device (not illustrated), such as an air-suction fan, is connected to the vacuum box 13 through an exhaust pipe (not illustrated), the inside of the vacuum box 13 can be maintained at negative pressure through the actuation of the exhaust device. Meanwhile, the vacuum box 13 is a unit stably transferring a fiber stack, which is present in a collecting/stacking recess 22, without causing the fiber stack to lose its shape. When a fiber stack 400 (see Fig. 22), which is relatively less likely to lose its shape, is obtained as in this embodiment, the vacuum box 13 may not be particularly provided or does not need to be used even if being provided. The mesh belt 14 is a member that is formed by endlessly connecting a band-shaped air-permeable belt having meshes, and moves continuously along a predetermined route by being guided by a plurality of free rollers 16 and the transfer roller 12. The mesh belt 14 is driven by the rotation of the transfer roller 12. As illustrated in Fig. 16, the mesh belt 14 is arranged so as to sequentially pass between the vacuum box 13 and the rotating drum 1 and between the transfer roller 12 and the rotating drum 1 after being introduced onto the outer peripheral surface of the rotating drum 1 in the vicinity of a downstream end 11a of the duct 11. The mesh belt 14 comes into contact with the outer peripheral surface of the rotating drum 1 while passing the front of the opening of the vacuum box 13, and the mesh belt is separated from the outer peripheral surface of the rotating drum 1 and moves onto the transfer roller 12 in the vicinity of a portion where the transfer roller 12 and the rotating drum 1 come nearest to each other.

The mesh belt 14 includes fine holes that are smaller than the suction holes of the transfer roller 12, and suction from the fine holes of the mesh belt 14, which overlap the suction holes of the transfer roller 12, is also performed with suction from the suction holes of the transfer roller 12. A pair of the windshield plates 15 is provided on both sides of a region, in which the suction holes are formed, of the outer peripheral surface of the transfer roller 12 in the width direction. The windshield plates 15 prevent the fiber stack (the shaped product), which has been released from the collecting/stacking recess 22, from losing its shape by preventing or reducing the inflow of air from the sides.

Next, a method of continuously manufacturing absorbent members (shaped products) by using the above-mentioned fiber stacking device 200, that is, an embodiment of a method of manufacturing an absorbent member of the invention (the second aspect of the invention) will be described. The manufacturing method of this embodiment includes a step of obtaining the fiber stack 400 by sucking and stacking a shaped-product material (an absorbent-member material), which is supplied on an air stream, in the collecting/stacking recess 22 of the rotating drum 1 of the fiber stacking device 200, a step of releasing the fiber stack 400 from the collecting/stacking recess 22, a step of covering the fiber stack 400 with core-wrap sheets 500, and a step of cutting the covered fiber stack 400 to a predetermined size for each core-wrap sheet 500. The method of manufacturing an absorbent member of this embodiment is to manufacture an absorbent member including a high basis-weight section which is stacked on a portion of the bottom section 22A of the collecting/stacking recess 22 corresponding to the porous member-side-small-open-type opening (the first opening 60A) and in which the stacked amount of a shaped-product material is relatively large and a low basis-weight section which is stacked on other portions of the bottom section 22A and in which the stacked amount of a shaped-product material is relatively small.

First, the pressure of the space A formed in the rotating drum 1, the pressure of the space D formed in the transfer roller 12, and the inside pressure of the vacuum box 13 are reduced to negative pressure through the actuation of the exhaust devices connected thereto. When the pressure of the space A is reduced to negative pressure, an air stream (vacuum air) for transporting the shaped-product material onto the outer peripheral surface of the rotating drum 1 is generated in the duct 11. In this embodiment, one exhaust device (suction means) is connected to the rotating drum 1, and suction from the inside of the drum body 3 (the bottom section 22A of the collecting/stacking recess 22) is performed by this single suction means. Suction of the shaped-product material into the collecting/stacking recess 22 is performed by suction from the rotation center (the rotation axis) of the rotating drum 1 that is performed by the single suction means. Accordingly, the pressure of the space A of the rotating drum 1, which has been reduced to negative pressure by the single suction means, is substantially uniform before a shaped-product material is stacked. Further, the rotating drum 1 and the transfer roller 12 are rotated, and a vacuum conveyor (not illustrated), which is arranged below the transfer roller 12, is actuated.

Then, when the shaped-product material introduction device is actuated and a shaped-product material is supplied into the duct 11, the shaped-product material floats on the air stream flowing in the duct 11 and is supplied toward the outer peripheral surface of the rotating drum 1 in a dispersed airborne state.

While the collecting/stacking recess 22 is being transported along the portion covered with the duct 11, a shaped-product material 410 is sucked and stacked in the collecting/stacking recess 22 of the rotating drum 1 as illustrated in Fig. 21. When single suction means is used in a general rotating drum, which is not provided with the flow rate adjusting member 6, as means for maintaining the inside of the general rotating drum at negative pressure, a suction force for sucking the shaped-product material on the bottom section of the collecting/stacking recess (the flow rate of an air stream) is substantially uniform without variation. However, in this embodiment, the suction force is partially different by the flow rate adjusting (distributing) function of the above-mentioned flow rate adjusting member 6 and is not uniform, and the suction force is relatively large at a portion of the bottom section 22A corresponding to the first opening 60A and is relatively small at other portions except for this portion. Accordingly, as illustrated in Fig. 21, relatively much shaped-product material is stacked on a portion of the bottom section 22A corresponding to the first opening 60A and relatively little shaped-product material is stacked on other portions of the bottom section 22A. In Fig. 21, a plurality of arrows, which are directed to the lower side from the upper side, indicate the suction force (air stream) and the size of the arrow schematically indicates the relative magnitude of the suction force. As schematically illustrated in Fig. 21, the suction force is substantially uniform over the total width in the drum's width direction X at an end portion of the opening defining section 65 [the plurality of openings 60 (60A, 60B, and 60C)] relatively distant from the bottom section 22A (the porous member 4) and in the vicinity thereof. However, at an end portion of the opening defining section 65 relatively close to the bottom section 22A and in the vicinity thereof, the suction force is relatively large in the first opening 60A that is the porous member-side-small-open-type opening and is relatively small in the second opening 60B (and the third opening 60C) that is the porous member-side-large-open-type opening.

After the shaped-product material 410 is stacked in the collecting/stacking recess 22 as described above and the fiber stack 400 is obtained, the rotating drum 1 is further rotated. Further, when the fiber stack 400 present in the recess 22 reaches a position facing the vacuum box 13, the fiber stack 400 is sucked onto the mesh belt 14 by suction from the vacuum box 13. In this state, the fiber stack 400 is transported to a portion where the transfer roller 12 and the rotating drum 1 come nearest to each other or to the vicinity thereof. Then, the fiber stack 400, which has been sucked onto the mesh belt 14, is released from the collecting/stacking recess 22 by suction from the transfer roller 12, and is transferred onto the transfer roller 12 together with the mesh belt 14.

Fig. 22 illustrates a part of the fiber stack 400 that has just been released from the collecting/stacking recess 22. As illustrated in Fig. 7, a portion of the fiber stack 400 corresponding to the first opening 60A (a porous member-side-small-open-type opening) of the bottom-section corresponding section 6A of the flow rate adjusting member 6 is a high basis-weight section 430 in which the stacked amount of the shaped-product material is relatively large, and portions of the fiber stack 400 corresponding to the other portions (the second opening 60B and the third opening 60C) of the bottom-section corresponding section 6A are low basis-weight sections 440 in which the stacked amount of the absorbent-member material is relatively small. Further, one surface 400a of the fiber stack 400 (the surface of the fiber stack 400 coming into contact with the bottom section 22A of the recess 22) is substantially flat. On the other hand, the other surface 400b (the surface of the fiber stack 400 that is opposite to the surface of the fiber stack 400 coming into contact with the bottom section 22A of the recess 22) is a projecting-and-recessed surface with large undulations. The fiber stack 400 includes the high basis-weight section 430 in the middle thereof in the drum's width direction X.

The fiber stack 400, which is released from the collecting/stacking recess 22 and is transferred onto the transfer roller 12, is transported while being sucked from the transfer roller 12, and is then passed on to a core-wrap sheet 500 that has been introduced onto the vacuum conveyor (not illustrated) arranged below the transfer roller 12 and is made of tissue paper, liquid-permeable nonwoven fabric, or the like. After that, both side portions of the core-wrap sheet 500 extending in the transporting direction are folded back, and both the upper and lower surfaces of the fiber stack 400 are covered with the core-wrap sheet 500. Then, after the fiber stack 400, which is covered with the core-wrap sheet 500, is compressed in the thickness direction by compressing means (not illustrated), such as press rollers, as necessary, the fiber stack 400 is cut to a predetermined size by a cutter. As a result, an absorbent member, which is formed of a shaped product covered with the core-wrap sheet 500, is obtained. Meanwhile, when the fiber stack 500 has been compressed in the thickness direction, the high basis-weight section 430 constitutes a high-density section of which the density is relatively high and the low basis-weight section 440 constitutes a low-density section of which the density is relatively low. The obtained fiber stack 400 constitutes an absorbent member (the shaped product) that includes a high basis-weight section at the center thereof and includes a low basis-weight section around the high basis-weight section.

According to the fiber stacking device 200 according to this embodiment and a method of manufacturing an absorbent member by using the fiber stacking device 200 that have been described above, the flow rate adjusting member 6 including the first opening 60A, which is the porous member-side-small-open-type opening, is arranged on the surface side, on which the shaped-product material is not stacked, of the bottom section 22A of the collecting/stacking recess 22 (the porous member 4). Accordingly, it is possible to make the flow rate of an air stream, which passes through the portion of the bottom section 22A corresponding to the first opening 60A, be higher than that of an air stream that passes through the other portions of the bottom section 22A. Therefore, since it is possible to easily and accurately increase the basis weight of a desired portion of the absorbent member (the fiber stack) even though single suction means (air stream generating means) is used, an absorbent member (the fiber stack) including the high basis-weight section and the low basis-weight sections is obtained.

When the high basis-weight section and the low basis-weight sections having different basis weights are formed in the absorbent member as described above, an advantage of obtaining an absorbent member which is flexible and of which a wearing feeling is improved, and the like are obtained. Particularly, an absorbent member in which low basis-weight sections are formed on the front and rear of or around a high basis-weight section is flexible and is excellent in a wearing feeling. Further, when an absorbent member, which includes a high basis-weight section and a low basis-weight section, is pressed and compressed in the thickness direction so that the high-density section and the low-density section having different density are formed in the absorbent member, an advantage of obtaining an absorbent member of which absorption performance has been improved, and the like are obtained. This absorbent member, for example, allows body fluid to quickly pass through the low-density section and allows the high-density section to absorb and hold body fluid.

Another embodiment of the fiber stacking device of the invention (the second aspect of the invention) will be described below with reference to the drawings. Meanwhile, components of another embodiment to be described below, which are different from the components of the above-mentioned embodiment, will be mainly described. The same components of another embodiment as those of the first embodiment will be denoted by the same reference numerals and the description thereof will be omitted. The description given for the above-mentioned embodiment is appropriately applied to components that are not particularly described below.

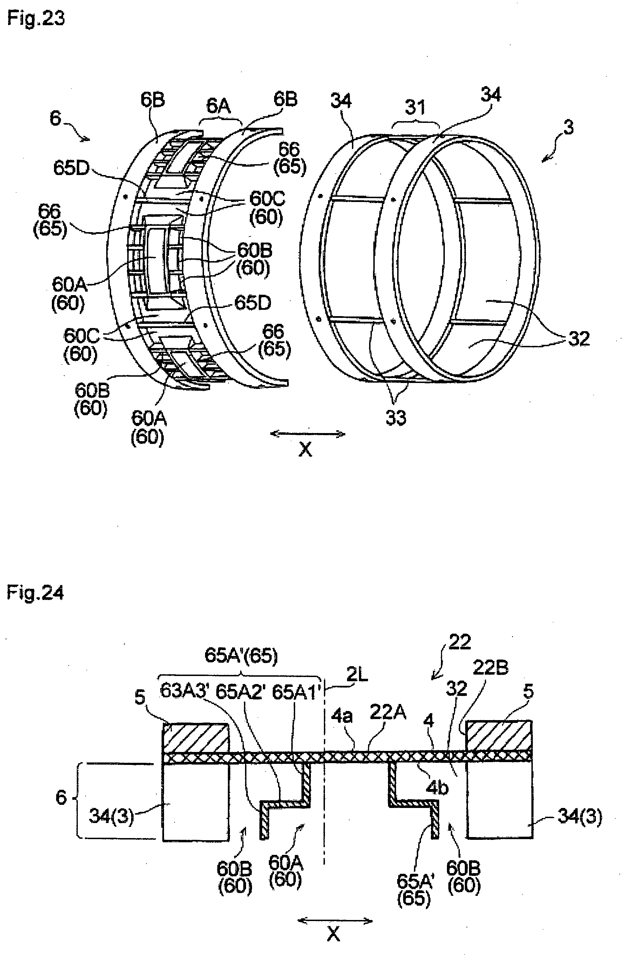

In the embodiment illustrated in Fig. 18, the outer peripheral portion (the ribs 33 and the pair of annular sections 34 and 34) of the drum body 3 functions as a part of the flow rate adjusting member 6 and the other portions (the defining member units 66) of the flow rate adjusting member 6 are detachably fixed to the drum body 3. However, as illustrated in Fig. 23, the entire flow rate adjusting member 6 may be detachably fixed to a drum body 3 that does not substantially function as a flow rate adjusting member. The length (width) of the flow rate adjusting member 6 illustrated in Fig. 23 in the drum's width direction X is the same as the length (width) of a porous member 4 in the drum's width direction X, and the flow rate adjusting member 6 includes a bottom-section corresponding section 6A, which overlaps a bottom section 22A in a planar view of a collecting/stacking recess 22, at the middle portion thereof in the drum's width direction X. Portions of the flow rate adjusting member 6 illustrated in Fig. 23 except for the bottom-section corresponding section 6A, that is, both side sections 6B and 6B of the flow rate adjusting member 6 in the drum's width direction X overlap ring members 5 and annular sections 34 of the drum body 3 in a planar view of the outer peripheral surface of the rotating drum 1. The length (width) of the side section 6B of the flow rate adjusting member 6 in the drum's width direction X is the same as the width of each of the ring member 5 and the annular section 34.

In the flow rate adjusting member 6 illustrated in Fig. 23, a plurality of defining member units 66 (defining members 65A, 65B, and 65C) are arranged in the bottom-section corresponding section 6A at a predetermined interval in the drum's circumferential direction, and a defining member 65D, which has a rectangular shape in planar view and extends in the drum's width direction X, is arranged between adjacent two defining member units 66 and 66. The defining members 65D are formed of sparingly air-permeable or air-impermeable members through which air does not pass substantially, continue between the side sections 6B and 6B of the flow rate adjusting member 6, and overlap the ribs 33 of the drum body 3 in a planar view of the outer peripheral surface of the rotating drum 1.

Like the porous member 4 and the ring members 5 of the embodiment illustrated in Fig. 18, the flow rate adjusting member 6 illustrated in Fig. 23 is detachably fixed to the outer peripheral portion of the drum body 3 by bolts (not illustrated) or the like. As illustrated in Fig. 23, the length of the flow rate adjusting member 6 illustrated in Fig. 23 in the longitudinal direction (the drum's circumferential direction) is the same as substantially the half of the perimeter of the rotating drum 1. Accordingly, the rotating drum 1 can be assembled by fixing two flow rate adjusting members 6 to the drum body 3. In the rotating drum 1 assembled in this way, each of a plurality of second openings 60B is partitioned and formed by two defining members 65C and 65C that extend in the drum's width direction X and the side section 6B and the defining member 65A of the flow rate adjusting member 6 that extend in the drum's circumferential direction, and each of a third openings 60C is partitioned and formed by the defining members 65B, 65C, and 65D that extend in the drum's width direction X and the side sections 6B and 6B of the flow rate adjusting member 6 that extend in the drum's circumferential direction.

Fig. 24 illustrates main parts of still another embodiment of the rotating drum according to the invention (the second aspect of the invention). In the embodiment illustrated in Fig. 24, a defining member 65A', which partitions and forms a first opening 60A (a porous member-side-small-open-type opening), is formed so as to be bent substantially in a stepwise shape in side view. As illustrated in Fig. 24, the defining member 65A' includes a first section 65A1' that extends toward the inside of the rotating drum 1 (the drum body 3) from the porous member 4 side so as to be parallel to a normal 2L to the bottom section 22A of the collecting/stacking recess 22, a second section 65A2' that extends in a direction orthogonal to the normal 2L from an end portion of the first section 65A1' toward the outside in the drum's width direction X, and a third section 65A3' that extends from an end portion of the second section 65A2' toward the drum body 3 side so as to be parallel to the normal 2L. Each of an angle between the first section 65A1' and the second section 65A2' and an angle between the second section 65A2' and the third section 65A3' is a right angle. Each of the first section 65A1', the second section 65A2', and the third section 65A3' has the shape of a straight line in side view (cross-sectional view). Although not illustrated, the defining member 65B is also formed so as to be bent substantially in a stepwise shape in side view, like the defining member 65A'. The same effects as those of the above-mentioned embodiment are achieved even by the embodiment illustrated in Fig. 24.

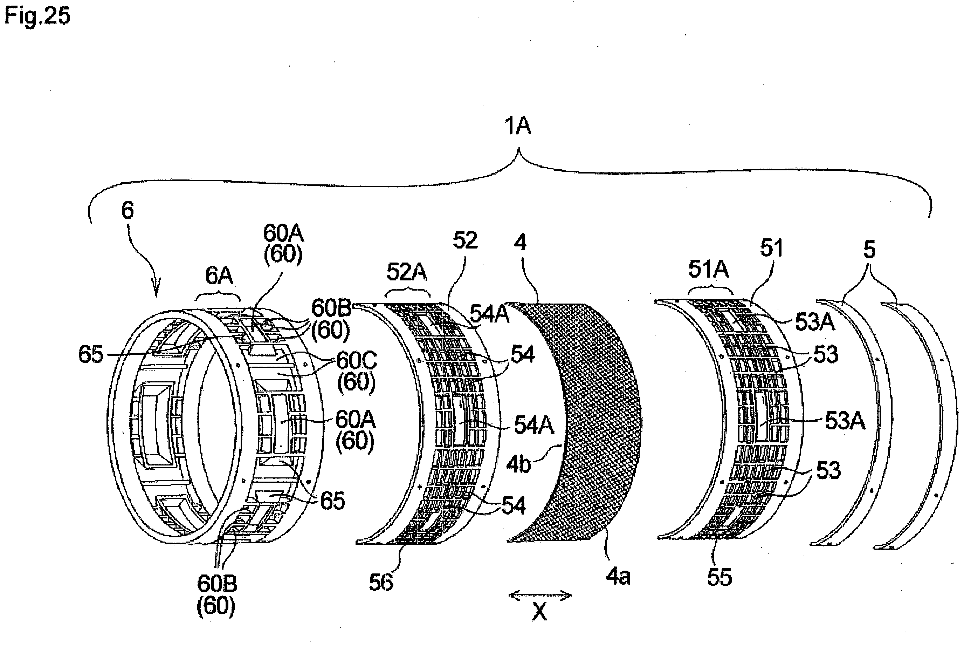

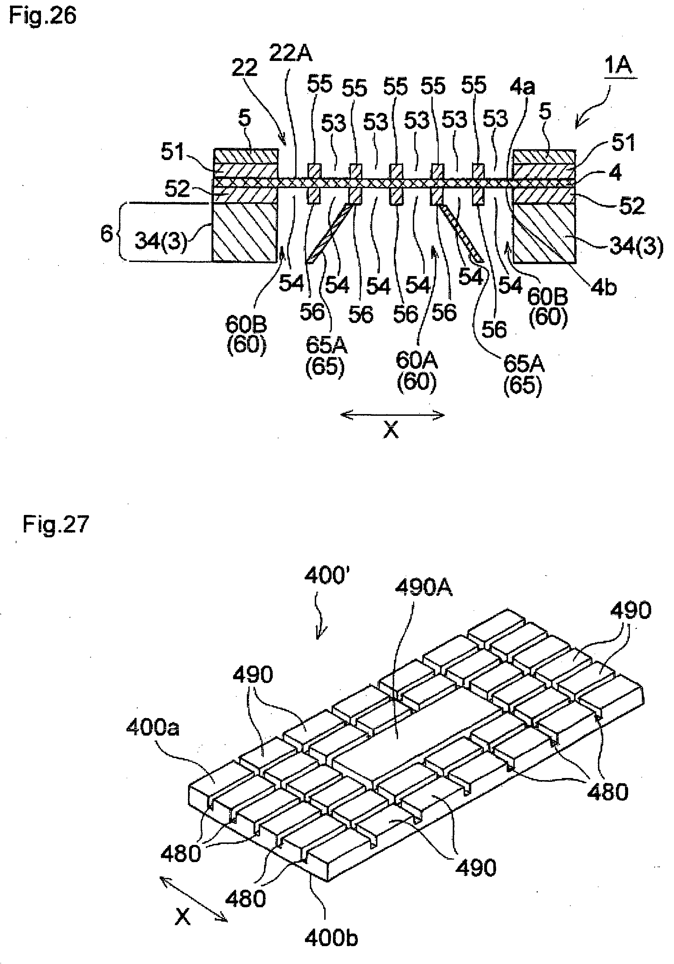

Figs. 25 and 26 illustrate main parts of yet another embodiment (a rotating drum 1A) of the rotating drum according to the invention (the second aspect of the invention). In the rotating drum 1A, a recess partitioning member 51, which partitions a collecting/stacking recess 22 into a plurality of regions in a direction parallel to an outer surface 4a of a porous member 4, is arranged on the outer surface 4a side so as to overlap the outer surface 4a.

The rotating drum 1A will be further described. As illustrated in Figs. 25 and 26, the porous member 4 is interposed between the recess partitioning member 51 that is arranged on the outer surface 4a side and a recess-partitioning-member corresponding member 52 that is arranged on an inner surface 4b side and outside a flow rate adjusting member 6 in the rotating drum 1A. Both the members 51 and 52 are disposed so as to overlap the porous member 4, and other members are not disposed between the recess partitioning member 51 and the porous member 4 and between the recess-partitioning-member corresponding member 52 and the porous member 4. Further, bottom-section corresponding sections 51A and 52A of both the members 51 and 52, which overlap a bottom section 22A of the collecting/stacking recess 22 in a planar view of the collecting/stacking recess 22, include a plurality of openings 53 and 54 that penetrate the bottom-section corresponding sections 51A and 52A in a thickness direction and opening defining sections 55 and 56 that partition and form the openings 53 and 54, respectively. The opening defining section 56 of the recess-partitioning-member corresponding member 52 corresponds to the opening defining section 55 of the recess partitioning member 51. The length (width) of each of the recess partitioning member 51 and the recess-partitioning-member corresponding member 52 in a drum's width direction X is the same as the length (width) of the porous member 4 in the drum's width direction X. The recess partitioning member 51 and the recess-partitioning-member corresponding member 52 include the bottom-section corresponding sections 51A and 52A, which overlap the bottom section 22A in a planar view of a collecting/stacking recess 22, at the middle portions thereof in the drum's width direction X, respectively. Here, "planar view" has the above-mentioned meaning.

When the porous member 4 is interposed between the recess partitioning member 51 and the recess-partitioning-member corresponding member 52 as described above, the respective members 4, 51, and 52 are likely to come into close contact with each other. Accordingly, the transfer property of a fiber stack that is present in the collecting/stacking recess 22, and the like can be improved. Particularly, when the opening defining section 55 of the recess partitioning member 51, which is positioned on a side on which a shaped-product material is stacked, and the porous member 4 come into close contact with each other, a disadvantage that a shaped-product material is caught between the opening defining section 55 and the porous member 4 is further effectively prevented and the defective transfer of the fiber stack, which is present in the collecting/stacking recess 22, is also further effectively prevented. Furthermore, if the opening defining member 56 of the recess-partitioning-member corresponding member 52 and the flow rate adjusting member 6 come into close contact with each other and a gap is not formed between both the members 56 and 6, the leakage (air leak) of an air stream (vacuum air) generated by suction from the inside of the rotating drum 1A is further effectively prevented. Accordingly, a function, which adjusts the flow rate of an air stream, of the flow rate adjusting member 6 is more reliably exerted.

The opening defining section 55 of the recess partitioning member 51 includes a plurality of width-wise linear members that extend in the drum's width direction X and have the shape of a straight line in planar view, and a plurality of circumference-wise linear members that are orthogonal to the plurality of width-wise linear members and have the shape of a straight line in planar view. The opening defining section 55 is formed by these linear members so as to have the shape of a lattice in planar view. The openings 53 of the recess partitioning member 51 are positioned at the cells of the lattice, and have a quadrangular shape in planar view. The opening defining section 55 (the width-wise linear members and the circumference-wise linear members) is formed of a sparingly air-permeable or air-impermeable member through which air does not pass substantially and which is made of metal, such as stainless steel, aluminum, or iron, a resin, or the like. Large openings 53A, each of which has the same shape and dimensions as those of an opening end portion of a first opening 60A relatively close to the porous member 4 and has an area larger than the areas of other openings 53 of the bottom-section corresponding section 51A, are formed at portions of the bottom-section corresponding section 51 A of the recess partitioning member 51 that overlap the first openings 60A of the bottom-section corresponding section 6A of the flow rate adjusting member 6 in planar view.

Further, like the opening defining section 55 of the recess partitioning member 51, the opening defining section 56 of the recess-partitioning-member corresponding member 52 is also formed by a plurality of linear members so as to have the shape of a lattice in planar view, and the openings 54 of the recess-partitioning-member corresponding member 52 are positioned at the cells of the lattice, and have a quadrangular shape in planar view. Large openings 54A, each of which has the same shape and dimensions as those of an opening end portion of the first opening 60A relatively close to the porous member 4 and has an area larger than the areas of other openings 54 of the bottom-section corresponding section 52A, are formed at portions of the bottom-section corresponding section 52A of the recess-partitioning-member corresponding member 52 that overlap the first openings 60A of the bottom-section corresponding section 6A of the flow rate adjusting member 6 in planar view.

Meanwhile, the size of the second opening 60B of the bottom-section corresponding section 6A of the flow rate adjusting member 6 corresponds to the size of two other openings 53 of the opening defining section 55 of the recess partitioning member 51 except for the large opening 53A. Further, an opening group (openings corresponding to a matrix that includes one line in the drum's circumferential direction and two lines in the drum's width direction) formed of two openings 53, which are adjacent to the large opening 53A in the drum's width direction X and are adjacent to each other in the drum's width direction X, of the opening defining section 55 of the recess partitioning member 51 is in one-to-one correspondence with the second openings 60B of the flow rate adjusting member 6 in a planar view of the collecting/stacking recess 22. A relationship between the second openings 60B of the flow rate adjusting member 6 and the openings 54 of the recess-partitioning-member corresponding member 52 is also the same as this relationship.

Furthermore, the size of the third opening 60C of the bottom-section corresponding section 6A of the flow rate adjusting member 6 corresponds to the size of twelve other openings 53 of the opening defining section 55 of the recess partitioning member 51 except for the large opening 53A. Moreover, an opening group (openings corresponding to a matrix that includes two lines in the drum's circumferential direction and six lines in the drum's width direction) formed of two lines in the drum's circumferential direction each of which includes six openings 53, which are adjacent to the large opening 53A in the drum's circumferential direction and are adjacent to each other in the drum's width direction X, of the opening defining section 55 of the recess partitioning member 51 is in one-to-one correspondence with the third openings 60C of the flow rate adjusting member 6 in a planar view of the collecting/stacking recess 22. A relationship between the third openings 60C of the flow rate adjusting member 6 and the openings 54 of the recess-partitioning-member corresponding member 52 is also the same as this relationship.

The collecting/stacking recess 22 is partitioned into a plurality of recesses, which corresponds to the plurality of openings 53 (53A) of the recess partitioning member 51, by the opening defining section 55 of the recess partitioning member 51. Each recess is formed by the porous member 4 (the bottom section 22A) and side walls that are erected from the porous member 4 in the direction of a normal to the porous member 4 and are formed of the opening defining section 55, and the entire porous member 4 constitutes a suction section that sucks a shaped-product material. A space of each recess, which is surrounded by the side walls formed of the opening defining section 55, (the internal space of the recess)is the opening 53 (53A).

As described above, the opening defining section 56 of the recess-partitioning-member corresponding member 52 corresponds to the opening defining section 55 of the recess partitioning member 51. That is, the opening defining section 56 of the recess-partitioning-member corresponding member 52 is disposed so as to necessarily face the opening defining section 55 of the recess partitioning member 51. In the rotating drum 1A, the opening defining section 56 (the width-wise linear members and the circumference-wise linear members) of the recess-partitioning-member corresponding member 52 are in one-to-one correspondence with the opening defining section 55 (the width-wise linear members and the circumference-wise linear members) of the recess partitioning member 51. Accordingly, the plurality of openings 53 (53A) of the recess partitioning member 51 and the plurality of openings 54 (54A) of the recess-partitioning-member corresponding member 52 are also in one-to-one correspondence with each other. That is, one opening 53 (53A) of the recess partitioning member 51 overlaps one opening 54 (54A) of the recess-partitioning-member corresponding member 52 in a planar view of the collecting/stacking recess 22. Further, one opening 53 and one opening 54, which is in one-to-one correspondence with the one opening 53, (openings 53 and 54 overlapping each other in a planar view of the collecting/stacking recess 22) have shapes similar to each other in planar view. In the rotating drum 1 A, a ratio of similitude of the opening 54 to the corresponding opening 53 is 1, and the shape of the opening 53 (53A) in planar view is congruent with the shape of the opening 54 (54A) in planar view.

A fiber stacking device, which includes the rotating drum 1A having the above-mentioned structure, can be used in the same manner as the above-mentioned fiber stacking device 200, and can continuously manufacture absorbent members according to the above-mentioned manufacturing method. While the collecting/stacking recess 22 passes over the space A (see Fig. 1) of the rotating drum 1A maintained at negative pressure, suction from the bottom section 22A is performed as usual at portions of the bottom section 22A of the recess 22 that corresponds to the openings 53 (53A) of the recess partitioning member 51 but suction from the bottom section 22A is not performed at a portion of the bottom section 22A of the recess 22 that corresponds to the opening defining section 55 of the recess partitioning member 51 (a portion of the bottom section 22A coming into contact with the opening defining section 55) since the opening defining section 55 is a sparingly air-permeable or air-impermeable member through which air does not pass substantially. However, in this embodiment, a shaped-product material is stacked not only in the openings 53 (53A) but also on the opening defining section 55. On the upstream side of a duct 11, the shaped-product material is stacked only in the openings 53 (53A), but the shaped-product material starts to be also stacked on the opening defining section 55 in accordance with the intertwining among pieces of the shaped-product material and the flow of air, which is present in the duct 11 and transports the shaped-product material, when the height of the stacked shaped-product material reaches the thickness of the opening defining section 55 (the recess partitioning member 51). When the recess 22 passes over the downstream end 11 a of the duct 11, the opening defining section 55 is completely covered with the shaped-product material.

Fig. 27 illustrates a fiber stack 400' that has just been released from the collecting/stacking recess 22 of the rotating drum 1A in the above-mentioned manufacturing method. As illustrated in Fig. 27, a plurality of grooves (recesses) 480, which extend in both an X direction (the drum's width direction) and a direction orthogonal to the X direction and have the shape of continuous straight lines in planar view, are arranged in the shape of a lattice on one surface 400a of the fiber stack 400' (the surface of the fiber stack 400' coming into contact with the bottom section 22A of the collecting/stacking recess 22) so as to correspond to the lattice-shaped opening defining section 55 of the recess partitioning member 51, and protrusions 490 (490A), which have a quadrangular shape in planar view, are arranged at cells of the lattice so as to correspond to the plurality of openings 53 (53A) of the recess partitioning member 51. Accordingly, one surface 400a forms a projecting-and-recessed surface. Meanwhile, like the other surface 400b of the fiber stack 400 illustrated in Fig. 22, the other surface 400b of the fiber stack 400' (the surface of the fiber stack 400' that is opposite to the surface of the fiber stack 400' coming into contact with the bottom section 22A of the recess 22) forms a projecting-and-recessed surface with large undulations by the high basis-weight section 430 and the low basis-weight sections 440, and includes the high basis-weight section 430 in the middle thereof in the drum's width direction X (see Fig. 22(a)). Since the grooves 480 serve as flexible shafts when the fiber stack 400' is used as, for example, an absorbent member of an absorbent article, the flexibility of the absorbent member is improved. Further, since the grooves 480 also have an effect of diffusing fluid that is excreted to the absorbent member (the fiber stack 400'), the entire absorbent member (the fiber stack 400') can be efficiently used. Furthermore, when this absorbent member (fiber stack 400') is compressed by a press machine or the like, the protrusion 490 forms a "dense portion" in which an absorbent material is present at high density and the groove forms a "coarse portion" in which an absorbent material is present at low density. That is, a coarse-dense structure in which "dense portions" and "coarse portions" are adjacent to each other is formed in a region around the absorbent member (a region of the fiber stack 400' except for the high basis-weight section 430). Accordingly, a fluid-absorption mechanism in which fluid excreted to the region around the absorbent member is absorbed and held by adjacent "dense portion" after quickly passing though the "coarse portions" is achieved. As a result, absorption performance in the region around the absorbent member is improved. Therefore, it is possible to obtain an effect of effectively preventing the reduction of absorption performance in the region around the absorbent member, which is caused by the lack of the absorbent material, in an absorbent member of which a wearing feeling has been improved through the reduction of a basis weight of the region around the absorbent member.

The invention (the second aspect of the invention) is not limited to the above-mentioned embodiments and may be appropriately modified. For example, the collecting/stacking recess 22 is continuously formed on the outer peripheral surface of the rotating drum 1 over the total length of the rotating drum 1 in the circumferential direction in the above-mentioned embodiments, but may be intermittently formed on the outer peripheral surface of the rotating drum 1 in the circumferential direction. In this case, the outermost surface of the rotating drum between two collecting/stacking recesses 22 and 22, which are adjacent to each other in the circumferential direction, can be formed of a ring member 5 that is formed of a sparingly air-permeable or air-impermeable member through which air does not pass substantially so that the shaped-product material is not stacked between the recesses 22 and 22. Further, in the embodiments illustrated in Figs. 18 and 23, each of the members 6, 4, and 5 fixed to the drum body 3 has the length of substantially the half of the perimeter of the rotating drum 1, and is formed by the combination of two pieces. However, the each of the members 6, 4, and 5 may be formed of a single annular member, and may be formed by the combination of three or more pieces.

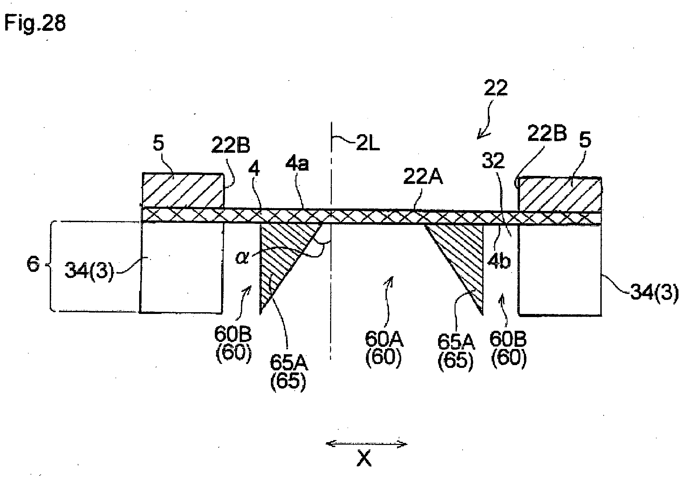

Furthermore, in the embodiments, the bottom-section corresponding section 6A of the flow rate adjusting member 6, a part (60B and 60C) of the plurality of openings 60 are porous member-side-large-open-type openings each of which the area of an opening end portion relatively close to the porous member is larger than the area of an opening end portion relatively distant from the porous member. However, in the invention, like the first openings 60A, a part of the plurality of openings 60 may be porous member-side-small-open-type openings each of which the area of an opening end portion relatively close to the porous member 4 is smaller than the area of an opening end portion relatively distant from the porous member 4, and the flow rate adjusting member 6 may not include the porous member-side-large-open-type opening in the bottom-section corresponding section 6A. In the embodiment illustrated in Fig. 28, a defining member 65A (a part of the opening defining section 65) partitioning the second opening 60B has the shape of a right-angled triangle in side view, and the surface, which faces an inner-side surface 22B (a rib 33) of the collecting/stacking recess 22, of the defining member 65A, which has the shape of a right-angled triangle in side view, is parallel to the inner-side surface 22B. Accordingly, the area of the second opening 60B is constant in a depth direction. For this reason, the second opening 60B is not a porous member-side-large-open-type opening. That is, a flow rate adjusting member 6 of the embodiment illustrated in Fig. 28 includes openings (the second openings 60B) each of which the area is constant in the depth direction, as a part of the plurality of openings 60.

Further, the flow rate adjusting member 6 is detachably fixed to the drum body 3 in the embodiments, but may be integrally fixed to the drum body 3. Furthermore, in the rotating drum 1A (see Fig. 25), all of the plurality of openings 53 of the bottom-section corresponding section 51A of the recess partitioning member 51 may have the same shape and the same dimensions, and the large openings 53A may not be formed on the bottom-section corresponding section 51A. All of the openings 54 of the recess-partitioning-member corresponding member 52 may also have the same shape and the same dimensions.

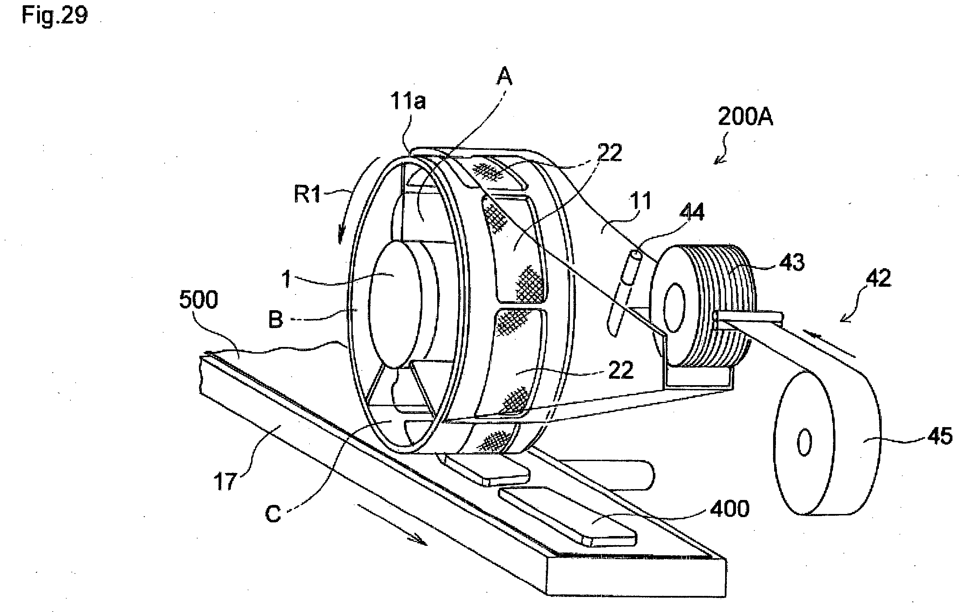

Furthermore, the fiber stacking device of the invention (the second aspect of the invention) is not limited to the fiber stacking device 200 illustrated in Fig. 16, and may be formed like a fiber stacking device 200A illustrated in Fig. 29. Meanwhile, for easy description, a part of one side plate of the rotating drum 2 in an axial direction (a plate that defines the spaces A, B, and C in the rotating drum 1 and has a circular shape in planar view) is transparently illustrated in Fig. 29. The fiber stacking device 200 illustrated in Fig. 16 includes the vacuum box 13 and the windshield plates 15 as means for stably transferring the fiber stack 400, which is present in the collecting/stacking recess 22, without causing the fiber stack 400 to lose its shape. However, as illustrated in Fig. 29, the fiber stacking device 200A does not include the vacuum box 13 and the windshield plate 15. Moreover, in the fiber stacking device 200A, the fiber stack 400 present in the collecting/stacking recess 22 is directly transferred onto a vacuum conveyor 17 that is arranged below the rotating drum 1 (onto a core-wrap sheet 500 that is introduced onto the conveyor 17) at the lowest point of the rotating drum 1. For this reason, the fiber stacking device 200A also does not include the transfer roller 12 and the mesh belt 14 that have been included in the fiber stacking device 200. In Fig. 29, reference numeral 42 denotes a shaped-product material introduction device that can also be applied to the fiber stacking device 200, and the shaped-product material introduction device 42 is adapted to pulverize a wood pulp sheet 45 into fibrillated pulp by a pulverizer 43 and to send the fibrillated pulp (a fiber material) into the duct 11. Further, a water-absorbent polymer introduction unit 44, which introduces water-absorbent polymer particles, is provided on the duct 11.

In the fiber stacking device 200A illustrated in Fig. 29, the pressures of a space A, a space B, and a part of a space C (a region of the space C that is positioned on the upstream side of a transfer position of the fiber stack 400 in a rotating direction R1 of the rotating drum 1) are set to negative pressure, the fiber stack 400 formed in the collecting/stacking recess 22 is transported to a predetermined transfer position (a position which is present above the space C and at which the rotating drum 1 and the vacuum conveyor 17 face each other: the lowest point of the rotating drum 1) by the rotation of the rotating drum 1 while being sucked in the recess 22 above the space A (in the duct 11), the fiber stack 400 is released from the recess 22 at the transfer position by suction from the conveyor 17, and the fiber stack 400 is transferred onto the core-wrap sheet 500 that is introduced onto the conveyor 17. At the transfer position, it is possible to facilitate the release of the fiber stack 400 from the recess 22 by actively blowing air to the vacuum conveyor 17 from the space C. When a fiber stack, which is relatively less likely to lose its shape like the fiber stack 400 illustrated in Fig. 22, is obtained, this so-called transfer blow is particularly effective in facilitating the release of the fiber stack from the collecting/stacking recess.

The absorbent member manufactured in the invention (the second aspect of the invention) is preferably used as an absorbent member of an absorbent article. An absorbent article is used to mainly absorb and hold body fluid, such as urine and menstrual blood, excreted from the body. An absorbent article includes, for example, a disposable diaper, a sanitary napkin, an incontinence pad, a panty liner, and the like. However, the absorbent article is not limited thereto, and widely includes articles that are used to absorb fluid discharged from a human body.

An absorbent article typically includes a topsheet, a backsheet, and a liquid-retaining absorbent member that is interposed between both the sheets. Upper and lower surfaces of the absorbent member may be covered with one or a plurality of core-wrap sheets. The backsheet may have or may not have vapor permeability. The absorbent article may further include various members that correspond to specific uses of the absorbent article. Such the members are known to those skilled in the art. For example, when the absorbent article is applied to a disposable diaper or a sanitary napkin, a pair of or two or more pairs of three-dimensional guards may be disposed outside both side portions, which stand up, of the absorbent member.

In relation to the above-mentioned embodiments of the invention (the second aspect of the invention), the following additional features (for the fiber stacking device, the method of manufacturing an absorbent member, and the absorbent member) are disclosed.
<1A> A fiber stacking device that includes a rotating drum including a collecting/stacking recess formed on an outer peripheral surface thereof and forms a shaped product by stacking a shaped-product material, which is transported on an air stream generated by suction from inside of the rotating drum, on a bottom section of the collecting/stacking recess,
   wherein the bottom section of the collecting/stacking recess is formed of an air-permeable porous member,
   the shaped-product material is stacked on an outer surface of the porous member,
   a flow rate adjusting member that adjusts the flow rate of the air stream is arranged on an inner surface of the porous member,
   the porous member and the flow rate adjusting member are integrally rotated by the rotation of the rotating drum,
   a bottom-section corresponding section of the flow rate adjusting member, which overlaps the bottom section of the collecting/stacking recess in a planar view of the collecting/stacking recess, includes a plurality of openings that penetrate the bottom-section corresponding section in a thickness direction and an opening defining section that partitions and forms the respective openings, and
   a part of the plurality of openings are porous member-side-small-open-type openings each of which the area of an opening end portion relatively close to the porous member is smaller than the area of an opening end portion relatively distant from the porous member.
<2A> The fiber stacking device as set forth in clause <1A>,
   wherein the bottom-section corresponding section of the flow rate adjusting member further includes porous member-side-large-open-type openings each of which the area of an opening end portion relatively close to the porous member is larger than the area of an opening end portion relatively distant from the porous member, as a part of the plurality of openings.
<3A> The fiber stacking device as set forth in clause <2A>,
   wherein the flow rate adjusting member includes opening groups, which include the plurality of opening having different shapes or sizes, in the bottom-section corresponding section, and
   a plurality of the opening groups are present in a drum's circumferential direction.
<4A> The fiber stacking device as set forth in clause <3A>,
   wherein the opening group includes first openings that are the porous member-side-small-open-type openings and are provided at a middle portion of the rotating drum in a width direction, second openings that are the porous member-side-large-open-type openings and are provided on both sides thereof in the width direction with the first opening interposed between the second openings, and third openings that are the porous member-side-large-open-type openings and are provided on front and rear sides thereof in a circumferential direction of the rotating drum with the first opening interposed between the third openings.
<5A> The fiber stacking device as set forth in any one of clauses <1A> to <4A>,
   wherein the sum of the areas of the plurality of openings of the bottom-section corresponding section of the flow rate adjusting member is constant in a thickness direction of the flow rate adjusting member.
<6A> The fiber stacking device as set forth in any one of clauses <1A> to <5A>,
   wherein at least a part (a defining member) of the opening defining section that partitions and forms the porous member-side-small-open-type openings extends so as to be inclined with respect to a normal to the bottom section of the collecting/stacking recess by a predetermined angle from the porous member toward the inside of the rotating drum in side view.
<7A> The fiber stacking device as set forth in clause <6A>,
   wherein an angle between a part (the defining member) of the opening defining section and the normal is preferably 5° or greater and more preferably 10° or greater, and preferably 80° or less and more preferably 45° or less, and more specifically, is preferably in the range of 5 to 80° and more preferably in the range of 10 to 45°.
<8A> The fiber stacking device as set forth in any one of clauses <1A> to <7A>,
   wherein the shape of a portion, which is close to the porous member, of the porous member-side-small-open-type opening in planar view is similar to the shape of a portion, which is distant from the porous member, of the porous member-side-small-open-type opening in planar view.
<9A> The fiber stacking device as set forth in any one of clauses <1A> to <5A>,
   wherein at least a part of the opening defining section, which partitions and forms the porous member-side-small-open-type openings, is formed so as to be bent substantially in a stepwise shape in side view.
<10A> The fiber stacking device as set forth in any one of clauses <1A> to <4A>,
   wherein the flow rate adjusting member includes openings each of which the area is constant in a depth direction, as a part of the plurality of openings.
<11A> The fiber stacking device as set forth in clause <10A>,
   wherein a part (a defining member) of the opening defining section, which partitions and forms the opening of which the area is constant in the depth direction, has the shape of a right-angled triangle in side view, and
   a surface, which faces an inner-side surface of the collecting/stacking recess, of the part (the defining member) of the opening defining section, which has the shape of a right-angled triangle in side view, is parallel to the inner-side surface.
<12A> The fiber stacking device as set forth in any one of clauses <1A> to <11A>,
   wherein a ratio [(S2/S1) x 100] of the area S2 of an opening end portion, which is relatively distant from the porous member, of the porous member-side-small-open-type opening to the area S 1 of an opening end portion, which is relatively close to the porous member, of the porous member-side-small-open-type opening is preferably 150% or greater, more preferably 200% or greater, and even more preferably 500% or greater, and preferably 1000% or less and more preferably 800% or less, and more specifically, is preferably in the range of 150 to 1000%, more preferably in the range of 200 to 1000%, and even more preferably in the range of 500 to 800%.
<13A> The fiber stacking device as set forth in any one of clauses <1A> to <12A>,
   wherein at least a part of the flow rate adjusting member is detachably fixed to the body of the rotating drum.
<14A> The fiber stacking device as set forth in any one of clauses <1A> to <13A>,
   wherein a plurality of spaces, which are partitioned from one another in a circumferential direction, are formed in the rotating drum,
   at least one specific space of the plurality of spaces is maintained at negative by the suction,
   the shaped-product material is supplied to an outer peripheral surface of the rotating drum that is positioned above the specific space, and
   the specific space is a single space that is not partitioned into a plurality of regions.
<15A> The fiber stacking device as set forth in any one of clauses <1A> to <14A>,
   wherein a recess partitioning member, which partitions the collecting/stacking recess into a plurality of regions in a direction parallel to an outer surface of the porous member, is arranged on the outer surface so as to overlap the outer surface.
<16A> The fiber stacking device as set forth in clause <14A>,
   wherein the pressure of the specific space, which is obtained when the specific space is maintained at negative pressure, is substantially uniform before the shaped-product material is stacked.
<17A> A method of manufacturing an absorbent member by using the fiber stacking device as set forth in any one of clauses <1A> to <16A>,
   manufacturing an absorbent member including a high basis-weight section which is stacked on a portion of the bottom section of the collecting/stacking recess corresponding to the porous member-side-small-open-type opening and in which the stacked amount of the shaped-product material is relatively large and a low basis-weight section which is stacked on other portions of the bottom section and in which the stacked amount of a shaped-product material is relatively small.
<18A> The method as set forth in clause <17A>, further including steps of:
   obtaining a fiber stack by sucking and stacking a shaped-product material (an absorbent-member material), which is supplied on an air stream, in the collecting/stacking recess of the rotating drum of the fiber stacking device;
   releasing the fiber stack from the collecting/stacking recess;
   covering the fiber stack with sheets; and
   cutting the covered fiber stack to a predetermined size for each sheet.
<19A> The method as set forth in clause <18A>, wherein suction of the shaped-product material into the collecting/stacking recess is performed by suction from a rotation center of the rotating drum that is performed by single suction means.
<20A> An absorbent member that is manufactured by using the fiber stacking device as set forth in any one of clauses <1A> to <16A>, the absorbent member including:
   a high basis-weight section in which the amount of a shaped-product material to be stacked is relatively large; and
   a low basis-weight section in which the amount of a shaped-product material to be stacked is relatively smaller than the amount of the shaped-product material stacked in the high basis-weight section,
   wherein the high basis-weight section is a portion of the bottom-section corresponding section of the flow rate adjusting member that corresponds to the porous member-side-small-open-type opening.
<21A> The absorbent member as set forth in clause <20A>, wherein the high basis-weight section is provided at the center and the low basis-weight section is provided around the high basis-weight section.

## Claims

1. A fiber stacking device comprising a collecting/stacking recess in which a shaped-product material is stacked and which is formed on an outer surface thereof, and stacking the shaped-product material, which is transported on an air stream generated by suction from inside, on a bottom surface of the collecting/stacking recess, which is formed on a porous member including a plurality of suction holes, while transporting the collecting/stacking recess in one direction,
wherein an adjuster, which adjusts the air stream, is arranged on an inner surface of the porous member so as to overlap at least a part of the inner surface of the porous member, and
the adjuster includes a plurality of openings that penetrates the adjuster in a thickness direction, and the area of an opening end portion of each opening distant from the porous member is smaller than the area of an opening end portion thereof close to the porous member,
wherein the adjuster includes a first layer and a second layer that are laminated in this order from the porous member, wherein the first layer includes a plurality of first openings that penetrate the first layer in the thickness direction, wherein the second layer includes a plurality of second openings which penetrate the second layer in the thickness direction, wherein the first openings
in one-to-one correspondence with the second openings.

2. The fiber stacking device according to claim 1, the first layer includes an opening defining section that partitions and forms the first openings, and the opening defining section includes MD defining members that extend in a transporting direction of the collecting/stacking recess, and CD defining members that extend in a direction orthogonal to the transporting direction,
the plurality of openings are formed of the first and second openings that overlap each other in the planar view, and
the second opening is present at a position separated from the CD defining members in each of the plurality of first openings in a planar view of the collecting/stacking recess, and portions of the second layer overlapping the CD defining members and the vicinity thereof have air impermeability where air does not pass.

3. The fiber stacking device according to claim 2,
wherein the second opening is present at a position separated from the MD defining members in each of the plurality of first openings in a planar view of the collecting/stacking recess, and portions of the second layer overlapping the MD defining members and the vicinity thereof have air impermeability where air does not pass.

4. The fiber stacking device according to any one of claims 2 or 3,
wherein the shape of one of the first openings in planar view and the shape of the second opening, which is present in the one first opening, in planar view are similar to each other in a planar view of the collecting/stacking recess.

5. The fiber stacking device according to any one of claims 1 to 4,
wherein a recess partitioning member, which partitions the recess into a plurality of regions in a direction parallel to the bottom surface, is arranged so as to overlap at least a part of an outer surface of the porous member that forms the bottom surface of the collecting/stacking recess.

6. The fiber stacking device according to claim 5,
wherein the recess partitioning member includes a plurality of openings that penetrate the recess partitioning member in the thickness direction, and
the openings of the recess partitioning member and the openings, which penetrate the adjuster in the thickness direction, overlap each other in a planar view of the collecting/stacking recess.

7. An absorbent member that is manufactured by using the fiber stacking device according to any one of claims 1 to 6,
wherein a portion corresponding to a region, in which the adjuster is not arranged on an inner surface of the porous member, of the collecting/stacking recess is a high basis-weight section in which the amount of a stacked shaped-product material is large, and
a portion corresponding to a region, in which the adjuster is arranged on the inner surface of the porous member, of the collecting/stacking recess is a low basis-weight section in which the amount of a stacked shaped-product material is small.

8. The absorbent member according to claim 7,
wherein the high basis-weight section is a thick section, and the low basis-weight section is a thin section.

9. The absorbent member according to any one of claims 7 or 8,
wherein the absorbent member includes a projecting-and-recessed section that is formed on one surface thereof and includes a plurality of grooves having the shape of continuous straight lines in planar view and arranged in the shape of a lattice and protrusions having a quadrangular shape in planar view and arranged at cells of the lattice.

10. A fiber stacking device that comprises a rotating drum including a collecting/stacking recess formed on an outer peripheral surface thereof and forms a shaped product by stacking a shaped-product material, which is transported on an air stream generated by suction from inside of the rotating drum, on a bottom section of the collecting/stacking recess,
wherein the bottom section of the collecting/stacking recess is formed of an air-permeable porous member,
the shaped-product material is stacked on an outer surface of the porous member,
a flow rate adjusting member that adjusts the flow rate of the air stream is arranged on an inner surface of the porous member,
the porous member and the flow rate adjusting member are integrally rotated by the rotation of the rotating drum,
a bottom-section corresponding section of the flow rate adjusting member, which overlaps the bottom section of the collecting/stacking recess in a planar view of the collecting/stacking recess, includes a plurality of openings that penetrate the bottom-section corresponding section in a thickness direction, and an opening defining section that partitions and forms the respective openings, and
a part of the plurality of openings are porous member-side-small-open-type openings where the area of an opening end portion close to the porous member is smaller than the area of an opening end portion distant from the porous member,
wherein the sum of the areas of the plurality of openings of the bottom-section corresponding section of the flow rate adjusting member is constant in a thickness direction of the flow rate adjusting member.

11. The fiber stacking device according to claim 10,
wherein the bottom-section corresponding section of the flow rate adjusting member further includes porous member-side-large-open-type openings where the area of an opening end portion close to the porous member is larger than the area of an opening end portion distant from the porous member, as a part of the plurality of openings.

12. The fiber stacking device according to any one of claims 10 to 11,
wherein a plurality of spaces, which are partitioned from one another in a circumferential direction, are formed in the rotating drum,
at least one specific space of the plurality of spaces is maintained at negative pressure by the suction,
the shaped-product material is supplied to an outer peripheral surface of the rotating drum that is positioned above the specific space, and
the specific space is a single space that is not partitioned into a plurality of regions.

13. An absorbent member that is manufactured by using the fiber stacking device according to any one of claims 10 to 12, the absorbent member comprising:
a high basis-weight section in which the amount of a stacked shaped-product material is large; and
a low basis-weight section in which the amount of a stacked shaped-product material is smaller than the amount of the shaped-product material stacked in the high basis-weight section,
wherein the high basis-weight section is a portion of the bottom-section corresponding section of the flow rate adjusting member that corresponds to the porous member-side-small-open-type opening.

14. The absorbent member according to claim 13,
wherein the high basis-weight section is provided at the center and the low basis-weight section is provided around the high basis-weight section.

## Patentansprüche

1. Faserschichtungsvorrichtung, die an ihrer Außenfläche eine Sammel/Schichtung-Aussparung, in welcher ein Formproduktmaterial geschichtet wird, aufweist und die ein Formproduktmaterial, das auf einem durch eine Ansaugung aus dem Inneren erzeugten Luftstrom transportiert wird, auf einer aus einem porösen Element mit mehreren Ansauglöchern gebildeten Bodenfläche der Sammel/Schichtung-Aussparung schichtet, während die Sammel/Schichtung-Aussparung in eine Richtung transportiert wird,
wobei ein Anpasser, der den Luftstrom anpasst, an einer Innenfläche des porösen Elements angeordnet ist, um mindestens einen Teil der Innenfläche des porösen Elements zu überlappen, und
der Anpasser mehrere Öffnungen aufweist, die den Anpasser in eine Dickenrichtung durchdringen, und eine Fläche eines bezüglich des porösen Elements fernen Öffnungsendabschnitts jeder Öffnung kleiner ist als die Fläche eines bezüglich des porösen Elements nahen Öffnungsendabschnitts,
wobei der Anpasser - in einer von dem porösen Element ausgehenden Reihenfolge - eine erste Schicht und eine zweite Schicht aufweist, die laminiert sind, wobei die erste Schicht mehrere erste Öffnungen aufweist, die die erste Schicht in einer Dickenrichtung durchdringen, wobei die zweite Schicht mehrere zweite Öffnungen aufweist, die die zweite Schicht in einer Dickenrichtung durchdringen, wobei die ersten Öffnungen in einer Eins-zu-eins-Entsprechung mit den zweiten Öffnungen sind.

2. Faserschichtungsvorrichtung nach Anspruch 1,
wobei die erste Schicht einen öffnungsdefinierenden Abschnitt aufweist, der die ersten Öffnungen voneinander trennt und bildet, und der öffnungsdefinierende Abschnitt MDdefinierende Elemente, die sich in einer Transportrichtung der Sammel/Schichtung-Aussparung erstrecken, und CD-definierende Elemente aufweist, die sich in einer zur Transportrichtung orthogonalen Richtung erstrecken,
die mehreren Öffnungen aus den ersten und zweiten Öffnungen gebildet sind, die in der Planansicht einander überlappen, und
in einer Planansicht der Sammel/Schichtung-Aussparung die zweite Öffnung an einer von den CD-definierenden Elementen getrennten Position in jeder der mehreren ersten Öffnungen vorhanden ist und Abschnitte der zweiten Schicht, die die CD-definierenden Elemente und deren nähere Umgebung überlappen, eine Luftundurchlässigkeit haben, wo Luft nicht hindurchgeht.

3. Faserschichtungsvorrichtung nach Anspruch 2,
wobei in einer Planansicht der Sammel/Schichtung-Aussparung die zweite Öffnung an einer von den MD-definierenden Elementen getrennten Position in jeder der mehreren ersten Öffnungen vorhanden ist und Abschnitte der zweiten Schicht, die die MD-definierenden Elemente und deren nähere Umgebung überlappen, eine Luftundurchlässigkeit haben, wo Luft nicht hindurchgeht.

4. Faserschichtungsvorrichtung nach einem der Ansprüche 2 oder 3,
wobei in Planansicht die Form einer der ersten Öffnungen und in Planansicht die Form der zweiten Öffnung, die in dieser ersten Öffnung vorhanden ist, in einer Planansicht der Sammel/Schichtung-Aussparung einander ähnlich sind.

5. Faserschichtungsvorrichtung nach einem der Ansprüche 1 bis 4,
wobei ein aussparungsunterteilendes Element, das in einer zur Bodenfläche parallelen Richtung die Aussparung in mehrere Zonen unterteilt, so angeordnet ist, um mindestens einen Teil einer Außenfläche des porösen Elements zu überlappen, die die Bodenfläche der Sammel/Schichtung-Aussparung bildet.

6. Faserschichtungsvorrichtung nach Anspruch 5,
wobei das aussparungsunterteilende Element mehrere Öffnungen aufweist, die das aussparungsunterteilende Element in der Dickenrichtung durchdringen, und
die Öffnungen des aussparungsunterteilenden Elements und die Öffnungen, die den Anpasser in der Dickenrichtung durchdringen, in einer Planansicht der Sammel/Schichtung-Aussparung einander überlappen.

7. Absorbierendes Element, das unter Verwendung der Faserschichtungsvorrichtung nach einem der Ansprüche 1 bis 6 hergestellt ist,
wobei ein Abschnitt, der einer Zone entspricht, in welcher der Anpasser nicht an einer Innenfläche des porösen Elements der Sammel/Schichtung-Aussparung angeordnet ist, ein Abschnitt mit hohem Basisgewicht ist, in welchem die Menge des geschichteten Formproduktmaterials groß ist, und
ein Abschnitt, der einer Zone entspricht, in welcher der Anpasser an einer Innenfläche des porösen Elements der Sammel/Schichtung-Aussparung angeordnet ist, ein Abschnitt mit niedrigem Basisgewicht ist, in welchem die Menge des geschichteten Formproduktmaterials klein ist.

8. Absorbierendes Element nach Anspruch 7,
wobei der Abschnitt mit hohem Basisgewicht ein dicker Abschnitt ist und der Abschnitt mit niedrigem Basisgewicht ein dünner Abschnitt ist.

9. Absorbierendes Element nach einem der Ansprüche 7 oder 8,
wobei das absorbierende Element einen an seiner Oberfläche gebildeten vorstehenden- und-vertieften Abschnitt aufweist, der mehrere Rillen, die in Planansicht die Form von kontinuierlichen geraden Linien haben und in Form eines Gitters angeordnet sind, und Vorsprünge aufweist, die in Planansicht viereckige Form haben und an den Zellen des Gitters angeordnet sind.

10. Faserschichtungsvorrichtung, die eine rotierende Trommel mit einer an einer Außenumfangsfläche gebildeten Sammel/Schichtung-Aussparung aufweist und durch Schichtung eines Formproduktmaterials, das auf einem aus dem Inneren der rotierenden Trommel erzeugten Luftstrom transportiert wird, auf einem Bodenabschnitt der Sammel/Schichtung-Aussparung ein Formprodukt bildet,
wobei der Bodenabschnitt der Sammel/Schichtung-Aussparung aus einem luftdurchlässigen porösen Element gebildet ist,
das Formproduktmaterial auf einer Außenfläche des porösen Elements geschichtet wird, ein Strömungsrateanpassungselement, das die Strömungsrate des Luftstroms anpasst, an einer Innenfläche des porösen Elements angeordnet ist,
das poröse Element und das Strömungsrateanpassungselement durch die Rotation der rotierenden Trommel integral rotiert werden,
ein dem Bodenabschnitt entsprechender Abschnitt des Strömungsrateanpassungselements, der in einer Planansicht der Sammel/Schichtung-Aussparung den Bodenabschnitt der Sammel/Schichtung-Aussparung überlappt, mehrere Öffnungen, die den dem Bodenabschnitt entsprechenden Abschnitt in einer Dickenrichtung durchdringen, und einen öffnungsdefinierenden Abschnitt aufweist, der die jeweiligen Öffnungen voneinander trennt und bildet, und
ein Teil der mehreren Öffnungen Öffnungen des Typs mit einer an der Seite des porösen Elements kleinen Öffnungsweite sind, wo die Fläche eines bezüglich des porösen Elements nahen Öffnungsendabschnitts kleiner ist als die Fläche eines bezüglich des porösen Elements fernen Öffnungsendabschnitts,
wobei die Summe der Flächen der mehreren Öffnungen des dem Bodenabschnitt entsprechenden Abschnitts des Strömungsrateanpassungselements in einer Dickenrichtung des Strömungsrateanpassungselements konstant ist.

11. Faserschichtungsvorrichtung nach Anspruch 10,
wobei der dem Bodenabschnitt entsprechende Abschnitt des Strömungsrateanpassungselements desweiteren als ein Teil der mehreren Öffnungen Öffnungen des Typs mit einer an der Seite des porösen Elements großen Öffnungsweite aufweist, wo die Fläche eines bezüglich des porösen Elements nahen Öffnungsendabschnitt größer ist als die Fläche eines bezüglich des porösen Elements fernen Öffnungsendabschnitts.

12. Faserschichtungsvorrichtung nach einem der Ansprüche 10 bis 11,
wobei mehrere Räume, die in einer Umfangsrichtung voneinander getrennt sind, in der rotierenden Trommel gebildet sind,
mindestens ein spezifischer Raum der mehreren Räume mittels Ansaugung auf Unterdruck gehalten wird,
das Formproduktmaterial zu einer Außenumfangsfläche der rotierenden Trommel geführt wird, die oberhalb des spezifischen Raums angeordnet ist, und
der spezifische Raum ein einziger Raum ist, der nicht in mehrere Zonen unterteilt ist.

13. Absorbierendes Element, das unter Verwendung der Faserschichtungsvorrichtung nach einem der Ansprüche 10 bis 12 hergestellt ist, wobei das absorbierende Element aufweist:
einen Abschnitt mit hohem Basisgewicht, in welchem die Menge eines geschichteten Formproduktmaterials groß ist; und
einen Abschnitt mit niedrigem Basisgewicht, in welchem die Menge eines geschichteten Formproduktmaterials kleiner ist als die Menge des in dem Abschnitt mit hohem Basisgewicht geschichteten Formproduktmaterials,
wobei der Abschnitt mit hohem Basisgewicht ein Teil des dem Bodenabschnitt entsprechenden Abschnitts des Strömungsrateanpassungselements ist, der der Öffnung des Typs mit einer an der Seite des porösen Elements kleinen Öffnungsweite entspricht.

14. Absorbierendes Element nach Anspruch 13,
wobei der Abschnitt mit hohem Basisgewicht in der Mitte bereitgestellt ist und der Abschnitt mit niedrigem Basisgewicht um den Abschnitt mit hohem Basisgewicht herum bereitgestellt ist.

## Revendications

1. Dispositif d'empilage de fibres comprenant une cavité de collecte/d'empilage dans laquelle un matériau de produit façonné est empilé et qui est formée sur une surface extérieure de celui-ci, et empilant le matériau de produit façonné, qui est transportée sur un flux d'air généré par une aspiration depuis l'intérieur, sur une surface inférieure de la cavité de collecte/d'empilage, qui est formée sur un élément poreux comportant une pluralité d'orifices d'aspiration, tout en transportant la cavité de collecte/d'empilage dans une direction,
dans lequel un organe de réglage, qui règle le flux d'air, est disposé sur une surface interne de l'élément poreux de façon à se superposer au moins à une partie de la surface interne de l'élément poreux, et
l'organe de réglage comporte une pluralité d'ouvertures qui pénètrent dans l'organe de réglage dans la direction de l'épaisseur, et la superficie d'une portion d'extrémité d'ouverture de chaque ouverture distante de l'élément poreux est inférieure à la superficie d'une portion d'extrémité d'ouverture de celui-ci proche de l'élément poreux,
dans lequel l'organe de réglage comporte une première couche et une seconde couche qui sont stratifiées dans cet ordre à partir de l'élément poreux, dans lequel la première couche comporte une pluralité de premières ouvertures qui pénètrent dans la première couche dans la direction de l'épaisseur, dans lequel la seconde couche comporte une pluralité de secondes ouvertures qui pénètrent dans la seconde couche dans la direction de l'épaisseur, dans lequel les premières ouvertures sont en correspondance en face à face avec les secondes ouvertures.

2. Dispositif d'empilage de fibres selon la revendication 1, la première couche comporte une section définissant une ouverture qui sépare et forme les premières ouvertures, et la section définissant l'ouverture comporte des éléments définissant un MD qui s'étendent dans une direction de transport de la cavité de collecte/d'empilage, et des éléments définissant un CD qui s'étendent dans une direction orthogonale à la direction de transport,
la pluralité d'ouvertures sont formées des premières et secondes ouvertures qui se superposent mutuellement dans une vue en plan, et
la seconde ouverture est présente dans une position séparée des éléments définissant un CD dans chacune de la pluralité des premières ouvertures dans une vue en plan de la cavité de collecte/d'empilage, et des portions de la seconde couche se superposant sur les éléments définissant le CD et la proximité de ceux-ci présentent une imperméabilité à l'air où l'air ne passe pas.

3. Dispositif d'empilage de fibres selon la revendication 2,
dans lequel la seconde ouverture est présente dans une position séparée des éléments définissant le MD dans chacune de la pluralité des premières ouvertures dans une vue en plan de la cavité de collecte/d'empilage, et des portions de la seconde couche se superposant aux éléments définissant le MD et la proximité de ceux-ci présentent une imperméabilité à l'air où l'air ne passe pas.

4. Dispositif d'empilage de fibres selon l'une quelconque des revendications 2 ou 3,
dans lequel la forme d'une des premières ouvertures dans une vue en plan et la forme de la seconde ouverture, qui est présente dans une première ouverture, dans une vue en plan, sont similaires l'une à l'autre dans une vue en plan de la cavité de collecte/empilage.

5. Dispositif d'empilage de fibres selon l'une quelconque des revendications 1 à 4,
dans lequel un élément de séparation de cavité, qui sépare la cavité en une pluralité de régions dans une direction parallèle à la surface inférieure, est disposé de façon à se superposer au moins sur une partie d'une surface extérieure de l'élément poreux qui forme la surface inférieure de la cavité de collecte/d'empilage.

6. Dispositif d'empilage de fibres selon la revendication 5,
dans lequel l'élément de séparation de cavité comporte une pluralité d'ouvertures qui pénètrent dans l'élément de séparation de cavité dans la direction de l'épaisseur, et
les ouvertures de l'élément de séparation de cavité et les ouvertures qui pénètrent dans l'organe de réglage dans la direction de l'épaisseur, se superposent mutuellement dans une vue en plan de la cavité de collecte/d'empilage.

7. Élément absorbant qui est fabriqué en utilisant le dispositif d'empilage de fibres selon l'une quelconque des revendications 1 à 6,
dans lequel une portion correspondant à une région, dans laquelle l'organe de réglage n'est pas disposé sur une surface interne de l'élément poreux, de la cavité de collecte/d'empilage est une section à poids de base élevé dans laquelle la quantité d'un matériau de produit façonné empilé est importante, et
une portion correspondant à une région, dans laquelle l'organe de réglage est disposé sur la surface intérieure de l'élément poreux de la cavité de collecte/d'empilage est une section à poids de base faible dans laquelle la quantité d'un matériau de produit façonné empilé est faible.

8. Élément absorbant selon la revendication 7,
dans lequel la section à poids de base élevé est une section épaisse, et la section à poids de base faible est une section mince.

9. Élément absorbant selon l'une quelconque des revendications 7 ou 8,
dans lequel l'élément absorbant comporte une section en saillie et en creux qui est formée sur une surface de celle-ci et comporte une pluralité de cannelures présentant la forme de lignes droites continues dans une vue en plan et disposées sous la forme d'un treillis et des saillies présentant une forme quadrangulaire dans une vue en plan et disposées au niveau des cellules du treillis.

10. Dispositif d'empilage de fibres qui comprend un tambour rotatif comportant une cavité de collecte/d'empilage formée sur une surface périphérique extérieure de celui-ci et forme un produit façonné en empilant un matériau de produit façonné, qui est transportée sur un flux d'air généré par aspiration depuis l'intérieur du tambour rotatif, sur une section inférieure de la cavité de collecte/empilage,
dans lequel la section inférieure de la cavité de collecte/d'empilage est formée d'un élément poreux perméable à l'air,
le matériau de produit façonné est empilé sur une surface extérieure de l'élément poreux,
un élément de réglage de débit qui règle le débit du flux d'air est disposé sur une surface interne de l'élément poreux,
l'élément poreux et l'élément de réglage de débit sont mis en rotation d'un seul tenant par la rotation du tambour de rotation,
une section correspondant à la section inférieure de l'élément de réglage de débit, qui se superpose à la section inférieure de la cavité de collecte/d'empilage dans une vue en plan de la cavité de collecte/d'empilage, comporte une pluralité d'ouvertures qui pénètrent dans la section correspondant à la section inférieure dans la direction de l'épaisseur, et une section définissant une ouverture qui sépare et forme les ouvertures respectives, et
une partie de la pluralité d'ouvertures est des ouvertures de type petite ouverture latérale de l'élément poreux où la superficie d'une portion d'extrémité d'ouverture proche de l'élément poreux est inférieure à la superficie d'une portion d'extrémité d'ouverture distante de l'élément poreux,
dans lequel la somme des superficies de la pluralité d'ouvertures de la section correspondant à la section inférieure de l'élément de réglage de débit est constante dans la direction de l'épaisseur de l'élément de réglage de débit.

11. Dispositif d'empilage de fibres selon la revendication 10,
dans lequel la section correspondant à la section inférieure de l'élément de réglage de débit comporte en outre des ouvertures de type grande ouverture latérale de l'élément poreux où la superficie d'une portion d'extrémité d'ouverture proche de l'élément poreux est supérieure à la superficie d'une portion d'extrémité d'ouverture distante de l'élément poreux, comme une partie de la pluralité des ouvertures.

12. Dispositif d'empilage de fibres selon l'une quelconque des revendications 10 à 11,
dans lequel une pluralité d'espaces, qui sont séparés les uns des autres dans une direction circonférentielle, sont formés dans le tambour rotatif,
au moins un espace spécifique de la pluralité d'espaces est maintenu en pression négative par l'aspiration,
le matériau de produit façonné est fourni à une surface périphérique extérieure du tambour rotatif qui est positionnée au-dessus de l'espace spécifique, et
l'espace spécifique est un espace unique qui n'est pas séparé en une pluralité de régions.

13. Élément absorbant qui est fabriqué en utilisant le dispositif d'empilage de fibres selon l'une quelconque des revendications 10 à 12, l'élément absorbant comprenant :
une section à poids de base élevé dans laquelle la quantité d'un matériau de produit façonné empilé est importante ; et
une section à poids de base faible dans laquelle la quantité de matériau de produit façonné empilé est inférieure à la quantité du matériau de produit façonné empilé dans la section à poids de base élevé,
dans lequel la section à poids de base élevé est une portion de la section correspondant à la section inférieure de l'élément de réglage de débit qui correspond à l'ouverture de type petite ouverture latérale de l'élément poreux.

14. Élément absorbant selon la revendication 13,
dans lequel la section à poids de base élevé est prévue au niveau du centre et la section à poids de base faible est prévue autour de la section à poids de base élevé.
